# EUROPEAN PATENT APPLICATION

(11) **EP 4 768 587 A1**
(43) Date of publication of application: **01.07.2026**
(21) Application number: 24870893.5
(22) Date of filing: 26.09.2024
(51) Int. Cl.: C12N 15/113, A61P 25/28, C07K 19/00, C07K 14/47

(54) **SIRNA INHIBITING EXPRESSION OF AMYLOID PRECURSOR PROTEIN (APP) GENE, DRUG, AND USE**

(30) Priority: 28.09.2023 CN 202311274910; 28.03.2024 CN 202410368873
(71) Applicant: Bebetter Med Inc., Guangzhou, Guangdong 510660 (CN)
(72) Inventor: FAN, Fushun, Guangzhou, Guangdong 510660 (CN); LIU, Xinjian, Guangzhou, Guangdong 510660 (CN); LIN, Yingying, Guangzhou, Guangdong 510660 (CN); FU, Tiancheng, Guangzhou, Guangdong 510660 (CN); QIAN, Changgeng, Guangzhou, Guangdong 510660 (CN)
(74) Representative: Fuchs Patentanwälte Partnerschaft mbB
(86) International application number: PCT/CN2024/121498
(87) International publication number: WO 2025/067354

(57) **Abstract**

The present invention provides siRNA, peptide oligonucleotide drugs, and their applications for suppressing the expression of the amyloid precursor protein (APP) gene in human cells. The siRNA exhibits potent activity in inhibiting APP expression. Through appropriate modifications, its ability to silence the target is enhanced while reducing off-target activity. The described siRNA and its conjugates hold promise for clinical application in the prevention and treatment of diseases associated with the APP target, including cerebral amyloid angiopathy (CAA), early-onset familial Alzheimer's disease (EOFAD), or Alzheimer's disease (AD).

## Description

The present invention claims priority to Chinese Patent Application No. 2023112749104 filed on September 28, 2023, and Chinese Patent Application No. 2024103688731 filed on March 28, 2024, both of which are incorporated herein by reference.

### TECHNICAL FIELD

The present invention relates to the field of biomedicine, specifically to siRNA and drugs for inhibiting the expression of the amyloid precursor protein (APP) gene and their applications.

### BACKGROUND ART

Alzheimer's disease (AD) is one of the most prevalent neurodegenerative diseases worldwide and a major cause of disability in the elderly, characterized by progressive cognitive decline. In 2019, AD affected over 50 million people worldwide, with projections reaching 152 million by 2050, imposing a substantial socioeconomic burden (Guzman-Martinez L et al., Curr Alzheimer Res. 2019;16(6):518-528.). Current clinical treatments for AD include acetylcholinesterase inhibitors or N-methyl-D-aspartate receptor antagonists. However, these drugs only mildly improve cognition and behavior in AD patients and cannot slow disease progression (Versijpt J et al., 2014;42 Suppl 3:S19-S25.). The reason lies in the fact that the etiology and pathogenesis of AD remain incompletely understood. The current consensus identifies pathological features including plaques composed of aggregated amyloid-β (Aβ) protein, neurofibrillary tangles containing hyperphosphorylated tau protein, and neuroinflammation (Li R et al., Proc Natl Acad Sci U S A. 2004;101(10):3632-3637.). The Aβ peptide, containing a large number of hydrophobic amino acids, readily forms self-aggregates associated with synaptic toxicity and neurodegeneration. The abnormal accumulation of the Aβ peptide is considered a key pathogenic event in AD.

Aβ peptides originate from proteolytic cleavage of the amyloid precursor protein (APP), a full-length membrane protein expressed in neurons and glia. These peptides are sequentially cleaved by beta-secretase (BACE1) and γ-secretase to generate Aβ peptides (Shankar GM et al., Nat Med. 2008;14(8):837-842.). Given the extensive substrate repertoire of γ-secretase and the adverse consequences of its pharmacological inhibition, BACE1 is considered a more suitable target for regulating the Aβ peptide pathway. Strategies to reduce Aβ levels by inhibiting BACE1 activity are regarded as potential therapeutic approaches for AD (Vassar R et al., 2014;130(1):4-28). A transdermal patch containing a BACE1 inhibitor is currently in Phase III clinical trials (Doody RS et al. N Engl J Med. 2013;369(4):341-350.). However, due to target toxicity and safety concerns, several BACE1 small-molecule inhibitors failed to advance into clinical trials. APP, as the precursor protein directly producing Aβ peptides, is considered a highly promising potential therapeutic target for AD or other neurodegenerative diseases.

Compared with traditional small-molecule drugs, small interfering RNA (siRNA) therapeutics leverage the cell's endogenous system to achieve targeted, specific degradation of the intended gene, offering promising clinical therapeutic prospects. In terms of mechanism, exogenously introduced double-stranded siRNAs can be recognized by the endoribonuclease Dicer in the cell and split into a single-stranded guide strand and its complementary strand in the RNA-induced silencing complex (RISC). The guide strand siRNA then binds to the Argonaute 2 protein (AGO2) and guides it to the target RNA, and AGO2 mediates the degradation of the target RNA. In addition to degrading RNAs in the cytoplasm, siRNAs also promote chromatin remodeling and histone modification in the nucleus, thereby inducing transcriptional silencing (Matzke et al. Nature Reviews Genetics vol. 6, 1 (2005): 24-35). siRNA drug delivery is a critical enabler for RNAi therapeutics. The most mature siRNA intrahepatic delivery system currently leverages the specific binding of N-acetylgalactosamine (GalNAc) to the hepatocyte-specific asialoglycoprotein receptor (ASGPR), thereby achieving active delivery into liver cells. This represents a significant breakthrough in the field of RNA therapeutics (Zhang L et al., Front Pharmacol. 2022;13:1090237.). Beyond liver tissue, researchers found that adenoviruses targeting mouse brains containing BACE1 siRNA demonstrated the potential to improve AD pathological states (Zhou Y et al., Sci Adv. 2020;6(41):eabc7031). However, effective, safe, and comprehensive siRNA extrahepatic delivery systems still face significant challenges, particularly in brain tissues where the blood-brain barrier is present.

Regarding the development of RNAi therapeutics targeting the central nervous system, researchers established a system of chemically modified siRNA conjugated to fatty alcohol 2'-O-hexadecyl (C16) (Nat Biotechnol. 2022 Oct; 40(10):1500-1508.). Intracerebroventricular injection of C16-siRNA targeting APP into Alzheimer's disease mice reduced amyloid-β deposition, inflammation, and behavioral deficits. Similarly, following administration of C16-siRNA into the central nervous system of non-human primates, extensive siRNA distribution and RNAi activity were achieved, with target gene silencing exceeding 75% and persisting for at least 3 months. This provides a crucial technical platform for off-liver targeted RNAi therapeutics (Brown KM et al., Nat Biotechnol. 2022;40(10):1500-1508.). Furthermore, utilizing target cell surface receptor proteins to achieve transcellular drug transport and thereby cross the blood-brain barrier represents another significant approach for delivering drugs to the central nervous system. Low-density lipoprotein receptor-related protein 1 (LRP1), widely expressed in neurons and brain endothelial cells, is a multifunctional endocytic receptor involved in numerous biological processes, including the transport of metabolites (Aβ peptides) across the blood-brain barrier. Consequently, it represents a highly attractive target for central nervous system drug delivery (Ulery PG et al., J Biol Chem. 2000; 275(10):7410-7415.). LRP1 possesses multiple ligands, reflecting its diverse physiological functions. In recent years, researchers have developed a transport peptide called Angiopep-2 (ANG2). By leveraging its interaction with the LRP1 receptor, drugs conjugated to ANG2 can be transported into brain tissue. ANG2-mediated paclitaxel delivery represents the first such instance and has undergone clinical trials (Régina A et al., Br J Pharmacol. 2008;155(2):185-197).

The present invention will develop an effective non-viral siRNA composition capable of permeating the blood-brain barrier and targeting APP, utilizing the principle of LRP1 ligand-receptor interaction to develop a delivery method for the siRNA composition.

### SUMMARY OF INVENTION

Accordingly, the present invention aims to provide an siRNA or its pharmaceutically acceptable salt or conjugate (drug) for suppressing amyloid precursor protein (APP) gene expression, along with its applications. The siRNA and its conjugate exhibit the advantages of excellent *in vitro* and *in vivo* efficacy with low toxicity.

The first aspect of the present invention provides an siRNA or its pharmaceutically acceptable salt for suppressing APP gene expression, comprising a sense strand and an antisense strand, wherein the base composition of the siRNA corresponds to each group of sense strands and their respective antisense strands as shown in Table 1, or comprises a nucleotide sequence differing by no more than one nucleotide from any sense strand and its corresponding antisense strand shown in Table 1.

Preferably, the composition of siRNA targeting the APP gene is selected from any of the following sequences in Table 2, or
sequences differing from its sense strand or antisense strand by no more than one nucleotide: 12006.1-1, 12006.2-4, 12037.1-1, 12037.2-4, 12075.1-1, 12075.2-4, 12076.1-1, and 12076.2-4.

A second aspect of the present invention provides a conjugate for inhibiting APP gene expression, wherein the conjugate comprises any of the described siRNA or its pharmaceutically acceptable salt, and either a targeted delivery ligand linked to the siRNA, or a targeted delivery carrier encapsulating the siRNA.

The third aspect of the present invention provides the use of any of the above-mentioned siRNA or its pharmaceutically acceptable salt, or any of the above-mentioned conjugates, in the preparation of biologicals or pharmaceuticals for inhibiting APP expression.

The fourth aspect of the present invention provides a method for inhibiting APP gene expression, comprising:
acceptable salt, or any of the aforementioned conjugates; and step (a) for a duration sufficient to allow degradation of mRNA transcripts expressing the APP, thereby simultaneously inhibiting APP expression in the cells.

The fifth aspect of the present invention is to provide the application of any of the aforementioned siRNA or any of the aforementioned conjugates in the preparation of drugs for preventing or treating diseases mediated by the APP gene.

The sixth aspect of the present invention provides a method for preventing or treating diseases mediated by the APP gene, wherein an appropriate dose of any of the aforementioned siRNA for inhibiting the expression of the amyloid precursor protein (APP) gene or its pharmaceutically acceptable salt, or any of the aforementioned conjugates, is administered to the subjects.

Through research on siRNA sequences that suppress APP gene expression, the present invention has identified multiple siRNAs capable of effectively inhibiting APP expression. Building upon this foundation, the siRNA sequences have undergone appropriate modifications to enhance target silencing activity while reducing off-target activity.

Another aspect of the present invention further employs a novel and unique delivery system to enable the siRNA to be delivered to the brain of the patient for active therapeutic effects. Therefore, the siRNA in the present invention holds promise for clinical application in the prevention and treatment of diseases associated with the APP target, including cerebral amyloid angiopathy (CAA), early-onset familial Alzheimer's disease (EOFAD), or Alzheimer's disease (AD).

### DESCRIPTION OF EMBODIMENTS

For ease of understanding of the present invention, the present invention will be described more fully below. The present invention may be implemented in many different forms and is not limited to the embodiments described herein. Rather, these embodiments are provided for a more thorough and complete understanding of the disclosure of the present invention.

The experimental methods in the following embodiments, where experimental conditions are not specified, are generally carried out under routine conditions, such as those described in Sambrook et al., Molecular Cloning: A Laboratory Manual (New York: Cold Spring Harbor Laboratory Press, 1989), or as recommended by the manufacturer. The common chemical reagents used in the embodiments are all commercially available products.

Unless otherwise defined, all technical and scientific terms used in the present invention have the same meaning as those commonly understood by those skilled in the art of the present invention. The terms used in the Description of the present invention are only for the purpose of describing specific embodiments and are not intended to limit the present invention. The term "and/or" as used herein encompasses any and all combinations of one or more of the related listed items. In the nucleic acid sequence expressions, the abbreviations and structures of the nucleotide monomers used are as follows:

| | |
|---|---|
| A | Adenosine-3'-phosphate |
| T | 5'-Methyluridine-3'-phosphate |
| C | Cytidine-3'-phosphate |
| G | Guanosine-3'-phosphate |
| U | Uridine-3'-phosphate |
| dA | 2'-Deoxyadenosine-3'-phosphate |
| dT | 2'-Deoxythymidine-3'-phosphate |
| dC | 2'-Deoxycytidine-3'-phosphate |
| dG | 2'-Deoxyguanosine-3'-phosphate |
| dU | 2'-Deoxyuridine-3'-phosphate |
| mA | 2'-O-methyladenosine-3'-phosphate |
| mC | 2'-O-Methylcytidine-3'-phosphate |
| mG | 2'-O-methylguanosine-3'-phosphate |
| mU | 2'-O-methyluridine-3'-phosphate |
| fA | 2'-Fluoroadenosine-3'-phosphate |
| fC | 2'-Fluorocytidine-3'-phosphate |
| fG | 2'-Fluoroguanosine-3'-phosphate |
| fU | 2'-Fluorouridine-3'-phosphate |
| VPU | 2'-O-methyluridine-5'-(E)-vinylphosphonate-3'-phosphate |
| VPU-S | 2'-S-methyluridine-5'-(E)-vinylphosphonate-3'-phosphate |
| isoGNA-A | Adenosine-isoglycerol nucleotide |
| isoGNA-T | Thymidine-isoglycerol nucleotide |
| isoGNA-C | Cytidine-isoglycerol nucleotide |
| isoGNA-G | Guanosine-isoglycerol nucleotide |
| Invab | Inverted abasic residue |
| * | Phosphorothioate bond |
| Ghd | 2'-O-Hexadecyluridine-3'-phosphate |
| Uhd | 2'-O-hexadecylguanosine-3'-phosphate |

The specific structure is as follows:

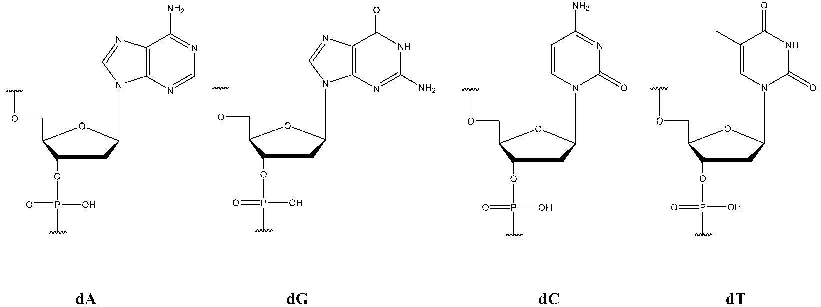

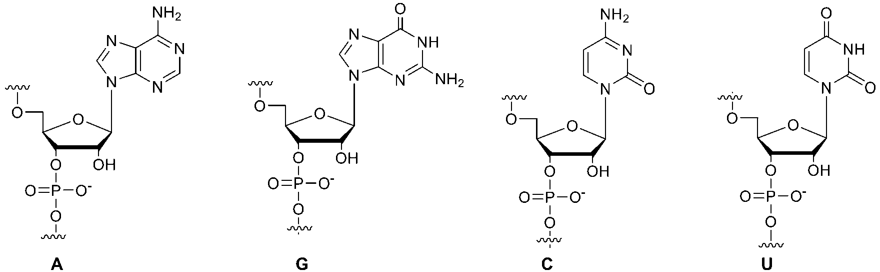

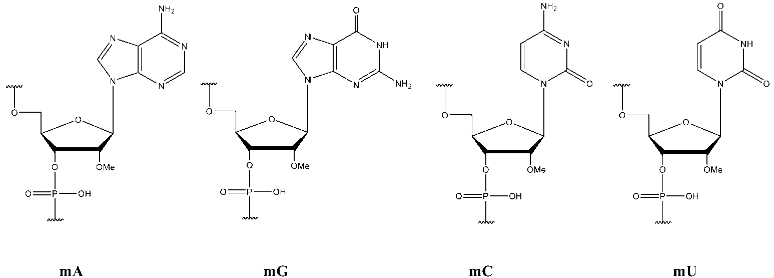

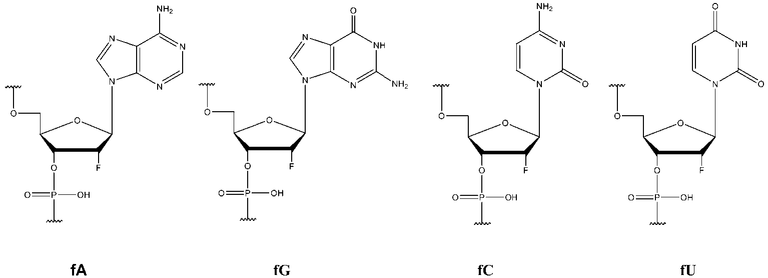

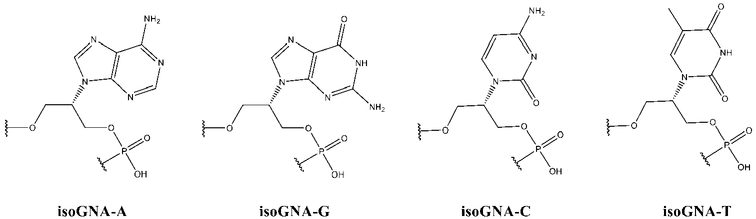

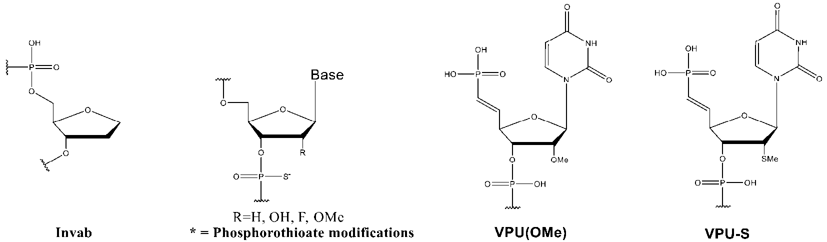

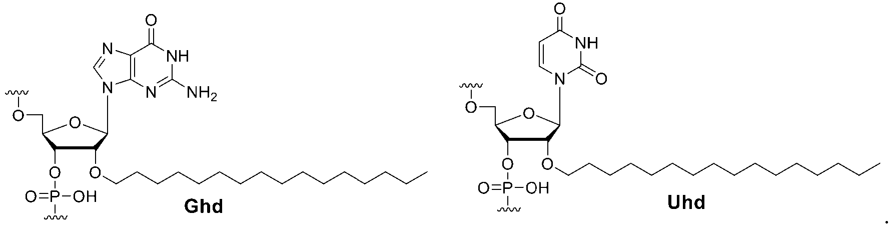

One aspect of the present invention relates to an siRNA or its pharmaceutically acceptable salt for suppressing APP gene expression in human cells, wherein the siRNA comprises a sense strand and an antisense strand, wherein the sense strand comprises at least 15, 16, 17, 18, or 19 consecutive nucleotides, differing by no more than 3 nucleotides from the sequence of any sense strand shown in Table 1; and wherein the antisense strand comprises at least 15, 16, 17, 18, or 19 consecutive nucleotides, differing by no more than 3 nucleotides from the nucleotide sequence of any antisense strand shown in Table 1.

The base composition of the siRNA in the present invention corresponds to each group of sense strands and their respective antisense strands as shown in Table 1, or differs by no more than one nucleotide from the nucleotide sequences of any of the sense strand and its corresponding antisense strand shown in Table 1.

Preferably, the siRNA composition targeting the APP gene is selected from any one of the following sequences, or sequences differing by no more than one nucleotide from its sense strand or antisense strand:
A) 12006: its sense strand is shown in SEQ ID NO: 11, and its antisense strand is shown as SEQ ID NO: 12;
B) 12037: its sense strand is shown in SEQ ID NO: 75, and its antisense strand is shown as SEQ ID NO: 76;
C) 12075: its sense strand is shown in SEQ ID NO: 151, and its antisense strand is shown as SEQ ID NO: 152;
D) 12076: its sense strand is shown in SEQ ID NO: 153, and its antisense strand is shown as SEQ ID NO: 154.

The present invention also provides methods for modifying the aforementioned multi-stranded siRNA to enhance its stability and activity *in vivo* and *in vitro* while reducing off-target activity. The nucleotides of the sense strand and antisense strand are modified, wherein the sense strand comprises no more than 3, 2, 1, or 0 unmodified nucleotides, and the modified nucleotides in the sense strand include those selected from 2'-O-methyl-modified nucleotides, 2'-deoxyribonucleotides, 2'-fluorinated nucleotides, or reverse non-base residues, and the 5'- and 3'-termini of the sense strand contain 0, 1, 2, or 3 phosphorothioate bonds; and the antisense strand includes no more than 3, 2, 1, or 0 unmodified nucleotides, wherein the modified nucleotides in the antisense strand are selected from 2'-O-methyl-modified nucleotides, 2'-deoxyribonucleotides, 2'-fluorinated nucleotides, VPU (2'-O-methyluridine-5'-(E)-vinylphosphate-3'-phosphate), VPU-S (2'-S-methyluridine-5'-(E)-vinylphosphate-3'-phosphate), or other VPU derivatives, and the antisense strand contains 1-3 phosphorothioate bonds at both its 5'- and 3'-termini.

In some embodiments, the modified double-stranded siRNA is selected from any double-stranded siRNA in Table 2, or a sequence differing from its sense or antisense strand by no more than one nucleotide; preferably, the siRNA is selected from any one of the following in Table 2: 12006.1-1, 12006.2-4, 12037.1-1, 12037.2-4, 12075.1-1, 12075.2-4, 12076.1-1, and 12076.2-4.

A modified siRNA exhibiting enhanced biological activity for suppressing APP gene expression, selected from one of the following sequences or sequences differing by no more than one nucleotide from its sense or antisense strand:
a) 12006.2-4: Its 5'-3' sense strand is mC*mA*mCmAmAmGmUmUfCfUfUfUmGfAmGmCmAmGmAmUmA, and its 5'-3' antisense strand is VPU-S*fA*mUmCmUmGmCmUmCmAmAdAmGfAmAfCmUmUmG*mU*mG;
b) 12037.2-4: Its 5'-3' sense strand is mG*mC*mUmGmUmCmCmAfAfGfAfUmGfCmAmGmCmAmGmAmA, and its 5'-3' antisense strand is VPU-S*fU*mCmUmGmCmUmGmCmAmUdCmUfUmGfGmAmCmA*mG*mC;
c) 12075.2-4: Its 5'-3' sense strand is mC*mA*mAmGmUmCmUmAfCfCfCfUmGfAmAmCmUmGmCmAmA, and its 5'-3' antisense strand is VPU-S*fU*mGmCmAmGmUmUmCmAmGdGmGfUmAfGmAmCmU*mU*mG;
d) 12076.2-4: Its 5'-3' sense strand is mG*mG*mUmCmUmAfCfCfCfUmGfAmAmCmUmGmCmAmA, and its 5'-3' antisense strand is VPU-S*fU*mGmCmAmGmUmUmCmAmGdGmGfUmAfGmAmCmC*mU*mC.
wherein VPU-S refers to 2'-S-methyluridine-5'-(E)-vinylphosphonate-3'-phosphate, mA refers to 2'-O-methyladenosine-3'-phosphate, mU refers to 2'-O-methyluridine-3'-phosphate, mC refers to 2'-O-methylcytidine-3'-phosphate, mG refers to 2'-O-methylguanosine-3'-phosphate, fA refers to 2'-fluoroadenosine-3'-phosphate, fU refers to 2'-fluorouridine-3'-phosphate, fC refers to 2'-fluorocytidine-3'-phosphate, fG refers to 2'-fluoroguanosine-3'-phosphate, dA refers to 2'-deoxyadenosine-3'-phosphate, dT refers to 2'-deoxythymidine-3'-phosphate, dC refers to 2'-deoxycytidine-3'-phosphate, dG refers to 2'-deoxyguanosine-3'-phosphate, and * refers to a phosphorothioate bond.

A second aspect of the present invention provides a conjugate for inhibiting APP gene expression, wherein the conjugate comprises any of the above described siRNA or its pharmaceutically acceptable salt, and either a targeted delivery ligand linked to the siRNA, or a targeted delivery carrier encapsulating the siRNA.

The targeted delivery carrier encapsulating the siRNA may be a lipid nanoparticle, such as LNP (Lipid Nanoparticle).

In some embodiments, the targeted delivery ligand comprises at least one peptide targeting low-density lipoprotein receptor-related protein 1 (LRP1) or transferrin receptor (TfR), and a compound (linker) connecting the peptide to any of the siRNAs.

One terminus of the linker may be chemically bonded, such as by a covalent bond, to a peptide, while the other terminus may also be chemically bonded, such as by a covalent bond, to an siRNA.

In some embodiments, siRNA is delivered to the CNS via peptides targeting LRP1 or TfR.

In some embodiments, the peptide targeting LRP1 comprises at least one ANG2 peptide. In some preferred embodiments, the N-to-C amino acid sequence of ANG2 peptide is as follows: TFFYGGSRGKRNNFKTEEY (SEQ ID NO: 185). At least one ANG2 peptide, comprising one, two, three, four, or more ANG2 peptides. In some embodiments, the targeted delivery ligand comprises two ANG2 peptides, which exhibit improved efficacy in maintaining siRNA activity.

When two ANG2 peptides are present, they are connected in parallel to the terminal groups at both termini of the linker, meaning the two ANG2 peptides are connected in parallel to the two terminal groups of the linker.

Those skilled in the art may also select other suitable peptides targeting LRP1 as needed, such as TFFYGGCRGKRNNFKTEEY (SEQ ID NO: 186).

In some embodiments, the linkage site between the linker and the ANG2 peptide is at the N-terminus or the amino group of the lysine side chain of each peptide; preferably, the compound is connected to lysine at position 10 of each ANG2 peptide.

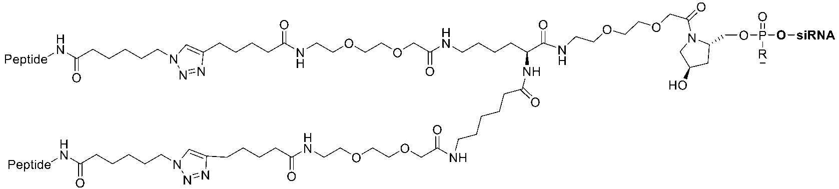

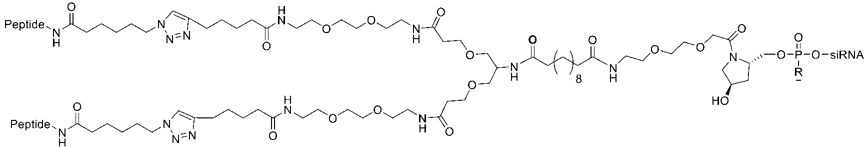

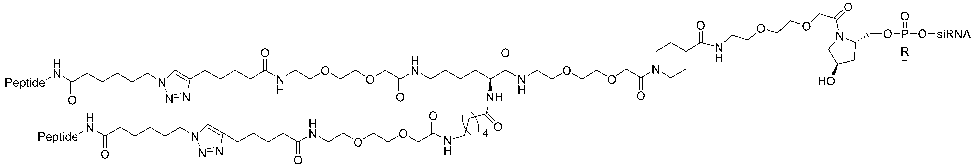

In some embodiments, the linker is chemically bonded to either the 3' or 5'-termini of the siRNA's sense strand, preferably via a phosphodiester bond or a phosphorothioate bond, respectively.

In some embodiments, the structure of the linker connecting the peptide and siRNA is selected from any of the following:

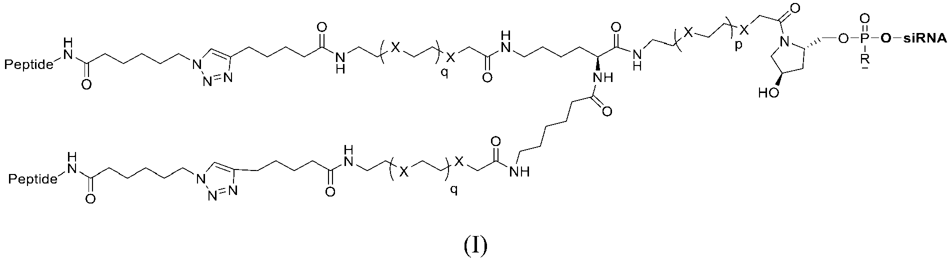

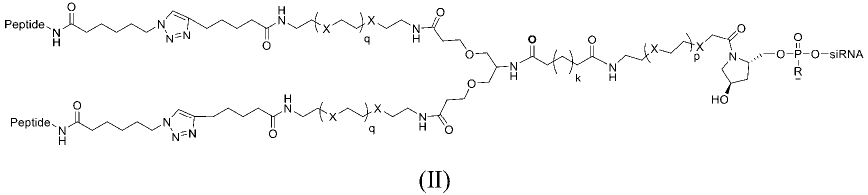

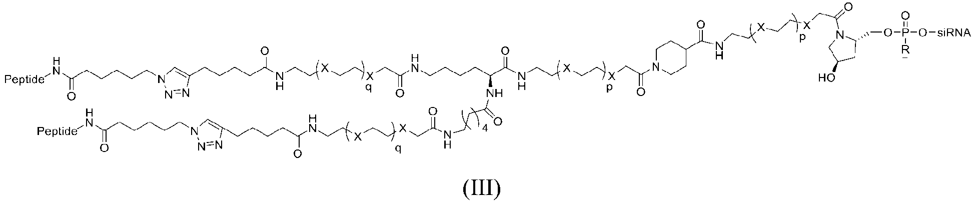

Wherein X and Y are O or CH₂; R is O or S; p and q are integers from 0 to 4; and k is 1, 2, 4, or 8. In some preferred embodiments, the structure of the linker connecting the peptide and siRNA is selected from any of the following:

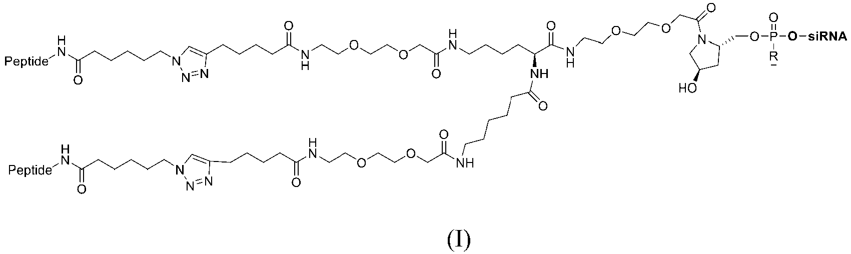

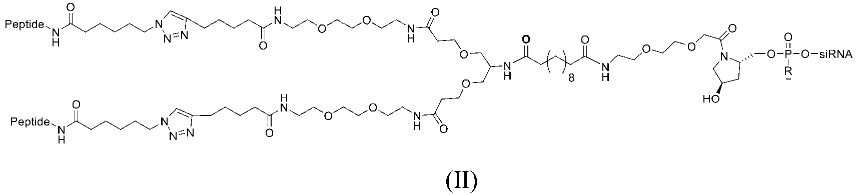

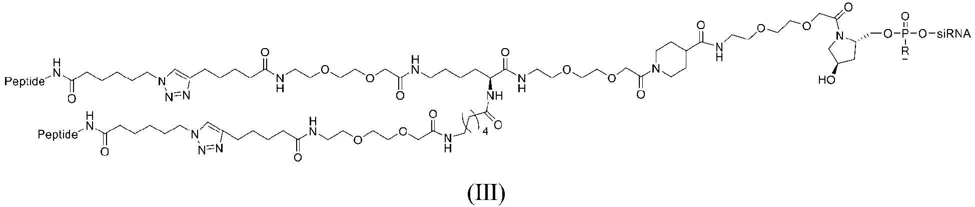

In some embodiments, the targeted delivery ligand structure of the ANG2 peptide linked to siRNA via a linker is any of the following:

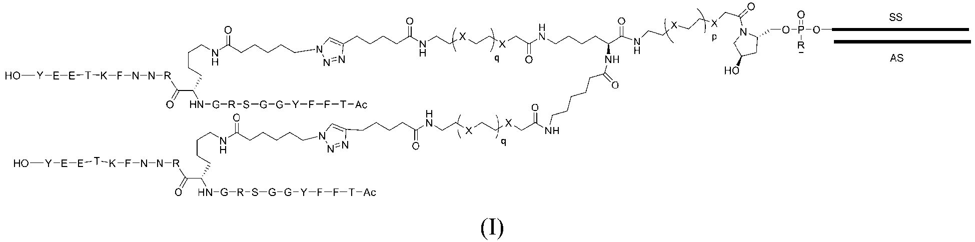

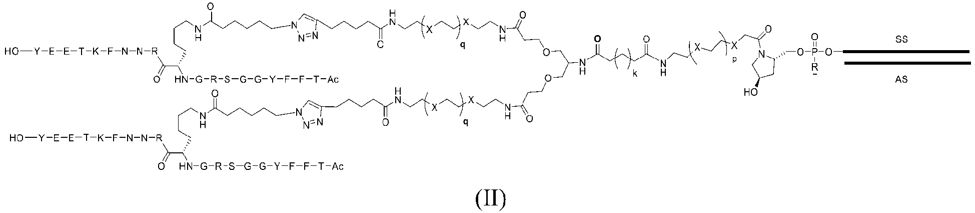

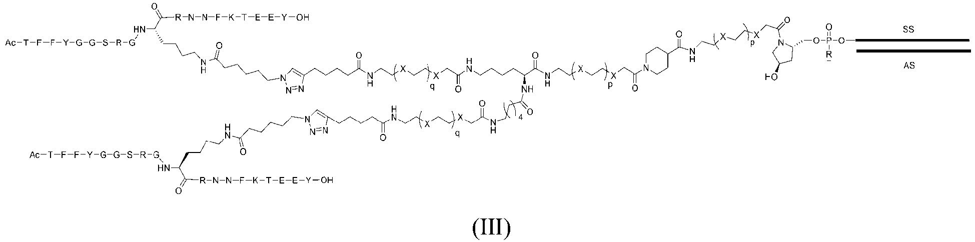

Wherein X and Y are O or CH₂; R is O or S; P and q are integers from 0 to 4; and k is 1, 2, 4, or 8. In some preferred embodiments exhibiting enhanced delivery efficacy, the structure of the targeted delivery ligand formed by linking the ANG2 peptide to the siRNA via a linker is any one of the following: 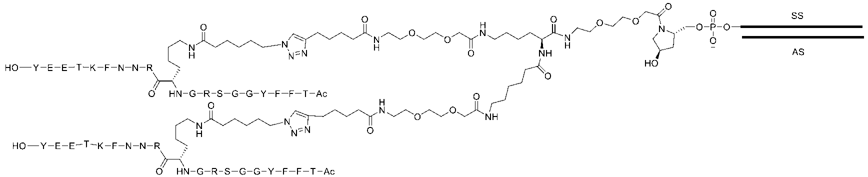 BiAng2(K10N₃)-B08-siRNA 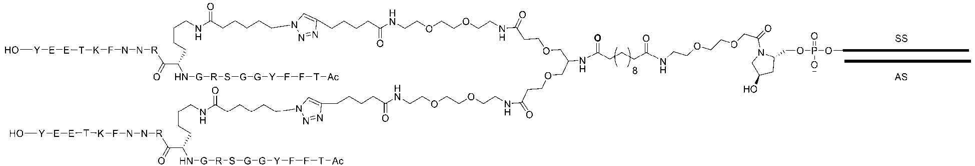 Bi-Sym-Ang2(K10N₃)-siRNA 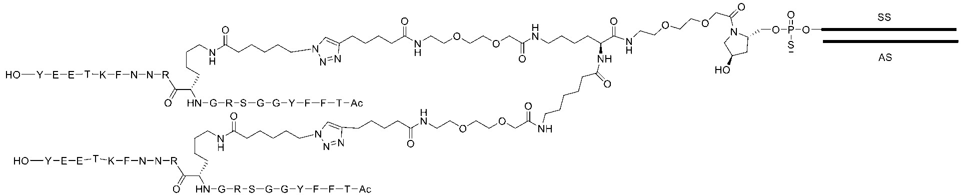 BiAng2(K10N₃)-B08-siRNA-2 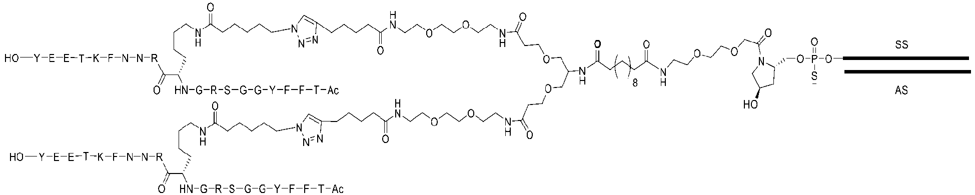 Bi-Sym-Ang2(K10N₃)-siRNA-2 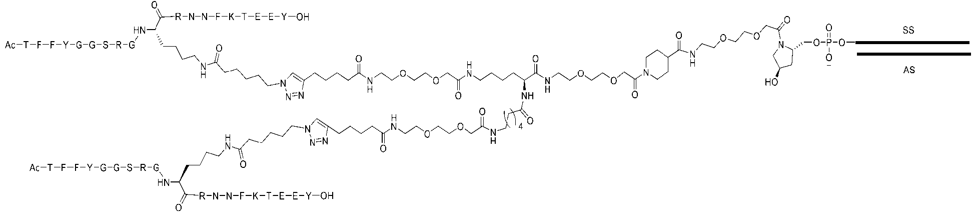 BiAng2(K10N₃)-Q22-siRNA 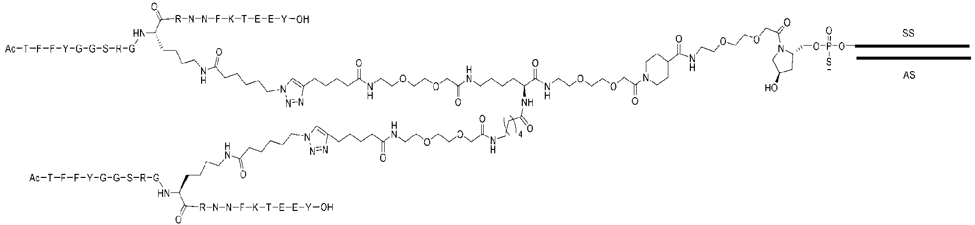 BiAng2(K10N₃)-Q22-siRNA-2.

In some embodiments of the present invention, the application of the siRNA or its pharmaceutically acceptable salts, or the conjugates, in the preparation of biologicals or pharmaceuticals for inhibiting APP gene expression is involved.

In some embodiments of the present invention, a method for inhibiting APP gene expression within cells is provided, comprising: pharmaceutically acceptable salt, or any conjugate; and for a duration sufficient to allow degradation of mRNA transcripts expressing the APP gene, thereby simultaneously suppressing APP gene expression within the cells.

According to conventional understanding in the field, contact may be made directly or indirectly. For example, an individual performing the method may bring siRNA or siRNA conjugates into physical contact with cells, or siRNA or conjugates may be placed in a situation that allows or results in their subsequent contact with cells.

In some embodiments, the cells are within the subject's body, or the cells are *ex vivo* cells from the subject.

In some embodiments, the subjects are mammals, including primates (e.g., humans, non-human primates such as monkeys and chimpanzees), non-primates, and preferably, the mammals are humans, rats, or mice. 40%, 50%, 60%, 70%, 80%, 90%, 95%, 98%, or 100%. invention, the application of the siRNA or any of the aforementioned conjugates in the preparation of drugs for preventing or treating diseases mediated by the APP gene is involved.

In some embodiments, the APP gene-mediated diseases include but are not limited to cerebral amyloid angiopathy (CAA), early-onset familial Alzheimer's disease (EOFAD), or Alzheimer's disease (AD).

The sixth aspect of the present invention provides a method for preventing or treating diseases mediated by the APP gene, wherein an appropriate dose of any of the aforementioned siRNA for inhibiting the expression of the APP gene or its pharmaceutically acceptable salt, or any of the aforementioned conjugates, is administered to the subjects.

The term "appropriate dose" as described herein also refers to a "therapeutically effective dose" when administered to individuals with APP-related conditions, sufficient to achieve treatment of the disease (e.g., by reducing, improving, or maintaining one or more symptoms of the existing disease or condition). The "therapeutically effective dose" may vary depending on the siRNA or conjugate, the mode of administration, the disease and its severity, medical history, age, weight, family history, genetic makeup, the type of prior or concomitant therapy (if any), and other individual characteristics of the subject to be treated.

As used herein, "prophylactically effective dose" is intended to include an amount of siRNA or conjugate sufficient to prevent or improve the disease or one or more symptoms thereof when administered to an individual suffering from an APP-related disorder, wherein improvement includes slowing the progression of the disease or reducing the severity of the disease as it subsequently develops. The "prophylactically effective dose" may vary depending on the siRNA or conjugate, the mode of drug administration, the level of disease risk, medical history, age, weight, family history, genetic makeup, the type of prior or concomitant therapy, and any other individual characteristics of the patient to be treated.

The "therapeutically effective dose" or "prophylactically effective dose" also includes the amount of a drug comprising siRNA or a conjugate that produces certain desired local or systemic effects at a reasonable benefit/risk ratio applicable to any treatment. The siRNA or conjugate-based drug used in the method of the present application may be administered in an amount sufficient to produce a reasonable benefit/risk ratio applicable to such therapy.

According to those skilled in the art, based on existing technology, the pharmaceutically acceptable salt of the siRNA may be a sodium salt or a potassium salt, such as the sodium salt of siRNA generated during purification.

Human APP mRNA NCBI Reference Sequence: NM_000484.4.

Mouse APP mRNA NCBI Reference Sequence: NM_001198823.

Rat APP mRNA NCBI Reference Sequence: NM_019288.

The present invention will be further described in detail below with reference to specific embodiments.

### Embodiment 1 Synthesis of siRNA

Oligonucleotide synthesis at a scale of 0.2-1 µmol was performed on a 12-channel nucleic acid synthesizer (Beijing Tsingke Biotech Co., Ltd.) using a solid-phase oligonucleotide synthesis protocol. An ammonolysis reagent was added to the synthesized oligonucleotide and the mixture was incubated at 45-80°C to separate the oligonucleotide from the solid support, releasing it into solution. The crude oligonucleotide was then precipitated with ethanol. After high-speed centrifugation, the supernatant was discarded. This was repeated twice to give the crude oligonucleotide and the precipitate was resuspended in DEPC-treated water. The crude oligonucleotide was purified by ion-pairing HPLC, and the collected product was dried to powder in a vacuum centrifugal dryer. The purified product was dissolved in DEPC-treated water and analyzed by TOF LC-MS. The concentration of the oligonucleotide was determined. The volumes required for equimolar amounts of the sense and antisense strands were calculated. The equimolar amounts of the sense and antisense strands were mixed well and annealed (heated at 95°C for 5 min, then naturally cooled to room temperature) to prepare a duplex.

**Table 1: Sequences of Sense and Antisense Strands of Unmodified siRNA Targeting the APP Gene**

| Sense Strand Name | Sense strand sequence 5'-3' (SEQ ID NO.) | Antisense Strand Name | Antisense strand sequence 5'-3' (SEQ ID NO.) |
|---|---|---|---|
| 12001-SS | GGCAGAACGGCUACGAAAA UA (1) | 12001-AS | UAUUUUCGUAGCCGUUCUGC C (2) |
| 12002-SS | GGCAGAACGGCUACGAAAA UA (3) | 12002-AS | UAUUUUCGUAGCCGUUCUGC UGC (4) |
| 12003-SS | GCCUACAAGUUCUUUGAGA (5) | 12003-AS | UCUCAAAGAACUUGUAGGCU G(6) |
| 12004-SS | CCCACAAGUUCUUUGAGCA (7) | 12004-AS | UGCUCAAAGAACUUGUCGGU U (8) |
| 12005-SS | CACAAGUUCUUUGAGCAGA (9) | 12005-AS | UCUGCUCAAAGAACUUGUGG G (10) |
| 12006-SS | CACAAGUUCUUUGAGCAGA UA (11) | 12006-AS | UAUCUGCUCAAAGAACUUGU G (12) |
| 12006a-SS | CACAAGUUCUUUGAGCAAA UA (13) | 12006a-A S | UAUUUGCUCAAAGAACUUGU G (14) |
| 12007-SS | CCACAAGUUCUUUGAGCAG AA (15) | 12007-AS | UUCUGCUCAAAGAACUUGUG G (16) |
| 12008-SS | CUAUGAGCGCAUGAAUCAG UA (17) | 12008-AS | UACUGAUUCAUGCGCUCAUA G (18) |
| 12009-SS | GCCGUGUGAUUUAUGAGCG CA (19) | 12009-AS | UGCGCUCAUAAAUCACACGGC (20) |
| 12010-SS | CCCCGUGUGAUUUAUGAGC GA (21) | 12010-AS | UCGCUCAUAAAUCACACGGG GGU (22) |
| 12011-SS | CAUGACACACCUCCGUGUG AA (23) | 12011-AS | UUCACACGGAGGUGUGUCAU G (24) |
| 12012-SS | CCGUAAAGAAUUUAGCUGU AA (25) | 12012-AS | UUACAGCUAAAUUCUUUACG G (26) |
| 12013-SS | CGGGUUUGUGUACUGUAA AA (27) | 12013-AS | UUUUACAGUACACAAAACCC G (28) |
| 12014-SS | CAUCCAGCAUUUCCAGGAG AA (29) | 12014-AS | UUCUCCUGGAAAUGCUGGAU GAC (30) |
| 12015-SS | [?] CUAUCCAGCAUUUCCAGGA GA (31) | 12015-AS | [?] UCUCCUGGAAAUGCUGGAUA GCU (32) |
| 12016-SS | CAUCAAACUAGUGCAUGAA UA (33) | 12016-AS | UAUUCAUGCACUAGUUUGAU G (34) |
| 12017-SS | CCGUAUCAAACUAGUGCAU GA (35) | 12017-AS | UCAUGCACUAGUUUGAUACG G (36) |
| 12018-SS | CGGUGAGUUUGUAAGUGAU GA (37) | 12018-AS | UCAUCACUUACAAACUCACCG (38) |
| 12019-SS | CAGUUUGUAAGUGAUGCCC UA (39) | 12019-AS | UAGGGCAUCACUUACAAACU G (40) |
| 12020-SS | CGCUUAGUUGGUGAGUUUG UA (41) | 12020-AS | UACAAACUCACCAACUAAGCG (42) |
| 12021-SS | GGGAUAUGAAGUUCAUCAU CA (43) | 12021-AS | UGAUGAUGAACUUCAUAUCC C (44) |
| 12022-SS | ACGACUCAGGAUAUGAAGU UA (45) | 12022-AS | UAACUUCAUAUCCUGAGUCG U (46) |
| 12023-SS | CGAACUGCAGAUCACCAAU GA (47) | 12023-AS | UCAUUGGUGAUCUGCAGUUC G (48) |
| 12024-SS | CUACCCUGAACUGCAGAUC AA (49) | 12024-AS | UUGAUCUGCAGUUCAGGUA GGC (50) |
| 12025-SS | GAAGUCUACCCUGAACUGC AA (51) | 12025-AS | UUGCAGUUCAGGUAGACUU CUU (52) |
| 12026-SS | CAGGUACCCACUGAUGGUA AA (53) | 12026-AS | UUUACCAUCAGUGGGUACCU G (54) |
| 12027-SS | GCACCCACUGAUGGUAAUG CA (55) | 12027-AS | UGCAUUACCAUCAGUGGGUG C (56) |
| 12028-SS | GCCAGUGACCAUCCAGAAC UA (57) | 12028-AS | UAGUUCUGGAUGGUCACUGG CUG (58) |
| 12029-SS | GGGAGUGGGAUUCAGAUCC AA (59) | 12029-AS | UUGGAUCUGAAUCCCACUCCC (60) |
| 12030-SS | CCCGACAUGACUCAGGAUA UA(61) | 12030-AS | UAUAUCCUGAGUCAUGUCGG G(62) |
| 12031-SS | CAGUACCAACUUGCAUGAC UA(63) | 12031-AS | UAGUCAUGCAAGUUGGUACU G(64) |
| 12032-SS | GGUGAGAAGAGUACCAACU UA(65) | 12032-AS | UAAGUUGGUACUCUUCUCAC C(66) |
| 12033-SS | CAGACAUGCAGUGAGAAGA GA(67) | 12033-AS | UCUCUUCUCACUGCAUGUCUG (68) |
| 12034-SS | UAUGCCCAUUUCCAGAAAA( 69) | 12034-AS | UUUUCUGGAAAUGGGCAUGU U (70) |
| 12035-SS | GCGAAGAUGGAUGCAGAAU UA (71) | 12035-AS | UAAUUCUGCAUCCAUCUUCGC (72) |
| 12036-SS | CGAAGUGAAGAUGGAUGCA GA (73) | 12036-AS | UCUGCAUCCAUCUUCACUUCG (74) |
| 12037-SS | GCUGUCCAAGAUGCAGCAG AA (75) | 12037-AS | UUCUGCUGCAUCUUGGACAG C (76) |
| 12038-SS | GAACCUACAAGUUCUUUGA GA (77) | 12038-AS | UCUCAAAGAACUUGUAGGUU C (78) |
| 12039-SS | GACCUACAAGUUCUUUGAG CA (79) | 12039-AS | UGCUCAAAGAACUUGUAGGU C (80) |
| 12040-SS | CCCACAAGUUCUUUGAGCA GA (81) | 12040-AS | UCUGCUCAAAGAACUUGUGG G (82) |
| 12041-SS | GAAAUCCAACCUACAAGUU CA (83) | 12041-AS | UGAACUUGUAGGUUGGAUUU C (84) |
| 12042-SS | GCAGGAGAAAGUGGAAUCU UA (85) | 12042-AS | UAAGAUUCCACUUUCUCCUGC (86) |
| 12043-SS | CACUCAGGAUAUGAAGUUC AA (87) | 12043-AS | UUGAACUUCAUAUCCUGAGU G (88) |
| 12044-SS | CCACCCUGAACUGCAGAUC AA (89) | 12044-AS | UUGAUCUGCAGUUCAGGGUG G (90) |
| 12045-SS | GACCAAUGUGGUAGAAGCC AA (91) | 12045-AS | UUGGCUUCUACCACAUUGGU C (92) |
| 12046-SS | GUGCACAUGAAUGUCCAGA AA (93) | 12046-AS | UUUCUGGACAUUCAUGUGCA C (94) |
| 12047-SS | CCGCCAAGAAGUCUACCCU GA (95) | 12047-AS | UCAGGGUAGACUUCUUGGCG G (96) |
| 12048-SS | GAGCAAAGAACUUGCCUAA AA (97) | 12048-AS | UUUUAGGCAAGUUCUUUGCU C (98) |
| 12049-SS | CAAGAAGGCAGUUAUCCAG CA (99) | 12049-AS | UGCUGGAUAACUGCCUUCUU G (100) |
| 12050-SS | GCUCAAUAUGCUAAAGAAG UA (101) | 12050-AS | UACUUCUUUAGCAUAUUGAG C (102) |
| 12051-SS | GAGAGCAAAACUAUUCAGA UA (103) | 12051-AS | UAUCUGAAUAGUUUUGCUCU C (104) |
| 12052-SS | CGAUGAGCUGCUUCAGAAA GA (105) | 12052-AS | UCUUUCUGAAGCAGCUCAUC G (106) |
| 12053-SS | GGCAAAACUAUUCAGAUGA CA (107) | 12053-AS | UGUCAUCUGAAUAGUUUUGC C (108) |
| 12054-SS | CACGGAAACGAUGCUCUCA UA (109) | 12054-AS | UAUGAGAGCAUCGUUUCCGU G (110) |
| 12055-SS | CCCUCAUGCCAUCUUUGAC CA (111) | 12055-AS | UGGUCAAAGAUGGCAUGAGG G (112) |
| 12056-SS | GGAUGCUCUCAUGCCAUCU UA (113) | 12056-AS | UAAGAUGGCAUGAGAGCAUC C (114) |
| 12057-SS | GGGUUCUGGGUUGACAAAU AA (115) | 12057-AS | UUAUUUGUCAACCCAGAACCC (116) |
| 12058-SS | CGAUAUGAAGUUCAUCAUC AA (117) | 12058-AS | UUGAUGAUGAACUUCAUAUC G (118) |
| 12059-SS | GGAGUUCAUCAUCAAAAAU UA (119) | 12059-AS | UAAUUUUUGAUGAUGAACUC C (120) |
| 12060-SS | GUCAUCAAAAUUGGUGUU CA (121) | 12060-AS | UGAACACCAAUUUUUGAUGA C (122) |
| 12061-SS | CCAUCAAAAUUGGUGUUC UA (123) | 12061-AS | UAGAACACCAAUUUUUGAUG G (124) |
| 12062-SS | GAUGGGUUUUGUGUACUGU AA (125) | 12062-AS | UUACAGUACACAAAACCCAUC (126) |
| 12063-SS | CUAAUGGGUUUUGUGUACU GA (127) | 12063-AS | UCAGUACACAAAACCCAUUA G (128) |
| 12064-SS | CAUUAAUGGGUUUUGUGUA CA (129) | 12064-AS | UGUACACAAAACCCAUUAAU G (130) |
| 12065-SS | CUAUUAAUGGGUUUUGUGU AA (131) | 12065-AS | UUACACAAAACCCAUUAAUA G (132) |
| 12066-SS | CACAUUAUUAAUGGGUUUU GA (133) | 12066-AS | UCAAAACCCAUUAAUAAUGU G (134) |
| 12067-SS | CGUAAAGAAUUUAGCUGUA UA (135) | 12067-AS | UAUACAGCUAAAUUCUUUAC G (136) |
| 12068-SS | CGAAUAGAUUCUCUCCUGA UA (137) | 12068-AS | UAUCAGGAGAGAAUCUAUUC G (138) |
| 12069-SS | GCCCAAUUAAGUCCUACUU UA (139) | 12069-AS | UAAAGUAGGACUUAAUUGGG C (140) |
| 12070-SS | CGAUCGCUUUCUACACUGU AA (141) | 12070-AS | UUACAGUGUAGAAAGCGAUC G (142) |
| 12071-SS | CCUUUCUACACUGUAUUAC AA (143) | 12071-AS | UUGUAAUACAGUGUAGAAAG G (144) |
| 12072-SS | CAACCAACCAGUGACCAUA (145) | 12072-AS | UGGAUGGUCACUGGUUGGUU G (146) |
| 12073-SS | CCCGAACUGCAGAUCACCA AA (147) | 12073-AS | UCAUUGGUGAUCUGCAGUUC GGG (148) |
| 12074-SS | CUGCCCUGAACUGCAGAUA (149) | 12074-AS | UUGAUCUGCAGUUCAGGGCA G (150) |
| 12075-SS | CAAGUCUACCCUGAACUGC AA (151) | 12075-AS | UUGCAGUUCAGGGUAGACUU G (152) |
| 12076-SS | GGUCUACCCUGAACUGCAA (153) | 12076-AS | UUGCAGUUCAGGUAGACCU C (154) |
| 12077-SS | UAGAAGUUCUUUGAGCAGA (155) | 12077-AS | UAUCUGCUCAAAGAACUUCU A (156) |
| 12078-SS | CCCACAAGUUCUUUGAGCA GA (157) | 12078-AS | UAUCUGCUCAAAGAACUUGU GGG (158) |
| 12079-SS | AACAAGUCUACCCUGAACU GA (159) | 12079-AS | UUGCAGUUCAGGGUAGACUU GUU (160) |
| 12080-SS | CACCAAUGUGGUAGAAGCA (161) | 12080-AS | UUGGCUUCUACCACAUUGGU G (162) |
| 12075a-SS | GAGGUCUACCCUGAACUGC AA (163) | 12075a-A S | UUGCAGUUCAGGGUAGACCU C (164) |
| 12006b-S S | UACAAGUUCUUUGAGCAGA UA (165) | 12006b-A S | UAUCUGCUCAAAGAACUUGU AGG (166) |
| 12006c-SS | CAAGUUCUUUGAGCAGAUA (167) | 12006c-A S | UAUCUGCUCAAAGAACUUGU A (168) |
| 12037a-SS | CCUGUCCAAGAUGCAGCAG AA (169) | 12037a-A S | UUCUGCUGCAUCUUGGACAG GUG (170) |
| 12037b-S S | UGUCCAAGAUGCAGCAGAA (171) | 12037b-A S | UCUGUCCAAGAUGCAGCAGA A (172) |
| 12075b-S S | GAAGUCUACCCUGAACUGC AA (173) | 12075b-A S | UUGCAGUUCAGGUAGACUU CUU (174) |
| 12075c-SS | AGUCUACCCUGAACUGCAA (175) | 12075c-A S | UUGCAGUUCAGGGUAGACUU C (176) |

Next, we modified the siRNA to enhance its stability both *in vivo* and *in vitro,* increase its activity at the target site, and reduce its activity at non-target sites.

The double-stranded oligonucleotide AD-454973 is the most active sequence in patent US11034957B2. The sense strand modified sequence is mG*mG*mCmUmAmCmGmAdAmAdAmUmCmCmAmAmCmCmU*mA*mA, and the antisense strand modified sequence is VPU*fU*mAmGmGmUGNA-TmGmGmAmUdTmUfUmCdGmUmAmGmCmC*mG*mU, serving as a positive control.

**Table 2: Sequences of Modified siRNA Targeting the APP Gene**

| **Duple x Name** | **Modified Sense Strand Name** | **Modified Sense Strand Sequence 5'-3'** | **Modified Antisense Strand Name** | **Modified Antisense Strand Sequence 5'-3'** |
|---|---|---|---|---|
| 12001. 1-1 | 12001SM 1 | mG*mG*mCmAmGmAfA mCfGfGdCmUmAmCmG mAmAmAmAmUmA | 12001AM 1 | VPU*fA*mUmUmUmUmCmG mUmAmGdCmCfGmUfUmCm UmG*mC*mC |
| 12002. 1-1 | 12002SM 1 | mG*mG*mCmAmGmAfA mCfGfGdCmUmAmCmG mAmAmAmAmUmA | 12002AM 1 | VPU*fA*mUmUmUmUmCmG mUmAmGdCmCfGmUfUmCm UmGmCmU*mG*mC |
| 12003. 1-1 | 12003SM 1 | mG*mC*mCmUfAmCfAf AdGmUmUmCmUmUmU mGmAmGmA | 12003AM 1 | VPU*fC*mUmCmAmAmAmG mAmAmCdTmUfGmUfAmGm GmC*mU*mG |
| 12004. 1-1 | 12004SM 1 | mC*mC*mCmAfCmAfAf GdTmUmCmUmUmUmG mAmGmCmA | 12004AM 1 | VPU*fG*mCmUmCmAmAmA mGmAmAdCmUfUmGfUmCm GmG*mU*mU |
| 12005. 1-1 | 12005SM 1 | mC*mA*mCmAfAmGfUf UdCmUmUmUmGmAmG mCmAmGmA | 12005AM 1 | VPU*fC*mUmGmCmUmCmA mAmAmGdAmAfCmUfUmGm UmG*mG*mG |
| 12006. 1-1 | 12006SM 1 | mC*mA*mCmAmAmGfU mUfCfUdTmUmGmAmG mCmAmGmAmUmA | 12006AM 1 | VPU*fA*mUmCmUmGmCmU mCmAmAdAmGfAmAfCmUm UmG*mU*mG |
| 12006 a.1-1 | 12006aS M1 | mC*mA*mCmAmAmGfU mUfCfUdTmUmGmAmG mCmAmAmAmUmA | 12006aA M1 | VPU*fA*mUmUmUmGmCmU mCmAmAdAmGfAmAfCmUm UmG*mU*mG |
| 12007. 1-1 | 12007SM 1 | mC*mC*mAmCmAmAfG mUfUfCdTmUmUmGmA mGmCmAmGmAmA | 12007AM 1 | VPU*fU*mCmUmGmCmUmC mAmAmAdGmAfAmCfUmUm GmU*mG*mG |
| 12014. 1-1 | 12014SM 1 | mC*mA*mUmCmCmAfG mCfAfUdTmUmCmCmA mGmGmAmGmAmA | 12014AM 1 | VPU*fU*mCmUmCmCmUmG mGmAmAdAmUfGmCfUmGm GmAmUmG*mA*mC |
| 12015. 1-1 | 12015SM 1 | mC*mU*mAmUmCmCfA mGfCfAdTmUmUmCmC mAmGmGmAmGmA | 12015AM 1 | VPU*fC*mUmCmCmUmGmG mAmAmAdTmGfCmUfGmGm AmUmAmG*mC*mU |
| 12023. 1-1 | 12023SM 1 | mC*mG*mAmAmCmUfG mCfAfGdAmUmCmAmC mCmAmAmUmGmA | 12023AM 1 | VPU*fC*mAmUmUmGmGmU mGmAmUdCmUfGmCfAmGm UmU*mC*mG |
| 12024. 1-1 | 12024SM 1 | mC*mU*mAmCmCmCfU mGfAfAdCmUmGmCmA mGmAmUmCmAmA | 12024AM 1 | VPU*fU*mGmAmUmCmUmG mCmAmGdTmUfCmAfGmGm GmUmAmG*mG*mC |
| 12025. 1-1 | 12025SM 1 | mG*mA*mAmGmUmCfU mAfCfCdCmUmGmAmA mCmUmGmCmAmA | 12025AM 1 | VPU*fU*mGmCmAmGmUmU mCmAmGdGmGfUmAfGmAm CmUmUmC*mU*mU |
| 12028. 1-1 | 12028SM 1 | mG*mC*mCmAmGmUfG mAfCfCdAmUmCmCmA mGmAmAmCmUmA | 12028AM 1 | VPU*fA*mGmUmUmCmUmG mGmAmUdGmGfUmCfAmCm UmGmGmC*mU*mG |
| 12034. 1-1 | 12034SM 1 | mU*mA*mUmGfCmCfCf AdTmUmUmCmCmAmG mAmAmAmA | 12034AM 1 | VPU*fU*mUmUmCmUmGmG mAmAmAdTmGfGmGfCmAm UmG*mU*mU |
| 12037. 1-1 | 12037SM 1 | mG*mC*mUmGmUmCfC mAfAfGdAmUmGmCmA mGmCmAmGmAmA | 12037AM 1 | VPU*fU*mCmUmGmCmUmG mCmAmUdCmUfUmGfGmAm CmA*mG*mC |
| 12038. 1-1 | 12038SM 1 | mG*mA*mAmCmCmUfA mCfAfAdGmUmUmCmU mUmUmGmAmGmA | 12038AM 1 | VPU*fC*mUmCmAmAmAmG mAmAmCdTmUfGmUfAmGm GmU*mU*mC |
| 12039. 1-1 | 12039SM 1 | mG*mA*mCmCmUmAfC mAfAfGdTmUmCmUmU mUmGmAmGmCmA | 12039AM 1 | VPU*fG*mCmUmCmAmAmA mGmAmAdCmUfUmGfUmAm GmG*mU*mC |
| 12040. 1-1 | 12040SM 1 | mC*mC*mCmAmCmAfA mGfUfUdCmUmUmUmG mAmGmCmAmGmA | 12040AM 1 | VPU*fC*mUmGmCmUmCmA mAmAmGdAmAfCmUfUmGm UmG*mG*mG |
| 12041. 1-1 | 12041SM 1 | mG*mA*mAmAmUmCfC mAfAfCdCmUmAmCmA mAmGmUmUmCmA | 12041AM 1 | VPU*fG*mAmAmCmUmUmG mUmAmGdGmUfUmGfGmAm UmU*mU*mC |
| 12044. 1-1 | 12044SM 1 | mC*mC*mAmCmCmCfU mGfAfAdCmUmGmCmA mGmAmUmCmAmA | 12044AM 1 | VPU*fU*mGmAmUmCmUmG mCmAmGdTmUfCmAfGmGm GmU*mG*mG |
| 12045. 1-1 | 12045SM 1 | mG*mA*mCmCmAmAfU mGfUfGdGmUmAmGmA mAmGmCmCmAmA | 12045AM 1 | VPU*fU*mGmGmCmUmUmC mUmAmCdCmAfCmAfUmUm GmG*mU*mC |
| 12047. 1-1 | 12047SM 1 | mC*mC*mGmCmCmAfA mGfAfAdGmUmCmUmA mCmCmCmUmGmA | 12047AM 1 | VPU*fC*mAmGmGmGmUmA mGmAmCdTmUfCmUfUmGm GmC*mG*mG |
| 12072. 1-1 | 12072SM 1 | mC*mA*mAmCfCmAfAf CdCmAmGmUmGmAmC mCmAmUmA | 12072AM 1 | VPU*fG*mGmAmUmGmGmU mCmAmCdTmGfGmUfUmGm GmU*mU*mG |
| 12073. 1-1 | 12073SM 1 | mC*mC*mCmGmAmAfC mUfGfCdAmGmAmUmC mAmCmCmAmAmA | 12073AM 1 | VPU*fC*mAmUmUmGmGmU mGmAmUdCmUfGmCfAmGm UmUmCmG*mG*mG |
| 12074. 1-1 | 12074SM 1 | mC*mU*mGmCfCmCfUf GdAmAmCmUmGmCmA mGmAmUmA | 12074AM 1 | VPU*fU*mGmAmUmCmUmG mCmAmGdTmUfCmAfGmGm GmC*mA*mG |
| 12075. 1-1 | 12075SM 1 | mC*mA*mAmGmUmCfU mAfCfCdCmUmGmAmA mCmUmGmCmAmA | 12075AM 1 | VPU*fU*mGmCmAmGmUmU mCmAmGdGmGfUmAfGmAm CmU*mU*mG |
| 12075 a.1-1 | 12075aS M1 | mG*mA*mGmGmUmCfU mAfCfCdCmUmGmAmA mCmUmGmCmAmA | 12075aA M1 | VPU*fU*mGmCmAmGmUmU mCmAmGdGmGfUmAfGmAm CmC*mU*mC |
| 12076. 1-1 | 12076SM 1 | mG*mG*mUmCfUmAfCf CdCmUmGmAmAmCmU mGmCmAmA | 12076AM 1 | VPU*fU*mGmCmAmGmUmU mCmAmGdGmGfUmAfGmAm CmC*mU*mC |
| 12077. 1-1 | 12077SM 1 | mU*mA*mGmAfAmGfUf UdCmUmUmUmGmAmG mCmAmGmA | 12077AM 1 | VPU*fA*mUmCmUmGmCmU mCmAmAdAmGfAmAfCmUm UmC*mU*mA |
| 12078. 1-1 | 12078SM 1 | mC*mC*mCmAmCmAfA mGfUfUdCmUmUmUmG mAmGmCmAmGmA | 12078AM 1 | VPU*fA*mUmCmUmGmCmU mCmAmAdAmGfAmAfCmUm UmGmUmG*mG*mG |
| 12080. 1-1 | 12080SM 1 | mC*mA*mCmCfAmAfUf GdTmGmGmUmAmGmA mAmGmCmA | 12080AM 1 | VPU*fU*mGmGmCmUmUmC mUmAmCdCmAfCmAfUmUm GmG*mU*mG |
| 12001. 1-3 | 12001SM 3 | mG*mG*mCmAmGmAm AmCfGmGfCmUfAmCm GmAmAmAmAmUmA | 12001AM 1 | VPU*fA*mUmUmUmUmCmG mUmAmGdCmCfGmUfUmCm UmG*mC*mC |
| 12002. 1-3 | 12002SM 3 | mG*mG*mCmAmGmAm AmCfGmGfCmUfAmCm GmAmAmAmAmUmA | 12002AM 1 | VPU*fA*mUmUmUmUmCmG mUmAmGdCmCfGmUfUmCm UmGmCmU*mG*mC |
| 12003. 1-3 | 12003SM 3 | mG*mC*mCmUmAmCfA mAfGmUfUmCmUmUm UmGmAmGmA | 12003AM 1 | VPU*fC*mUmCmAmAmAmG mAmAmCdTmUfGmUfAmGm GmC*mU*mG |
| 12004. 1-3 | 12004SM 3 | mC*mC*mCmAmCmAfA mGfUmUfCmUmUmUm GmAmGmCmA | 12004AM 1 | VPU*fG*mCmUmCmAmAmA mGmAmAdCmUfUmGfUmCm GmG*mU*mU |
| 12005. 1-3 | 12005SM 3 | mC*mA*mCmAmAmGfU mUfCmUfUmUmGmAm GmCmAmGmA | 12005AM 1 | VPU*fC*mUmGmCmUmCmA mAmAmGdAmAfCmUfUmGm UmG*mG*mG |
| 12006. 1-3 | 12006SM 3 | mC*mA*mCmAmAmGm UmUfCmUfUmUfGmAm GmCmAmGmAmUmA | 12006AM 1 | VPU*fA*mUmCmUmGmCmU mCmAmAdAmGfAmAfCmUm UmG*mU*mG |
| 12006 a.1-3 | 12006aS M3 | mC*mA*mCmAmAmGm UmUfCmUfUmUfGmAm GmCmAmAmAmUmA | 12006aA M1 | VPU*fA*mUmUmUmGmCmU mCmAmAdAmGfAmAfCmUm UmG*mU*mG |
| 12007. 1-3 | 12007SM 3 | mC*mC*mAmCmAmAm GmUfUmCfUmUfUmGm AmGmCmAmGmAmA | 12007AM 1 | VPU*fU*mCmUmGmCmUmC mAmAmAdGmAfAmCfUmUm GmU*mG*mG |
| 12014. 1-3 | 12014SM 3 | mC*mA*mUmCmCmAm GmCfAmUfUmUfCmCm AmGmGmAmGmAmA | 12014AM 1 | VPU*fU*mCmUmCmCmUmG mGmAmAdAmUfGmCfUmGm GmAmUmG*mA*mC |
| 12015. 1-3 | 12015SM 3 | mC*mU*mAmUmCmCm AmGfCmAfUmUfUmCm CmAmGmGmAmGmA | 12015AM 1 | VPU*fC*mUmCmCmUmGmG mAmAmAdTmGfCmUfGmGm AmUmAmG*mC*mU |
| 12023. 1-3 | 12023SM 3 | mC*mG*mAmAmCmUm GmCfAmGfAmUfCmAm CmCmAmAmUmGmA | 12023AM 1 | VPU*fC*mAmUmUmGmGmU mGmAmUdCmUfGmCfAmGm UmU*mC*mG |
| 12024. 1-3 | 12024SM 3 | mC*mU*mAmCmCmCm UmGfAmAfCmUfGmCm AmGmAmUmCmAmA | 12024AM 1 | VPU*fU*mGmAmUmCmUmG mCmAmGdTmUfCmAfGmGm GmUmAmG*mG*mC |
| 12025. 1-3 | 12025SM 3 | mG*mA*mAmGmUmCm UmAfCmCfCmUfGmAm AmCmUmGmCmAmA | 12025AM 1 | VPU*fU*mGmCmAmGmUmU mCmAmGdGmGfUmAfGmAm CmUmUmC*mU*mU |
| 12028. 1-3 | 12028SM 3 | mG*mC*mCmAmGmUm GmAfCmCfAmUfCmCm AmGmAmAmCmUmA | 12028AM 1 | VPU*fA*mGmUmUmCmUmG mGmAmUdGmGfUmCfAmCm UmGmGmC*mU*mG |
| 12034. 1-3 | 12034SM 3 | mU*mA*mUmGmCmCfC mAfUmUfUmCmCmAmG mAmAmAmA | 12034AM 1 | VPU*fU*mUmUmCmUmGmG mAmAmAdTmGfGmGfCmAm UmG*mU*mU |
| 12037. 1-3 | 12037SM 3 | mG*mC*mUmGmUmCm CmAfAmGfAmUfGmCm AmGmCmAmGmAmA | 12037AM 1 | VPU*fU*mCmUmGmCmUmG mCmAmUdCmUfUmGfGmAm CmA*mG*mC |
| 12038. 1-3 | 12038SM 3 | mG*mA*mAmCmCmUm AmCfAmAfGmUfUmCm UmUmUmGmAmGmA | 12038AM 1 | VPU*fC*mUmCmAmAmAmG mAmAmCdTmUfGmUfAmGm GmU*mU*mC |
| 12039. 1-3 | 12039SM 3 | mG*mA*mCmCmUmAm CmAfAmGfUmUfCmUm UmUmGmAmGmCmA | 12039AM 1 | VPU*fG*mCmUmCmAmAmA mGmAmAdCmUfUmGfUmAm GmG*mU*mC |
| 12040. 1-3 | 12040SM 3 | mC*mC*mCmAmCmAm AmGfUmUfCmUfUmUm GmAmGmCmAmGmA | 12040AM 1 | VPU*fC*mUmGmCmUmCmA mAmAmGdAmAfCmUfUmGm UmG*mG*mG |
| 12041. 1-3 | 12041SM 3 | mG*mA*mAmAmUmCm CmAfAmCfCmUfAmCm AmAmGmUmUmCmA | 12041AM 1 | VPU*fG*mAmAmCmUmUmG mUmAmGdGmUfUmGfGmAm UmU*mU*mC |
| 12044. 1-3 | 12044SM 3 | mC*mC*mAmCmCmCm UmGfAmAfCmUfGmCm AmGmAmUmCmAmA | 12044AM 1 | VPU*fU*mGmAmUmCmUmG mCmAmGdTmUfCmAfGmGm GmU*mG*mG |
| 12045. 1-3 | 12045SM 3 | mG*mA*mCmCmAmAm UmGfUmGfGmUfAmGm AmAmGmCmCmAmA | 12045AM 1 | VPU*fU*mGmGmCmUmUmC mUmAmCdCmAfCmAfUmUm GmG*mU*mC |
| 12047. 1-3 | 12047SM 3 | mC*mC*mGmCmCmAm AmGfAmAfGmUfCmUm AmCmCmCmUmGmA | 12047AM 1 | VPU*fC*mAmGmGmGmUmA mGmAmCdTmUfCmUfUmGm GmC*mG*mG |
| 12072. 1-3 | 12072SM 3 | mC*mA*mAmCmCmAfA mCfCmAfGmUmGmAmC mCmAmUmA | 12072AM 1 | VPU*fG*mGmAmUmGmGmU mCmAmCdTmGfGmUfUmGm GmU*mU*mG |
| 12073. 1-3 | 12073SM 3 | mC*mC*mCmGmAmAm CmUfGmCfAmGfAmUm CmAmCmCmAmAmA | 12073AM 1 | VPU*fC*mAmUmUmGmGmU mGmAmUdCmUfGmCfAmGm UmUmCmG*mG*mG |
| 12074. 1-3 | 12074SM 3 | mC*mU*mGmCmCmCfU mGfAmAfCmUmGmCmA mGmAmUmA | 12074AM 1 | VPU*fU*mGmAmUmCmUmG mCmAmGdTmUfCmAfGmGm GmC*mA*mG |
| 12075. 1-3 | 12075SM 3 | mC*mA*mAmGmUmCm UmAfCmCfCmUfGmAm AmCmUmGmCmAmA | 12075AM 1 | VPU*fU*mGmCmAmGmUmU mCmAmGdGmGfUmAfGmAm CmU*mU*mG |
| 12075 a.1-3 | 12075aS M3 | mG*mA*mGmGmUmCm UmAfCmCfCmUfGmAm AmCmUmGmCmAmA | 12075aA M1 | VPU*fU*mGmCmAmGmUmU mCmAmGdGmGfUmAfGmAm CmC*mU*mC |
| 12076. 1-3 | 12076SM 3 | mG*mG*mUmCmUmAfC mCfCmUfGmAmAmCmU mGmCmAmA | 12076AM 1 | VPU*fU*mGmCmAmGmUmU mCmAmGdGmGfUmAfGmAm CmC*mU*mC |
| 12077. 1-3 | 12077SM 3 | mU*mA*mGmAmAmGf UmUfCmUfUmUmGmA mGmCmAmGmA | 12077AM 1 | VPU*fA*mUmCmUmGmCmU mCmAmAdAmGfAmAfCmUm UmC*mU*mA |
| 12078. 1-3 | 12078SM 3 | mC*mC*mCmAmCmAm AmGfUmUfCmUfUmUm GmAmGmCmAmGmA | 12078AM 1 | VPU*fA*mUmCmUmGmCmU mCmAmAdAmGfAmAfCmUm UmGmUmG*mG*mG |
| 12080. 1-3 | 12080SM 3 | mC*mA*mCmCmAmAfU mGfUmGfGmUmAmGm AmAmGmCmA | 12080AM 1 | VPU*fU*mGmGmCmUmUmC mUmAmCdCmAfCmAfUmUm GmG*mU*mG |
| 12001. 2-4 | 12001SM 4 | mG*mG*mCmAmGmAm AmCfGfGfCfUmAfCmG mAmAmAmAmUmA | 12001AM 2 | VPU-S*fA*mUmUmUmUmCm GmUmAmGdCmCfGmUfUmC mUmG*mC*mC |
| 12002. 2-4 | 12002SM 4 | mG*mG*mCmAmGmAm AmCfGfGfCfUmAfCmG mAmAmAmAmUmA | 12002AM 2 | VPU-S*fA*mUmUmUmUmCm GmUmAmGdCmCfGmUfUmC mUmGmCmU*mG*mC |
| 12003. 2-4 | 12003SM 4 | mG*mC*mCmUmAmCfA fAfGfUmUfCmUmUmUm GmAmGmA | 12003AM 2 | VPU-S*fC*mUmCmAmAmAm GmAmAmCdTmUfGmUfAmG mGmC*mU*mG |
| 12004. 2-4 | 12004SM 4 | mC*mC*mCmAmCmAfA fGfUfUmCfUmUmUmGm AmGmCmA | 12004AM 2 | VPU-S*fG*mCmUmCmAmAm AmGmAmAdCmUfUmGfUmC mGmG*mU*mU |
| 12005. 2-4 | 12005SM 4 | mC*mA*mCmAmAmGfU fUfCfUmUfUmGmAmGm CmAmGmA | 12005AM 2 | VPU-S*fC*mUmGmCmUmCm AmAmAmGdAmAfCmUfUmG mUmG*mG*mG |
| 12006. 2-4 | 12006SM 4 | mC*mA*mCmAmAmGm UmUfCfUfUfUmGfAmG mCmAmGmAmUmA | 12006AM 2 | VPU-S*fA*mUmCmUmGmCm UmCmAmAdAmGfAmAfCmU mUmG*mU*mG |
| 12006 a.2-4 | 12006aS M4 | mC*mA*mCmAmAmGm UmUfCfUfUfUmGfAmG mCmAmAmAmUmA | 12006aA M2 | VPU-S*fA*mUmUmUmGmCm UmCmAmAdAmGfAmAfCmU mUmG*mU*mG |
| 12007. 2-4 | 12007SM 4 | mC*mC*mAmCmAmAm GmUfUfCfUfUmUfGmA mGmCmAmGmAmA | 12007AM 2 | VPU-S*fU*mCmUmGmCmUm CmAmAmAdGmAfAmCfUmU mGmU*mG*mG |
| 12014. 2-4 | 12014SM 4 | mC*mA*mUmCmCmAm GmCfAfUfUfUmCfCmA mGmGmAmGmAmA | 12014AM 2 | VPU-S*fU*mCmUmCmCmUm GmGmAmAdAmUfGmCfUmG mGmAmUmG*mA*mC |
| 12015. 2-4 | 12015SM 4 | mC*mU*mAmUmCmCm AmGfCfAfUfUmUfCmC mAmGmGmAmGmA | 12015AM 2 | VPU-S*fC*mUmCmCmUmGm GmAmAmAdTmGfCmUfGmG mAmUmAmG*mC*mU |
| 12023. 2-4 | 12023SM 4 | mC*mG*mAmAmCmUm GmCfAfGfAfUmCfAmC mCmAmAmUmGmA | 12023AM 2 | VPU-S*fC*mAmUmUmGmGm UmGmAmUdCmUfGmCfAmG mUmU*mC*mG |
| 12024. 2-4 | 12024SM 4 | mC*mU*mAmCmCmCm UmGfAfAfCfUmGfCmA mGmAmUmCmAmA | 12024AM 2 | VPU-S*fU*mGmAmUmCmUm GmCmAmGdTmUfCmAfGmG mGmUmAmG*mG*mC |
| 12025. 2-4 | 12025SM 4 | mG*mA*mAmGmUmCm UmAfCfCfCfUmGfAmA mCmUmGmCmAmA | 12025AM 2 | VPU-S*fU*mGmCmAmGmUm UmCmAmGdGmGfUmAfGmA mCmUmUmC*mU*mU |
| 12028. 2-4 | 12028SM 4 | mG*mC*mCmAmGmUm GmAfCfCfAfUmCfCmA mGmAmAmCmUmA | 12028AM 2 | VPU-S*fA*mGmUmUmCmUm GmGmAmUdGmGfUmCfAmC mUmGmGmC*mU*mG |
| 12034. 2-4 | 12034SM 4 | mU*mA*mUmGmCmCfC fAfUfUmUfCmCmAmGm AmAmAmA | 12034AM 2 | VPU-S*fU*mUmUmCmUmGm GmAmAmAdTmGfGmGfCmA mUmG*mU*mU |
| 12037. 2-4 | 12037SM 4 | mG*mC*mUmGmUmCm CmAfAfGfAfUmGfCmA mGmCmAmGmAmA | 12037AM 2 | VPU-S*fU*mCmUmGmCmUm GmCmAmUdCmUfUmGfGmA mCmA*mG*mC |
| 12038. 2-4 | 12038SM 4 | mG*mA*mAmCmCmUm AmCfAfAfGfUmUfCmU mUmUmGmAmGmA | 12038AM 2 | VPU-S*fC*mUmCmAmAmAm GmAmAmCdTmUfGmUfAmG mGmU*mU*mC |
| 12039. 2-4 | 12039SM 4 | mG*mA*mCmCmUmAm CmAfAfGfUfUmCfUmU mUmGmAmGmCmA | 12039AM 2 | VPU-S*fG*mCmUmCmΛmΛm AmGmAmAdCmUfUmGfUmA mGmG*mU*mC |
| 12040. 2-4 | 12040SM 4 | mC*mC*mCmAmCmAm AmGfUfUfCfUmUfUmG mAmGmCmAmGmA | 12040AM 2 | VPU-S*fC*mUmGmCmUmCm AmAmAmGdAmAfCmUfUmG mUmG*mG*mG |
| 12041. 2-4 | 12041SM 4 | mG*mA*mAmAmUmCm CmAfAfCfCfUmAfCmA mAmGmUmUmCmA | 12041AM 2 | VPU-S*fG*mAmAmCmUmUm GmUmAmGdGmUfUmGfGmA mUmU*mU*mC |
| 12044. 2-4 | 12044SM 4 | mC*mC*mAmCmCmCm UmGfAfAfCfUmGfCmA mGmAmUmCmAmA | 12044AM 2 | VPU-S*fU*mGmAmUmCmUm GmCmAmGdTmUfCmAfGmG mGmU*mG*mG |
| 12045. 2-4 | 12045SM 4 | mG*mA*mCmCmAmAm UmGfUfGfGfUmAfGmA mAmGmCmCmAmA | 12045AM 2 | VPU-S*fU*mGmGmCmUmUm CmUmAmCdCmAfCmAfUmU mGmG*mU*mC |
| 12047. 2-4 | 12047SM 4 | mC*mC*mGmCmCmAm AmGfAfAfGfUmCfUmA mCmCmCmUmGmA | 12047AM 2 | VPU-S*fC*mAmGmGmGmUm AmGmAmCdTmUfCmUfUmG mGmC*mG*mG |
| 12072. 2-4 | 12072SM 4 | mC*mA*mAmCmCmAfA fCfCfAmGfUmGmAmCm CmAmUmA | 12072AM 2 | VPU-S*fG*mGmAmUmGmGm UmCmAmCdTmGfGmUfUmG mGmU*mU*mG |
| 12073. 2-4 | 12073SM 4 | mC*mC*mCmGmAmAm CmUfGfCfAfGmAfUmC mAmCmCmAmAmA | 12073AM 2 | VPU-S*fC*mAmUmUmGmGm UmGmAmUdCmUfGmCfAmG mUmUmCmG*mG*mG |
| 12074. 2-4 | 12074SM 4 | mC*mU*mGmCmCmCfU fGfAfAmCfUmGmCmAm GmAmUmA | 12074AM 2 | VPU-S*fU*mGmAmUmCmUm GmCmAmGdTmUfCmAfGmG mGmC*mA*mG |
| 12075. 2-4 | 12075SM 4 | mC*mA*mAmGmUmCm UmAfCfCfCfUmGfAmA mCmUmGmCmAmA | 12075AM 2 | VPU-S*fU*mGmCmAmGmUm UmCmAmGdGmGfUmAfGmA mCmU*mU*mG |
| 12075 a.2-4 | 12075aS M4 | mG*mA*mGmGmUmCm UmAfCfCfCfUmGfAmA mCmUmGmCmAmA | 12075aA M2 | VPU-S*fU*mGmCmAmGmUm UmCmAmGdGmGfUmAfGmA mCmC*mU*mC |
| 12076. 2-4 | 12076SM 4 | mG*mG*mUmCmUmAfC fCfCfUmGfAmAmCmUm GmCmAmA | 12076AM 2 | VPU-S*fU*mGmCmAmGmUm UmCmAmGdGmGfUmAfGmA mCmC*mU*mC |
| 12077. 2-4 | 12077SM 4 | mU*mA*mGmAmAmGf UfUfCfUmUfUmGmAmG mCmAmGmA | 12077AM 2 | VPU-S*fA*mUmCmUmGmCm UmCmAmAdAmGfAmAfCmU mUmC*mU*mA |
| 12078. 2-4 | 12078SM 4 | mC*mC*mCmAmCmAm AmGfUfUfCfUmUfUmG mAmGmCmAmGmA | 12078AM 2 | VPU-S*fA*mUmCmUmGmCm UmCmAmAdAmGfAmAfCmU mUmGmUmG*mG*mG |
| 12080. 2-4 | 12080SM 4 | mC*mA*mCmCmAmAfU fGfUfGmGfUmAmGmAm AmGmCmA | 12080AM 2 | VPU-S*fU*mGmGmCmUmUm CmUmAmCdCmAfCmAfUmU mGmG*mU*mG |

wherein VPU-S refers to 2'-S-methyluridine-5'-(E)-vinylphosphonate-3'-phosphate, mA refers to 2'-O-methyladenosine-3'-phosphate, mU refers to 2'-O-methyluridine-3'-phosphate, mC refers to 2'-O-methylcytidine-3'-phosphate, mG refers to 2'-O-methylguanosine-3'-phosphate, fA refers to 2'-fluoroadenosine-3'-phosphate, fU refers to 2'-fluorouridine-3'-phosphate, fC refers to 2'-fluorocytidine-3'-phosphate, fG refers to 2'-fluoroguanosine-3'-phosphate, dA refers to 2'-deoxyadenosine-3'-phosphate, dT refers to 2'-deoxythymidine-3'-phosphate, dC refers to 2'-deoxycytidine-3'-phosphate, dG refers to 2'-deoxyguanosine-3'-phosphate, and * refers to a phosphorothioate bond

### Embodiment 2 In Vitro siRNA Screening Using Liposome-mediated Transfection in Hep3B and U87-MG Cells

Cell culture and 96-well plate transfection: *In vitro* experiments were conducted in Hep3B and U87-MG cells using MEM + 10% FBS + 1X penicillin-streptomycin + 1X non-essential amino acids medium. When cells reached 80% confluence, they were digested with trypsin. Cell density was determined using a Scepter automated cell counter (Millipore, #PHCC00000). Concurrently, siRNA, Opti-MEM, and INTERFERin (Polyplus) were mixed in a 96-well plate, and the plate was incubated at room temperature for 10 min. Then, complete medium containing cells was added to each well. The 96-well plate was incubated at 37°C in a 5% CO₂ incubator for 24 h.

96-well plate RNA extraction and reverse transcription: mRNA was extracted from cells in the 96-well plate using Oligo d(T)25 Magnetic Beads reagent (NEB). The culture medium was aspirated from the 96-well plate, and the wells were washed once with DPBS. Then, 100 µL of cell lysate was added to each well, followed by 20 µL of beads. The plate was shaken on an oscillator and then placed on a magnetic separation rack. The lysate was aspirated from the wells. Then, 100 µL of wash buffer A was added to each well, pipetted up and down, and the plate was placed back on the magnetic separation rack. Wash buffer A was aspirated. The beads were then pipetted up with 100 µL of wash buffer B and transferred to a new 96-well plate. The plate was placed on the magnetic separation rack, wash buffer B was aspirated, and the beads were pipetted up with 100 µL of low-salt buffer and transferred to a 96-well PCR plate. The 96-well PCR plate was placed on the magnetic separation rack, the low-salt buffer was aspirated, and 10 µL of elution buffer was added to each well to pipette up the beads. The plate was incubated at 50°C for 2 min to elute mRNA from the beads. The reverse transcription system was prepared using the StarScript Pro One-Tube De-genomic Reverse Transcription Premix (Genstar); 5 µL was dispensed into each well of a 96-well PCR plate, 5 µL of the mRNA solution obtained in the previous step was added, the mixture was mixed thoroughly, briefly centrifuged, and the plate was sealed with a sealing film. The plate was incubated on the PCR instrument at 37°C for 3 min, then at 50°C for 50 min, followed by 85°C for 2 min. Then, it was cooled to 4°C to complete reverse transcription.

Real-time fluorescent quantitative PCR: After reverse transcription, the 96-well plate was placed on the magnetic separation rack until all beads were adsorbed to the bottom. The reverse transcription reagent was aspirated. The prepared QPCR system was added to the 96-well PCR plate. The plate was sealed with sealing film, and PCR was performed on a StepOnePlus Real-Time PCR System (Applied Biosystems). Data were analyzed using the ΔΔCt method and normalized using cells transfected with the negative control sequence at the same concentration.

The negative control AD-1955 sequence is as follows:
Sense strand: CUUACGCUGAGUACUUCGAdTdT (SEQ ID NO: 177)
Antisense strand: UCGAAGUACUCAGCGUAAGdTdT (SEQ ID NO: 178).

The primers for detecting APP are as follows:
Forward primer: CCTTCCCGTGAATGGAGAGTT (SEQ ID NO: 179)
Reverse primer: CTGGTCGAGTGGTCAGTCC (SEQ ID NO: 180)

Primers for detecting GAPDH with probe: CCTGGACGATCTCCAGCCGTGG (SEQ ID NO: 181) (Reporter gene 5'FAM, Quencher group 3'BHQ1) are as follows:
Forward primer: GACAGTCAGCCGCATCTTC (SEQ ID NO: 182)
Reverse primer: ACTCCGACCTTCACCTTCC (SEQ ID NO: 183)
Probe: CGCCAGCCGAGCCACATCGC (SEQ ID NO: 184) (Reporter gene 5'VIC, Quencher group 3'MGB).

**Table 3: Experimental Results of 10 nM and 1 nM Modified siRNA Transfection via Liposome in Hep3B Cells**

| siRNA | Residual APP mRNA Levels in Hep3B Cells (%) (relative to negative control cells) | | | |
|---|---|---|---|---|
| | Mean at 10 nM | Standard Deviation at 10 nM | Mean at 1 nM | Standard Deviation at 1 nM |
| 12003.1-1 | 8.43 | 1.12 | 12.65 | 0.75 |
| 12004.1-1 | 4.59 | 0.78 | 18.77 | 1.25 |
| 12006.1-1 | 2.79 | 0.78 | 6.55 | 1.97 |
| 12014.1-1 | 36.17 | 6.76 | 61.93 | 9.63 |
| 12015.1-1 | 9.05 | 0.53 | 16.88 | 1.46 |
| 12024.1-1 | 5.16 | 0.58 | 13.23 | 1.81 |
| 12025.1-1 | 4.11 | 0.08 | 7.10 | 0.91 |
| 12028.1-1 | 9.50 | 2.27 | 22.81 | 0.18 |
| 12034.1-1 | 15.33 | 1.23 | 36.34 | 4.41 |

**Table 4: Experimental Results of 10 nM Modified siRNA Transfection via Liposome in U87-MG Cells**

| siRNA | Residual APP mRNA Levels in U87-MG Cells (%) (relative to negative control cells) | |
|---|---|---|
| | Mean at 10 nM | Standard Deviation at 10 nM |
| 12003.1-1 | 14.26 | 0.36 |
| 12004.1-1 | 5.59 | 1.26 |
| 12006.1-1 | 7.29 | 0.95 |
| 12014.1-1 | 57.11 | 2.86 |
| 12015.1-1 | 13.31 | 1.49 |
| 12024.1-1 | 7.96 | 0.26 |
| 12025.1-1 | 5.11 | 0.73 |
| 12028.1-1 | 12.29 | 1.37 |
| 12034.1-1 | 33.40 | 1.35 |

**Table 5: Experimental Results of 10 nM and 1 nM Modified siRNA Transfection via Liposome in U87-MG Cells**

| siRNA | Residual APP mRNA Levels in U87-MG Cells (%) (relative to negative control cells) | | | |
|---|---|---|---|---|
| | Mean at 10 nM | Standard Deviation at 10 nM | Mean at 1 nM | Standard Deviation at 1 nM |
| AD-454973 | 2.46 | 0.96 | 3.13 | 0.48 |
| 12001.1-3 | 71.48 | 5.17 | 91.28 | 10.83 |
| 12002.1-3 | 82.64 | 5.93 | 87.73 | 4.53 |
| 12005.1-3 | 6.56 | 0.60 | 15.96 | 3.32 |
| 12006.1-1 | 1.87 | 0.04 | 3.53 | 0.75 |
| 12007.1-3 | 7.10 | 1.18 | 38.92 | 4.16 |
| 12023.1-3 | 38.92 | 8.47 | 39.43 | 3.51 |
| 12037.1-3 | 2.49 | 0.07 | 6.18 | 1.40 |
| 12038.1-1 | 6.39 | 0.79 | 25.21 | 5.53 |
| 12039.1-3 | 12.17 | 2.53 | 40.34 | 2.51 |
| 12040.1-3 | 16.73 | 1.56 | 46.78 | 3.93 |
| 12041.1-3 | 1.81 | 0.12 | 4.13 | 0.72 |
| 12044.1-1 | 10.18 | 0.48 | 27.94 | 0.49 |
| 12045.1-3 | 32.84 | 6.99 | 66.55 | 7.50 |
| 12047.1-1 | 15.30 | 2.06 | 39.13 | 7.40 |
| 12072.1-1 | 22.61 | 5.00 | 30.62 | 3.36 |
| 12073.1-3 | 41.33 | 6.95 | 38.14 | 4.24 |
| 12074.1-1 | 31.07 | 9.84 | 35.90 | 6.47 |
| 12075.1-3 | 2.37 | 0.64 | 3.25 | 0.67 |
| 12076.1-3 | 2.79 | 0.35 | 4.31 | 0.13 |
| 12077.1-3 | 23.64 | 4.69 | 32.54 | 5.84 |
| 12078.1-3 | 10.65 | 7.02 | 24.12 | 2.07 |
| 12080.1-3 | 32.00 | 6.57 | 49.67 | 5.38 |

**Table 6: Experimental Results of 10 nM and 1 nM Modified siRNA Transfection via Liposome in Rat Primary Hepatocytes**

| siRNA | Residual APP mRNA Levels in Rat Primary Hepatocytes (%) (relative to negative control cells) | | | |
|---|---|---|---|---|
| | Mean at 10 nM | Standard Deviation at 10 nM | Mean at 1 nM | Standard Deviation at 1 nM |
| 12003.1-1 | 65.58 | 24.42 | 94.81 | 22.99 |
| 12004.1-1 | 47.47 | 5.43 | 92.49 | 27.88 |
| 12006.1-1 | 22.62 | 9.85 | 16.42 | 8.73 |
| 12014.1-1 | 65.76 | 26.57 | 55.36 | 16.25 |
| 12015.1-1 | 32.52 | 9.47 | 53.80 | 16.61 |
| 12024.1-1 | 18.02 | 13.85 | 27.32 | 18.57 |
| 12025.1-1 | 26.97 | 15.67 | 10.36 | 3.75 |
| 12028.1-1 | 45.94 | 17.29 | 50.46 | 17.80 |
| 12034.1-1 | 38.55 | 10.92 | 45.44 | 15.65 |

**Table 7: Experimental Results of 10 nM Modified siRNA Transfection via Liposome in Mouse Primary Hepatocytes**

| siRNA | Residual APP mRNA Levels in Mouse Primary Hepatocytes (%) (relative to negative control cells) | |
|---|---|---|
| | Mean at 10 nM | Standard Deviation at 10 nM |
| AD-454973 | 54.89 | 47.99 |
| 12003.1-1 | 37.90 | 3.63 |
| 12004.1-1 | 10.21 | 1.86 |
| 12006.1-1 | 5.43 | 0.39 |
| 12014.1-1 | 62.80 | 5.81 |
| 12015.1-1 | 22.02 | 1.85 |
| 12024.1-1 | 3.18 | 0.40 |
| 12025.1-1 | 3.57 | 0.78 |
| 12028.1-1 | 9.81 | 0.66 |
| 12034.1-1 | 15.04 | 4.37 |
| 12075.1-3 | 3.60 | 0.24 |
| 12076.1-3 | 2.44 | 1.26 |

Based on the above *in vitro* results, multiple sequences, including 12006.1-1, demonstrated excellent activity in human, rat, and mouse cells. Subsequently, the most active sequences were linked to peptides to form peptide-oligonucleotide conjugates for *in vivo* activity validation experiments.

### Embodiment 3 Stability Testing of Different siRNA Sequences Targeting APP

The experimental methods for siRNA stability studies are as follows:
1. The test compound sequence was diluted precisely using DEPC-treated water to a working solution concentration of 10 µM.
2. Preparation of liver homogenate: A total of 200 mg of mouse liver was weighed, with 1 mL of potassium phosphate buffer solution and 5 magnetic beads added. The sample was homogenized three times in a homogenizer at 60 Hz for 30 seconds per cycle to obtain a 200 mg/mL liver homogenate.
3. Preparation of sample at 72 h: A total of 17 µL of the 10 µM working solution of the test/positive compound was taken, with 153 µL of liver homogenate added. The mixture was vortexed for 30 s. Then, 75 µL of the compound-liver homogenate mixture was transferred to an EP tube. The sample was prepared in duplicate and incubated at 37°C for 72 h. After incubation, 50 µL was withdrawn.
4. Preparation of sample at 0 h: A total of 75 µL of liver homogenate was transferred into an EP tube. The sample was prepared in duplicate and incubated at 37°C for 72 h. After incubation, 45 µL of liver homogenate was withdrawn, and 5 µL of 10 µM test/positive compound was added. Then, the mixture was mixed thoroughly.
5. Each tube containing the incubated sample was added with 5 µL of 50 µg/mL internal standard and mixed well. Then, 100 µL of lysate was added to each tube, and the mixture was vortexed for 30 s and allowed to stand for 20 min.
6. After all samples were treated with appropriate pretreatment methods, the supernatant was collected for instrumental analysis. Subsequently, the relative residual rate (%) was calculated as: (Compound content at 72 h / Compound content at 0 h) × 100%.

**Table 8: Experimental Results of Stability in Liver Homogenate for Different siRNA Sequences Targeting APP**

| siRNA | Residual Percentage of Antisense Strand at 72 h (%) |
|---|---|
| AD-454913 | 43.36 |
| 12006.1-1 | 80.95 |
| 12024.1-1 | 72.88 |
| 12025.1-1 | 67.66 |
| 12028.1-1 | 73.22 |
| 12075.1-3 | 80.68 |
| 12076.1-3 | 78.94 |

As shown in Table 8, the stability of different sequences in liver homogenate was different. Among these, sequences 12006.1-1, 12075.1-3, and 12076.1-3 exhibited significantly higher stability than the positive control AD-454913.

### Embodiment 4. Synthesis of Peptides

Peptide synthesis was performed at the 0.2 mmol scale on the PurePep Chorus 4-channel peptide synthesizer following standard solid-phase peptide synthesis protocols, such as those described by Adam G et al. in Standard practices for Fmoc-based solid-phase peptide synthesis in the Nowick laboratory*.* The main process conditions are as follows: (1) Resin swelling: Fmoc-Tyr-Wang Resin and Rink-Amide-MBHA-Resin were swollen in DMF at room temperature for 30 min; (2) Deprotection: the resin was treated with 20% piperidine in DMF at 50°C for 2 min; (3) Amino acid condensation: Fmoc amino acid (5 eq, resin = 1 eq) was coupled using HCTU (5 eq) as the condensing agent and DIPEA (5 eq) as the base at 50°C for 10 min; (4) N-terminal acetylation: 5% N-acetylimidazole was applied at room temperature for 1 h; (5) TFA cleavage: after synthesis, the resin was treated with TFA cleavage reagent (TFA/Tips/H₂O = 95:2.5:2.5; TFA/Tips/EDT/H₂O = 92.5:2.5:2.5:2.5) for over 2 h. Then, the resin was filtered off and the filtrate was collected. (6) Precipitation and purification: Pre-chilled anhydrous diethyl ether was added to the filtrate. The mixture was centrifuged to obtain a white precipitate, which was washed 3 times with anhydrous diethyl ether and dried under reduced pressure at room temperature to give a whitish powdery crude peptide product. Crude peptides were dissolved in an acetonitrile/water system and purified using high-performance liquid chromatography (HPLC).

For peptides bearing a 6-azido group modification on the lysine side chain, the synthesis process and conditions are essentially identical to the aforementioned method, with the following distinctions: (1) Fmoc-Lys(Mtt)-OH was used instead of the conventional Fmoc-Lys(Boc)-OH amino acid raw material; (2) After solid-phase synthesis completion, the Mtt protecting group was selectively removed from the lysine side-chain amino group using a DCM solution containing 2% TFA. Subsequently, the side chain was reacted with 6-azidohexanoic acid under the conventional condensation conditions described above before peptide cleavage.

The theoretical and measured molecular weights of the peptides synthesized according to the above method are shown in Table 9 below.

**Table 9: Theoretical and Measured Molecular Weights of Synthetic Peptides**

| Peptide Name | Peptide Sequence Structure | Theoretic al Molecular Weight | Measured Molecular Weight |
|---|---|---|---|
| L57-AZK | 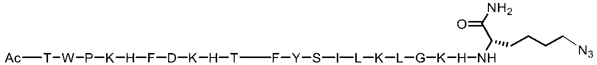 | 2679.13 | 894.05 [M+3H]³⁺ |
| Ang2-AZK | 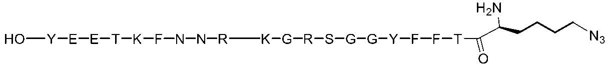 | 2455.68 | 819.55 [M+3H]³⁺ |
| Reversed Ang2-AZK | 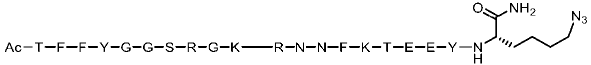 | 2496.73 | 1249.41 [M+2H]²⁺ |
| Ang2 (K10AZK) | 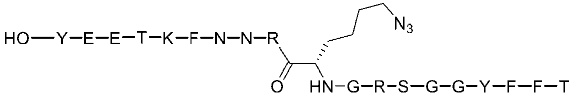 | 2326.49 | 582.60 [M+4H]⁴⁺ |
| Ang2 (K10N₃) | 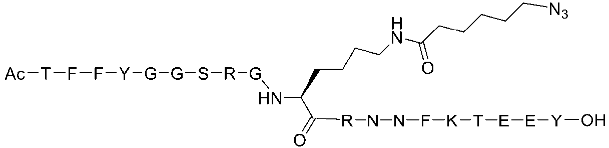 | 2482.70 | 1242.30 [M+2H]²⁺ |
| Ang2-Ang2-AZK | 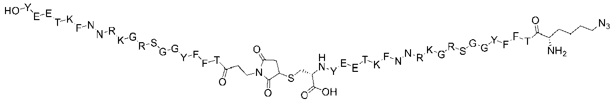 | 5010.46 | 1002.8 [M+5H]⁵⁺ |

### Embodiment 5 Synthesis of Ang2 (K10N₃)

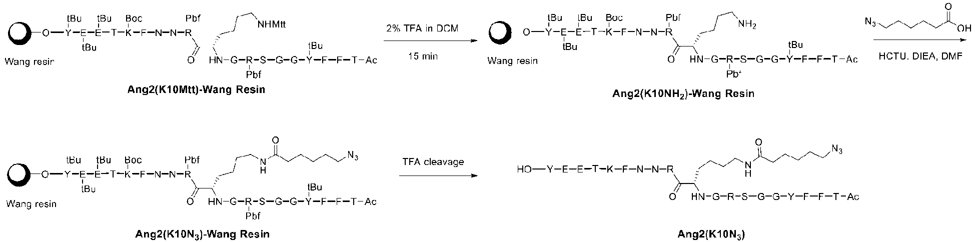

On the PurePep Chorus 4-channel peptide synthesizer, Ang2(K10Mtt)-Wang Resin was synthesized at a scale of 0.2 mmol under the conditions described in Embodiment 4. To Ang2(K10Mtt)-Wang Resin, 20 mL of DCM and 0.4 mL of TFA were added. After shaking at room temperature for 15 min, the resin was washed 3 times with DCM to obtain Ang2(K10NH₂)-Wang Resin. Ang2(K10NH₂)-Wang Resin was transferred into 15 mL of DMF, and then 6-azido-hexanoic acid (157 mg, 1 mmol), HCTU (414 mg, 1 mmol), and DIEA (129 mg, 1 mmol) were sequentially added. The mixture was shaken at room temperature for 1 h, washed with DMF and DCM, cleaved with TFA at room temperature for 2 h, precipitated with diethyl ether, and purified by HPLC to obtain the target peptide Ang2(K10N₃) (150 mg, white powdery solid) (theoretical molecular weight: 2482.70, measured molecular weight: 1242.30 [M+2H]²⁺).

### Embodiment 6 Synthesis of Peptide Ang2-Ang2-AZK

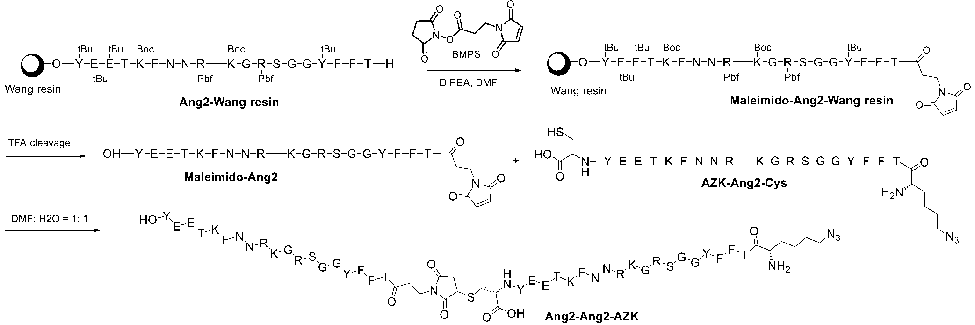

On the PurePep Chorus 4-channel peptide synthesizer, Ang2-Wang resin was synthesized at a 0.2 mmol scale under the conditions described in Embodiment 4. Ang2-Wang resin was reacted with 1.5 eq of N-succinimidyl 3-maleimidopropionate (BMPS) for 1 h to give Maleimido-Ang2-Wang resin. TFA cleavage (TFA/Tips/H₂O = 95:2.5:2.5) was performed, followed by precipitation with diethyl ether and HPLC purification to give Maleimido-Ang2. Dissolved 1 eq of Maleimido-Ang2 and 1.1 eq of AZK-Ang-Cys in a 1:1 DMF/H₂O mixed solvent. The reaction was carried out at room temperature for 2 h and was monitored by LC-MS until completion. The solvent was evaporated under reduced pressure, the residue was dissolved in an acetonitrile/water system, and the product was purified by HPLC to give 25 mg of the target peptide Ang2-Ang2-AZK (theoretical molecular weight: 5010.46, measured molecular weight: 1002.8 [M+5H]⁵⁺).

### Embodiment 7 Synthesis of Compound B08

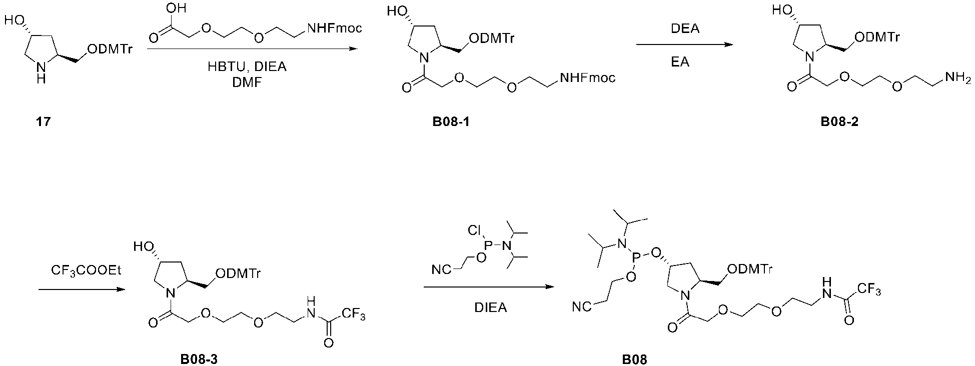

### 1. Synthesis of Compound B08-1

Compound [2-[2-(Fmoc-amino)ethoxy]ethoxy]acetic acid (10 g, 26.0 mmol), DIEA (6.7 g, 52.0 mmol), and HBTU (10.8 g, 28.6 mmol) were dissolved in DMF (100 mL) and stirred at room temperature for 5 min. Then, compound 17 (11.4 g, 27.3 mmol) was dissolved in DMF (20 mL) and added to the above reaction solution. The reaction system was stirred at room temperature for 2 h. The reaction mixture was added to a saturated NaHCO₃ solution, followed by DCM extraction. The organic phase was dried over anhydrous sodium sulfate, filtered, concentrated, and separated by column chromatography (DCM/MeOH = 100:1, 2% TEA) to give a colorless oil (17.4 g, yield: 85.2%). LCMS: 786.9, found: 808.9 [M+Na].

### 2. Synthesis of Compound B08-2

B08-1 (8.0 g, 10.2 mmol) was dissolved in EA (76 mL), and diethylamine (12.6 g) was added. The reaction system was stirred at room temperature overnight. The reaction mixture was concentrated under reduced pressure and separated by column chromatography (DCM/MeOH = 20:1, 0.5% TEA → 10:1, 0.1% TEA) to afford a white, foamy solid (3.8 g, yield: 66.2%). LCMS: 564.7, found: 565.8 [M+H]⁺.

### 3. Synthesis of Compound B08-3

B08-2 (2.0 g, 3.5 mmol) was dissolved in ACN (35 mL), and ethyl trifluoroacetate (1.3 g, 8.8 mmol) was added. The reaction system was stirred at room temperature overnight. The reaction mixture was concentrated and purified by column chromatography (DCM/MeOH = 30:1, 0.1% TEA) to afford a white, foamy solid (1.87 g, yield: 79.9%). LCMS: 660.7, found: 682.7 [M+Na]⁺.

### 4. Synthesis of Compound B08

B08-3 (1.5 g, 2.3 mmol) and DIPEA (0.9 g, 6.8 mmol) were dissolved in anhydrous DCM (10 mL). Cyanoethyl-N,N-diisopropylchlorophosphoramidite (0.6 g, 2.7 mmol) was added, and the reaction system was stirred at room temperature for 1 h. The reaction mixture was concentrated at 30°C and separated by column chromatography (PE, 2% TEA → PE/EA = 4:1, 2% TEA) to give a whitish, foamy solid (0.8 g, yield: 38.8%). LCMS: 860.9, found: 861.9 [M+H]⁺.

### Embodiment 8 Synthesis of Compound B12

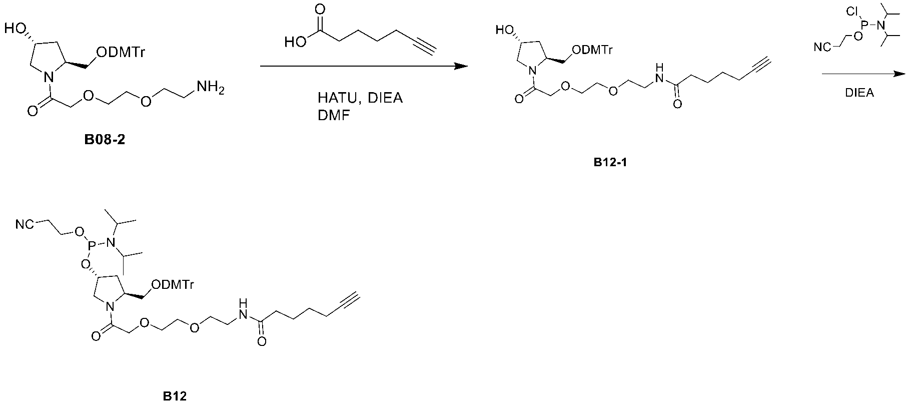

### 1. Synthesis of Compound B 12-1

Compound 6-heptynoic acid (0.35 g, 2.77 mmol), DIEA (0.90 g, 6.94 mmol), and HATU (1.16 g, 3.05 mmol) were dissolved in DMF (15 mL) and stirred at room temperature for 5 min. Then, compound B08-2 (1.64 g, 2.91 mmol) was dissolved in DMF (15 mL) and added to the above reaction solution. The reaction system was stirred at room temperature for 2 h. The reaction mixture was added to a saturated NaHCO₃ solution, followed by DCM extraction. The organic phase was dried over anhydrous sodium sulfate, filtered, concentrated, and separated by column chromatography (DCM/MeOH = 100:1, 2% TEA) to give a colorless oil (0.5 g, yield: 26.8%). LCMS: 672.8, found: 695.0 [M+Na]⁺.

### 2. Synthesis of Compound B12

Compound B12-1 (0.5 g, 0.74 mmol) and DIEA (0.3 g, 2.25 mmol) were dissolved in anhydrous DCM (10 mL). Cyanoethyl-N,N-diisopropylchlorophosphoramidite (0.21 g, 0.89 mmol) was added, and the reaction system was stirred at room temperature for 1 h. The reaction mixture was concentrated at 30°C and separated by column chromatography (PE, 2% TEA → PE/EA = 4:1, 2% TEA) to give a whitish, foamy solid (0.39 g, yield: 60.1%). LCMS: 873.0, found: 874 [M+H]⁺.

### Embodiment 9 Synthesis of Dipeptide Linker BPL-1

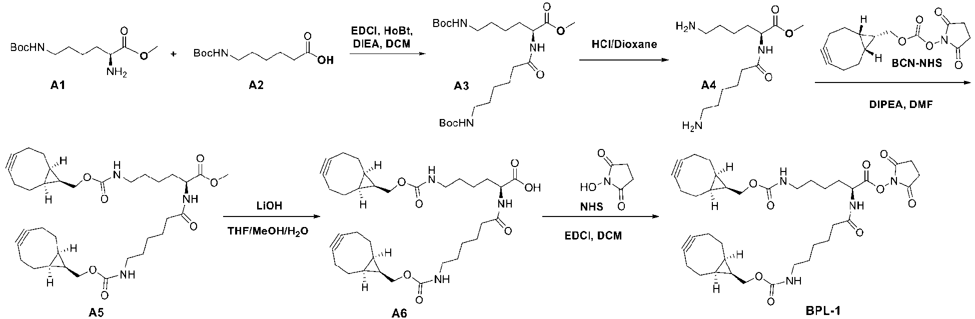

Step 1. Preparation of N6-(tert-butoxycarbonyl)-N2-(6-((tert-butoxycarbonyl)amino)hexanoyl)-L-lysine methyl ester (Compound A3): At room temperature, a mixture of N6-(tert-butoxycarbonyl)-L-lysine methyl ester (5.0 g, 19.2 mmol, 1.0 eq), boc-6-AMinocaproic acid (4.89 g, 21.1 mmol, 1.1 eq), and N,N-diisopropylethylamine (5.46 g, 42.3 mmol, 2.2 eq) in dichloromethane was stirred thoroughly. Then, 1-hydroxybenzotriazole (5.71 g, 42.3 mmol, 2.2 eq) and 1-(3-dimethylaminopropyl)-3-ethylcarbodiimide hydrochloride (8.08 g, 42.3 mmol, 2.2 eq) were added and reacted for 1 h. The reaction was monitored by thin-layer chromatography (DCM:MeOH=30:1, R_{f}=0.3) and LC-MS until completion. The reaction mixture was diluted with dichloromethane and extracted. The organic layer was washed twice with saturated sodium bicarbonate solution, followed by two washes with saturated ammonium chloride solution. The mixture was dried over anhydrous sodium sulfate, and the filtrate was concentrated under reduced pressure. The residue was purified by silica gel column chromatography (eluent: dichloromethane: methanol) to give the product,

N6-(tert-butoxycarbonyl)-N2-(6-((tert-butoxycarbonyl)amino)hexanoyl)-L-lysine methyl ester (8.5 g, yield: 93.8%). MS (ES⁺): m/z=474 (M+H)⁺.

Step 2. Preparation of (6-aminohexanoyl)-L-lysine methyl ester (Compound A4): At room temperature, N6-(tert-butoxycarbonyl)-N2-(6-((tert-butoxycarbonyl)amino)hexanoyl)-L-lysine methyl ester (1.0 g, 2.11 mmol, 1.0 eq) and hydrogen chloride in dioxane (10 mL, 2 mol/L) were mixed and reacted for 1.5 h. The reaction was monitored by LC-MS until completion. The reaction mixture was concentrated under reduced pressure, and water (30 mL) and sodium hydroxide solution were added to adjust the pH to approximately 7-8. The aqueous phase was concentrated under reduced pressure using an oil pump. The solid obtained was washed with a dichloromethane/methanol = 10/1 solution. The filtrate was concentrated under reduced pressure to give (6-aminohexanoyl)-L-lysine methyl ester as a pale yellow oil (450 mg, yield: 77.9%). MS (ES⁺): m/z=274 (M+H)⁺.

Step 3. Preparation of N6-((((1R,8S,9S)-bicyclo[6.1.0]non-4-yne-9-yl)methoxy)carbonyl)-N2-(6-(1R,8S,9R)-bicyclo[6.1. 0]non-4-en-9-ylmethoxy)carbonyl)amino)hexanoyl)-L-lysine methyl ester (Compound A5): Under a nitrogen atmosphere, (6-aminohexanoyl)-L-lysine methyl ester (450 mg, 1.65 mmol, 1.0 eq), ((1R,8S,9S)-bicyclo[6.1.0]non-4-yn-9-yl)methyl (2,5-dioxopyrrolidin-1-yl) carbonate (960 mg, 3.29 mmol, 2.0 eq) and N,N-diisopropylethylamine (468 mg, 3.63 mmol, 2.2 eq) in N,N-dimethylformamide (5 mL) was stirred at room temperature for 1 h. The reaction was monitored by thin-layer chromatography (DCM:MeOH=10:1, R_{f}=0.4) and LC-MS until completion. After cooling to room temperature, the solvent was concentrated under reduced pressure. The residue was diluted with water (20 mL) and extracted with dichloromethane/methanol = 10/1 (30 mL). The organic layer was washed with brine, dried over anhydrous sodium sulfate, and concentrated under reduced pressure. The crude product was purified by column chromatography (eluent: dichloromethane/methanol = 30/1) to give N6-((((1R,8S,9S)-bicyclo[6.1.0]non-4-yne-9-yl)methoxy)carbonyl)-N2-(6-(1R,8S,9R)-bicyclo[6.1. 0]non-4-en-9-ylmethoxy)carbonyl)amino)hexanoyl)-L-lysine methyl ester as a white solid (280 mg, yield: 27.1%). MS (ES⁺): m/z=626 (M+H)⁺.

Step 4. Preparation of N6-((((1R,8S,9S)-bicyclo[6.1.0]non-4-yne-9-yl)methoxy)carbonyl)-N2-(6-(1R,8S,9R)-bicyclo[6.1. 0]non-4-en-9-ylmethoxy)carbonyl)amino)hexanoyl)-L-lysine (Compound A6): N6-((((1R,8S,9S)-bicyclo[6.1.0]non-4-yne-9-yl)methoxy)carbonyl)-N2-(6-(1R,8S,9R)-bicyclo[6.1. 0]non-4-en-9-ylmethoxy)carbonyl)amino)hexanoyl)-L-lysine methyl ester (280 mg, 0.448 mmol, 1.0 eq) was added to a mixed solvent of tetrahydrofuran, methanol, and water (6 mL), followed by addition of an aqueous solution of lithium hydroxide (13 mg, 0.538 mmol, 1.2 eq) (1 mL). The mixture was stirred at room temperature for 1 h. The reaction was monitored by LC-MS until completion. The reaction mixture was concentrated under reduced pressure. Water (30 mL) was added, and then 1 mol/L dilute hydrochloric acid solution was added to adjust the pH to approximately 3-4. Then, the mixture was diluted with water (20 mL) and extracted with dichloromethane (30 mL). The organic layer was washed with brine, dried over anhydrous sodium sulfate, and the organic phase was concentrated under reduced pressure to give N6-((((1R,8S,9S)-bicyclo[6.1.0]non-4-yne-9-yl)methoxy)carbonyl)-N2-(6-(1R,8S,9R)-bicyclo[6.1. 0]non-4-en-9-ylmethoxy)carbonyl)amino)hexanoyl)-L-lysine as a white solid (220 mg, yield: 80.2%). MS (ES⁺): m/z=612 (M+H)⁺.

Step 5. Preparation of 2,5-dioxopyrrolidin-1-yl-N6-((1R,8S,9S)-bicyclo[6.1.0]non-4-yl-9-yl)methoxy)carbonyl)-N2-(6-(1 R,8S,9R)-bicyclo[6.1.0]non-4-yne-9-yl)methoxy)carbonyl)amino)hexanoyl)-L-lysine salt (BPL-1): N6-((((1R,8S,9S)-bicyclo[6.1.0]non-4-yne-9-yl)methoxy)carbonyl)-N2-(6-(1R,8S,9R)-bicyclo[6.1. 0]non-4-en-9-ylmethoxy)carbonyl)amino)hexanoyl)-L-lysine (220 mg, 0.36 mmol, 1.0 eq), N-hydroxysuccinimide (49.7 mg, 0.43 mmol, 1.2 eq), and 1-(3-dimethylaminopropyl)-3-ethylcarbodiimide hydrochloride (82.5 mg, 0.432 mmol, 2.2 eq) were added to dichloromethane. The mixture was stirred at room temperature for 1.5 h. The reaction was monitored by LC-MS until completion. The reaction mixture was diluted with dichloromethane and extracted. The organic layer was washed twice with saturated sodium bicarbonate solution, followed by two washes with saturated ammonium chloride solution. It was then dried over anhydrous sodium sulfate, and the organic phase was concentrated under reduced pressure. A white solid, 2,5-dioxopyrrolidin-1-yl-N6-((1R,8S,9S)-bicyclo[6.1.0]non-4-yl-9-yl)methoxy)carbonyl)-N2-(6-(1 R,8S,9R)-bicyclo[6.1.0]non-4-yne-9-yl)methoxy)carbonyl)amino)hexanoyl)-L-lysine salt (200 mg, yield: 78.7%), was given and used directly in the next step without purification. MS (ES⁺): m/z=709 (M+H)⁺.

### Embodiment 10 Synthesis of Dipeptide Linker BPL-2

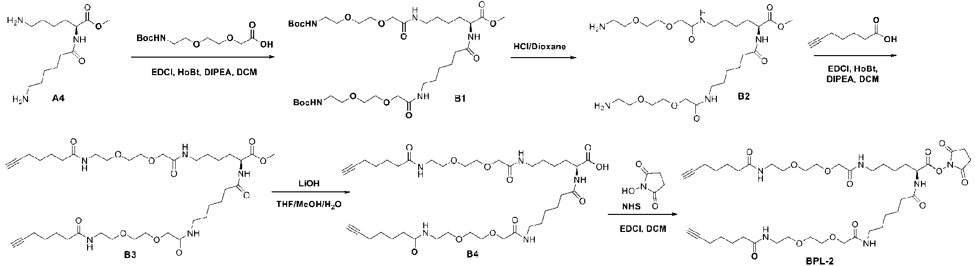

Step 1. Preparation of (S)-22-(2,2-dimethyl-4,13-dioxo-3,8,11-trioxatridec-5,14-diacten-18-yl)-2,2-dimethyl-4,3,20-trioxa -3,8,11-trioxa-5,14,21-triazacaplan-23-anoic acid methyl ester (Compound B1): At room temperature, a mixture of (6-aminohexanoyl)-L-lysine methyl ester (3.0 g, 10.9 mmol, 1.0 eq), 2,2-dimethyl-4-oxo-3,8,11-trioxo-5-azatridecane-13-oic acid (6.35 g, 24.2 mmol, 2.2 eq), and N,N-diisopropylethylamine (5.7 mL, 32.9 mmol, 3.0 eq) in dichloromethane was stirred thoroughly. Then, 1-hydroxybenzotriazole (3.7 g, 27.5 mmol, 2.5 eq) and 1-(3-dimethylaminopropyl)-3-ethylcarbodiimide hydrochloride (5.24 g, 27.5 mmol, 2.5 eq) were added and reacted for 1 h. The reaction was monitored by thin-layer chromatography (DCM:MeOH=20:1, R_{f}=0.3) and LC-MS until completion. The reaction mixture was diluted with dichloromethane and extracted. The organic layer was washed twice with saturated sodium bicarbonate solution, followed by two washes with saturated ammonium chloride solution. The mixture was dried over anhydrous sodium sulfate, and the filtrate was concentrated under reduced pressure. The residue was purified by silica gel column chromatography (eluent: dichloromethane: methanol) to give the product (S)-22-(2,2-dimethyl-4,13-dioxo-3,8,11-trioxatridec-5,14-diacten-18-yl)-2,2-dimethyl-4,3,20-trioxa -3,8,11-trioxa-5,14,21-triazacapronane-23-carboxylic acid methyl ester (3.9 g, yield: 46.8%), MS (ES⁺): m/z=764 (M+H)⁺.

Step 2. Preparation of methyl-N2-(6-(2-(2-(2-(2-(2-aminoethoxy)ethoxy)acetamido)hexanoyl)-N6-(2-(2-(2-aminoethoxy)e thoxy)acetyl)-L-lysyl ester (Compound B2): At room temperature, methyl-22-(2,2-dimethyl-4,13-dioxo-3,8,11-trioxo-5,14-diactaoctadec-18-yl)-2,2-dimethyl-4,3,20-tr ioxa-3,8,11-trioxa-5,14,21-triazacapric acid 23-ester (3.9 g, 5.11 mmol, 1.0 eq) and hydrogen chloride in dioxane (40 mL, 2 mol/L) were mixed and reacted for 1.5 h. The reaction was monitored by LC-MS until completion. The reaction mixture was concentrated under reduced pressure and added with water (30 mL) and sodium hydroxide solution to adjust the pH to approximately 7-8. The aqueous phase was concentrated under reduced pressure using an oil pump. The solid obtained was washed with a dichloromethane/ methanol = 10/1 solution. The filtrate was concentrated under reduced pressure to give methyl-N2-(6-(2-(2-(2-(2-(2-aminoethoxy)ethoxy)acetamido)hexanoyl)-N6-(2-(2-(2-aminoethoxy)e thoxy)acetyl)-L-lysyl ester as a pale yellow oil (2.9 g, yield: 85.8%). MS (ES⁺): m/z=564 (M+H)⁺.

Step 3. Preparation of (S)-2-(6,15-dioxo-8,11-dioxo-5,14-diazaeicos-20-yn-1-yl)-4,11,20-trioxo-13,16-dioxo-3,10,19-triaz ahexadecano-25-acetylene acid methyl ester (Compound B3): At room temperature, a mixture of methyl-N2-(6-(2-(2-(2-(2-(2-aminoethoxy)ethoxy)acetamido)hexanoyl)-N6-(2-(2-(2-aminoethoxy)e thoxy)acetyl)-L-lysyl ester (440 mg, 0.691 mmol, 1.0 eq), 6-heptynoic acid (178 mg, 1.41 mmol, 2.04 eq), and N,N-diisopropylethylamine (263 µL, 1.52 mmol, 2.2 eq) in dichloromethane was stirred thoroughly. Then 1-hydroxybenzotriazole (205 mg, 1.52 mmol, 2.2 eq) and 1-(3-dimethylaminopropyl)-3-ethylcarbodiimide hydrochloride (290 mg, 1.52 mmol, 2.2 eq) were added and reacted for 1 h. The reaction was monitored by thin-layer chromatography (DCM:MeOH=20:1, R_{f}=0.2) and LC-MS until completion. The reaction mixture was diluted with dichloromethane and extracted. The organic layer was washed twice with saturated sodium bicarbonate solution, followed by two washes with saturated ammonium chloride solution. The mixture was dried over anhydrous sodium sulfate, and the filtrate was concentrated under reduced pressure. The residue was purified by silica gel column chromatography (eluent: dichloromethane: methanol) to give the product (S)-2-(6,15-dioxo-8,11-dioxo-5,14-diazaeicos-20-yn-1-yl)-4,11,20-trioxo-13,16-dioxo-3,10,19-triaz ahexadecano-25-acetylene acid methyl ester (310 mg, yield: 57.6%). MS (ES⁺): m/z=780 (M+H)⁺.

Step 4. Preparation of (S)-2-(6,15-dioxo-8,11-dioxo-5,14-diazaeicos-20-yn-1-yl)-4,11,20-trioxo-13,16-dioxo-3,10,19-triaz ahexadecano-25-acetylene acid (Compound B4): (S)-2-(6,15-dioxo-8,11-dioxo-5,14-diazaeicos-20-yn-1-yl)-4,11,20-trioxo-13,16-dioxo-3,10,19-triaz ahexadecano-25-acetylene acid methyl ester (310 mg, 0.398 mmol, 1.0 eq) was added to a mixed solvent of tetrahydrofuran, methanol, and water (6 mL), followed by the addition of an aqueous solution (1 mL) of lithium hydroxide (20.1 mg, 0.478 mmol, 1.2 eq). The mixture was stirred at room temperature for 1 h. The reaction was monitored by LC-MS until completion. The reaction mixture was concentrated under reduced pressure. Water (25 mL) was added, and then 1 mol/L dilute hydrochloric acid solution was added to adjust the pH to approximately 3-4. Then, the mixture was diluted with water (20 mL) and extracted with dichloromethane (25 mL). The organic layer was washed with brine, dried over anhydrous sodium sulfate, and the organic phase was concentrated under reduced pressure to give (S)2-(6,15-dioxo-8,11-dioxo-5,14-diaza-20-eptadec-20-yn-1-yl)-4,11, 20-trioxo-13,16-dioxo-3,10,19-triazacyclohexadeca-25-carboxylic acid as a white solid (290 mg, yield: 95.4%). ¹H NMR (500 MHz, CDCl₃) δ 6.90 (s, 2H), 6.83 (d, *J* = 8.3 Hz, 1H), 6.55 (s, 2H), 4.15-4.10 (m, 1H), 3.69-3.62 (m, 4H), 3.55 (s, 8H), 3.50 (t, *J =* 4.8 Hz, 10H), 3.41-3.35 (m, 10H), 2.45-2.36 (m, 4H), 2.32 (t, *J* = 6.8 Hz, 2H), 2.24 (t, *J =* 6.9 Hz, 2H), 2.15 (dt, *J* = 7.1, 6.2 Hz, 7H), 1.89 (dd, *J =* 15.2, 8.1 Hz, 4H), 1.72-1.64 (m, 4H), 1.52-1.46 (m, 4H). MS (ES⁺): m/z=766 (M+H)⁺.

Step 5. Preparation of BPL-2: Compound B4 (290 mg, 0.38 mmol) was dissolved in 4 mL of dichloromethane. N-hydroxysuccinimide (52 mg, 0.46 mmol, 1.2 eq) and 1-(3-dimethylaminopropyl)-3-ethylcarbodiimide hydrochloride (88 mg, 0.46 mmol, 1.2 eq) were sequentially added. The mixture was stirred at room temperature for 1.5 h. The reaction was monitored by LC-MS until completion. The reaction mixture was diluted with dichloromethane and extracted. The organic layer was washed twice with saturated sodium bicarbonate solution, followed by two washes with saturated ammonium chloride solution. It was then dried over anhydrous sodium sulfate, and the organic phase was concentrated under reduced pressure. A white viscous solid, BPL-2 (310 mg, yield: 94.5%), was given and used directly in the next step without further purification. MS (ES⁺): m/z = 864 (M+H)⁺.

### Embodiment 11 Synthesis of Dipeptide Linker BPL-3

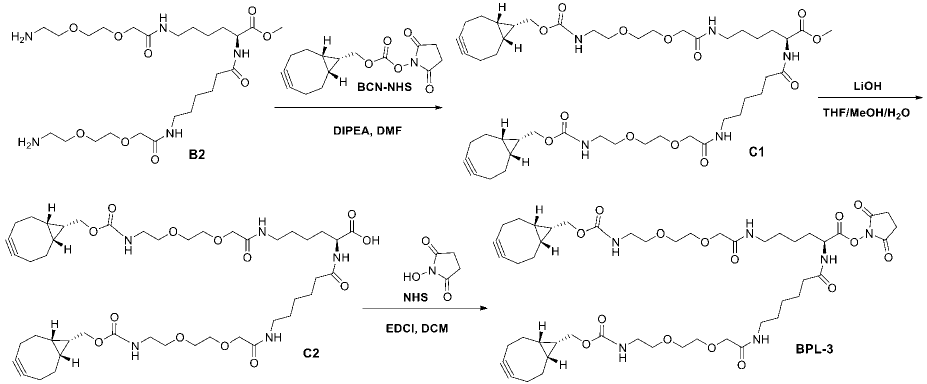

Step 1. Preparation of (S)-1-((1R,8S,9S)-bicyclo[6.1.0]nonan-4-yl)-9-yl)-21-(1-(1R,8S,9S)-bicyclo[6.1.0]heptan-4-yl)-3,1 2-dioxo-2,7,10-trioxa-4,13-diazaheptadec-17-yl)-3,12,19-trioxy-2,7,10-trioxa-4,13,20-triazadocosa ne-22-acid methyl ester (Compound C1): Under a nitrogen atmosphere, a mixture of methyl-N2-(6-(2-(2-(2-(2-(2-aminoethoxy)ethoxy)acetamido)hexanoyl)-N6-(2-(2-(2-aminoethoxy)e thoxy)acetyl)-L-lysyl ester (900 mg, 1.59 mmol, 1.0 eq), ((1R,8S,9S)-bicyclo[6.1.0]non-4-yn-9-yl)methyl (2,5-dioxopyrrolidin-1-yl) carbonate (928 mg, 3.19 mmol, 2.0 eq) and N,N-diisopropylethylamine (450 mg, 3.49 mmol, 2.2 eq) in N,N-dimethylformamide (10 mL) was stirred at room temperature for 1 h. The reaction was monitored by thin-layer chromatography (DCM:MeOH=10:1, R_{f}=0.3) and LC-MS until completion. After cooling to room temperature, the solvent was concentrated under reduced pressure. The residue was diluted with water (30 mL) and extracted with dichloromethane/methanol = 10/1 (30 mL). The organic layer was washed with brine, dried over anhydrous sodium sulfate, and concentrated under reduced pressure. The crude product was purified by column chromatography (eluent: dichloromethane/methanol = 30/1) to give (S)-1-((1R,8S,9S)-bicyclo[6.1.0]nonan-4-yl)-9-yl)-21-(1-(1R,8S,9S)-bicyclo[6.1.0]heptan-4-yl)-3,1 2-dioxo-2,7,10-trioxa-4,13-diazaheptadec-17-yl)-3,12,19-trioxy-2,7,10-trioxa-4,13,20-triazadocosa ne-22-acid methyl ester as a white solid (580 mg, yield: 39.8%). MS (ES⁺): m/z=916 (M+H)⁺.

Step 2. Preparation of (S)-1-((1R,8S,9S)-bicyclo[6.1.0]non-4-but-9-yl)-21-(1-(1R,8S,9)-bicyclo[1.1.0]non-4-but-9-yl)-3,1 2-dioxo-2,7,10-trioxa-4,13-diazaheptadecan-17-yl)-3,12,19-trioxa-2,7,10-trioxa-4,13,20-triazadocos an-22-oic acid (Compound C2): (S)-1-((1R,8S,9S)-bicyclo[6.1.0]nonan-4-yl)-9-yl)-21-(1-(1R,8S,9S)-bicyclo[6.1.0]heptan-4-yl)-3,1 2-dioxo-2,7,10-trioxa-4,13-diazaheptadec-17-yl)-3,12,19-trioxy-2,7,10-trioxa-4,13,20-triazadocosa ne-22-acid methyl ester (300 mg, 0.327 mmol, 1.0 eq) was added to a mixed solvent of tetrahydrofuran, methanol, and water (7.5 mL), followed by the addition of an aqueous solution (0.5 mL) of lithium hydroxide (9.5 mg, 0.40 mmol, 1.2 eq). The mixture was stirred at room temperature for 1 h. The reaction was monitored by LC-MS until completion. The reaction mixture was concentrated under reduced pressure. Water (25 mL) was added, and then 1 mol/L dilute hydrochloric acid solution was added to adjust the pH to approximately 3-4. Then, the mixture was diluted with water (25 mL) and extracted with dichloromethane (30 mL). The organic layer was washed with brine, dried over anhydrous sodium sulfate, and the organic phase was concentrated under reduced pressure to give (S)-1-((1R,8S,9S)-bicyclo[6.1.0]non-4-but-9-yl)-21-(1-(1R,8S,9)-bicyclo[1.1.0]non-4-but-9-yl)-3,1 2-dioxo-2,7,10-trioxa-4,13-diazaheptadecan-17-yl)-3,12,19-trioxa-2,7,10-trioxa-4,13,20-triazadocos an-22-oic acid as a white solid (260 mg, yield: 88.4%). MS (ES⁺): m/z=902 (M+H)⁺.

Step 3. Preparation of 2,5-dioxopyrrolidin-1-yl-(S)-1-((1R,8S,9s)-bicyclo[6.1.0]non-4-yne-9-yl)-21-(1-((1R,8S,9s)-bicycl o[6.1.0]non-4-yne-9-yl)-3,12-dioxo-2,7,10-trioxo-4,13-diazaheptadecan-17-yl)-3,12,19-trioxo-2,7,1 0-trioxo-4,13, 20-triaza-22-acid ester (BPL-3): (S)-1-((1R,8S,9S)-bicyclo[6.1.0]non-4-but-9-yl)-21-(1-(1R,8S,9)-bicyclo[1.1.0]non-4-but-9-yl)-3,1 2-dioxo-2,7,10-trioxa-4,13-diazaheptadecan-17-yl)-3,12,19-trioxa-2,7,10-trioxa-4,13,20-triazadocos an-22-oic acid (260 mg, 0.288 mmol, 1.0 eq), N-hydroxysuccinimide (40 mg, 0.35 mmol, 1.2 eq) and 1-(3-dimethylaminopropyl)-3-ethylcarbodiimide hydrochloride (121 mg, 0.634 mmol, 2.2 eq) were dissolved in dichloromethane. The mixture was stirred at room temperature for 1.5 h. The reaction was monitored by LC-MS until completion. The reaction mixture was diluted with dichloromethane and extracted. The organic layer was washed twice with saturated sodium bicarbonate solution, followed by two washes with saturated ammonium chloride solution. It was then dried over anhydrous sodium sulfate, and the organic phase was concentrated under reduced pressure. A white solid 2,5-dioxopyrrolidin-1-yl-(S)-1-((1R,8S,9s)-bicyclo[6.1.0]non-4-yne-9-yl)-21-(1-((1R,8S,9s)-bicycl o[6.1.0]non-4-yne-9-yl)-3,12-dioxo-2,7,10-trioxo-4,13-diazaheptadecan-17-yl)-3,12,19-trioxo-2,7,1 0-trioxo-4,13, 20-triaza-22-acid ester (200 mg, yield: 69.7%) was given and used directly in the next step without purification. MS (ES⁺): m/z=999 (M+H)⁺.

### Embodiment 12 Synthesis of Solid-Phase Carrier Q22-CPG

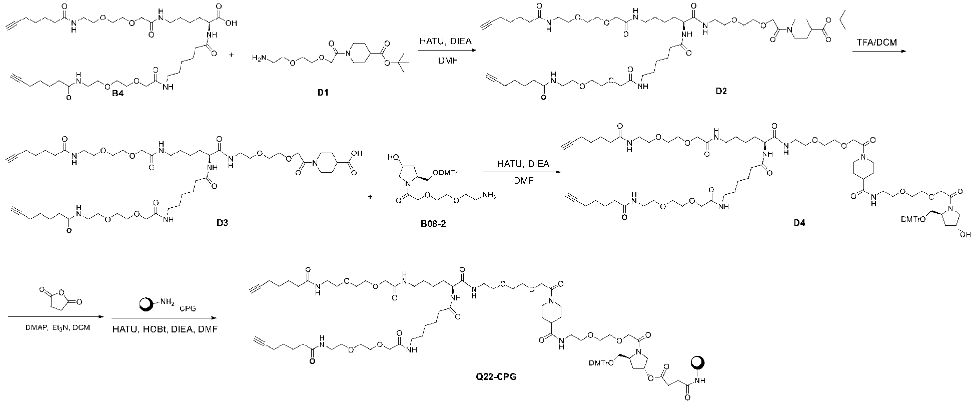

Step 1. A mixture of B4 (5.0 g, 6.53 mmol, 1.0 eq), D1 (2.26 g, 6.86 mmol, 1.05 eq), DIEA (1.68 g, 13.06 mmol, 2.0 eq), and HATU (3.72 g, 9.8 mmol, 1.5 eq) in DMF (10 mL) was stirred at room temperature for 2 h. The reaction system was diluted with ethyl acetate and washed twice each with saturated water, saturated Na₂CO₃ solution, and NH₄Cl solution. The organic phase was washed with brine, dried over anhydrous sodium sulfate, and concentrated to give 6.14 g of crude D2. MS (ES⁺): m/z= 1079.46(M+H)⁺
Step 2. In 5 mL of dichloromethane, 6.14 g of crude product D2 was dissolved. The mixture was added with 5 mL of trifluoroacetic acid while stirring, and the system was stirred at room temperature for 1 h. The solvent was concentrated under reduced pressure. The residue was diluted with dichloromethane, washed with brine, dried over anhydrous sodium sulfate, and concentrated to give 5.53 g of crude D3. MS (ES⁺): m/z= 1023.25(M+H)⁺
Step 3. A mixture of 5.53 g of crude D3, B08-2 (3.2 g, 5.67 mmol), DIEA (1.4 g, 10.8 mmol), and HATU (3.07 g, 8.1 mmol) in DMF (10 mL) was stirred at room temperature for 2 h. The reaction system was diluted with ethyl acetate and washed twice with water and twice with saturated Na₂CO₃ solution. The organic phase was washed with brine, dried over anhydrous sodium sulfate, and concentrated. Purification by RP-HPLC was performed to give the white target product D4 (5.1 g, three-step reaction yield: 49.7%).

¹H NMR (500 MHz, DMSO-*d*₆) δ 7.90 - 7.77 (m, 5H), 7.61 (t, *J =* 5.9 Hz, 2H), 7.35 - 7.26 (m, 4H), 7.21 - 7.17 (m, 4H), 6.91 - 6.84 (m, 4H), 4.97 (s, 1H), 4.42 - 4.36 (m, 1H), 4.27 (d, *J =* 14.0 Hz, 1H), 4.19 (td, *J =* 8.6, 5.1 Hz, 2H), 4.11 (t, *J =* 13.4 Hz, 2H), 4.06 (s, 1H), 3.85 (d, *J =* 1.7 Hz, 4H), 3.73 (s, 7H), 3.62 - 3.46 (m, 17H), 3.46 - 3.35 (m, 9H), 3.19 (p, *J =* 7.5, 6.6 Hz, 9H), 3.10 - 3.01 (m, 5H), 2.90 (d, *J =* 12.7 Hz, 1H), 2.72 (d, *J* = 2.6 Hz, 2H), 2.54 (s, 1H), 2.43 - 2.30 (m, 1H), 2.18 - 2.04 (m, 11H), 2.01 (dd, *J =* 12.5, 5.9 Hz, 1H), 1.84 (ddd, *J =* 12.9, 8.3, 4.4 Hz, 1H), 1.64 (s, 2H), 1.56 (p, *J =* 7.5 Hz, 5H), 1.43 (ddq, *J* = 24.8, 17.3, 10.1, 8.8 Hz, 14H), 1.22 (q, *J =* 7.5 Hz, 4H). MS (ES⁺):m/z= 1267.30[(M-303)+H]⁺.

Step 4. D4 (5.1 g, 3.25 mmol, 1 eq) was dissolved in dichloromethane (20 mL). Under stirring, succinic anhydride (1.95 g, 19.5 mmol, 6.0 eq), triethylamine (3.94 g, 39 mmol, 12.0 eq), and 4-dimethylaminopyridine (39 mg, 0.33 mmol, 0.1 eq) were sequentially added. The reaction system was allowed to proceed at room temperature overnight. The reaction system was diluted with dichloromethane, then washed three times with 10% sodium bicarbonate solution and once with saturated sodium chloride solution. The organic phase was dried over anhydrous sodium sulfate, then concentrated to give a brown oil. MS (ES⁺): m/z = 1367.35 [(M-303)+H]⁺.

The above oil (5.26 g) was dissolved in DMF. Under stirring, DIEA (1.63 g, 12.6 mmol), HATU (1.44 g, 3.78 mmol), and HOBt (510 mg, 3.78 mmol) were added. The mixture was stirred at room temperature for 5 min, and 5.3 g of controlled-pore glass carrier (CPG, substitution rate: 1.2 mmol/g) was added to the reaction mixture. The system was stirred overnight at room temperature, filtered, and the filter cake was sequentially washed with dichloromethane, acetonitrile, and dichloromethane, then dried under vacuum for 1 h. The dried solid-phase carrier was placed in pyridine/acetic anhydride (20 mL/7 mL) and shaken at room temperature for 3 h. The mixture was filtered, and the filter cake was successively washed with dichloromethane, acetonitrile, and dichloromethane and then dried under vacuum for 1 h to give a light yellow solid-phase carrier Q22-CPG (10.2 g).

Other compounds of the same type may be obtained by referring to the aforementioned preparation method.

### Embodiment 13 siRNA Synthesis and Annealing

On the LGC MerMade 12-channel nucleic acid synthesizer, oligonucleotides were synthesized at a scale of 10-40 µmol using the solid-phase oligonucleotide synthesis protocol. An ammonolysis reagent was added to the synthesized oligonucleotide and the mixture was incubated at 45-80°C to separate the oligonucleotide from the solid support, releasing it into solution. The crude oligonucleotide was then precipitated with ethanol. After high-speed centrifugation, the supernatant was discarded. This was repeated twice to give the crude oligonucleotide and the precipitate was resuspended in DEPC-treated water. The crude oligonucleotide was purified by ion-pairing HPLC, and the collected product was dried to powder in a vacuum centrifugal dryer. The purified product was dissolved in DEPC-treated water and analyzed by TOF LC-MS.

After the purified peptide-sense strand conjugate was obtained, the nucleotide concentration was determined by dissolving it in DEPC-treated water. The volume required for an equimolar amount of sense and antisense strands was calculated. Equimolar amounts of the peptide-sense strand and the antisense strand were mixed thoroughly. The peptide-siRNA double strand was prepared by heating at 75°C for 5 min, followed by annealing through natural cooling to room temperature. After the product was cooled to room temperature through annealing, it was diluted with 10 volumes of DEPC-treated water and then transferred to a Millipore 15 mL ultrafiltration centrifuge tube (molecular weight cut-off: 3 kDa) for ultrafiltration. This step aimed to remove residual chemical reagents (such as n-hexylamine) from the peptide and nucleic acid preparation process. The ultrafiltration centrifuge tube was centrifuged at 4000 RPM for approximately 40 min. Once the product volume was reduced to about 1 mL, the product was collected from the ultrafiltration tube and any residual product in the tube was washed with 3 mL of DEPC-treated water. The ultrafiltration product was sterilized by filtration through a 0.22 µm membrane. The collected product was then dried to a powder form in a vacuum centrifugal dryer. After dissolution in an appropriate solvent, it could be used for subsequent *in vivo* and *in vitro* experiments.

### Embodiment 14 Synthesis of Peptide-Oligonucleotide Conjugate Ang2(K10N₃)-12006a.1-1

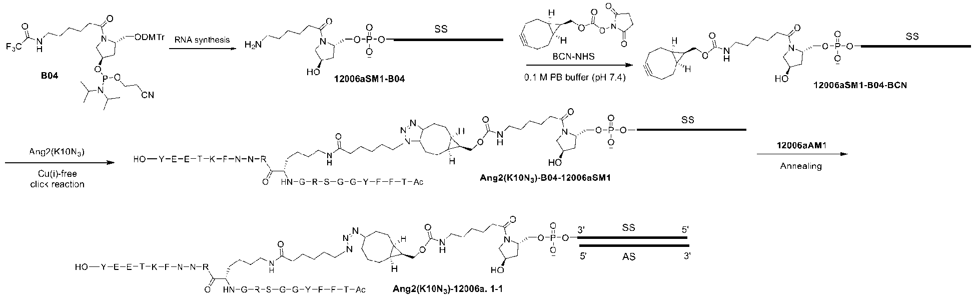

1. Using conventional nucleotide monomers and special monomer B04, synthesis and purification were performed using the standard solid-phase oligonucleotide program according to the method of Embodiment 13 to obtain an oligonucleotide sense strand 12006aSM1-B04 (33 mg, 4.56 µmol) containing B04 modification at the 3'-terminus (theoretical molecular weight: 7230.8, measured molecular weight: 7230.8).
2. Synthesis of siRNA-BCN: In 30 mL of phosphate buffer (0.1 M, pH 7.4), 12006aSM1-B04 (33 mg, 4.56 µmol) was dissolved; in 15 mL of DMF, BCN-NHS (93 mg, 320 µmol) was dissolved. The DMF solution of BCN-NHS was mixed with the phosphate buffer of 12006aSM1-B04, and the mixture was shaken at 40°C for 30 min. The reaction was monitored by LC-MS until completion, then 4.5 mL of 3M sodium chloride solution and 150 mL of pre-chilled ethanol were added to the reaction mixture. After thorough shaking, the mixture was centrifuged for 3 min (4°C, 15,000 rpm), the supernatant was discarded, and the precipitate was dissolved in approximately 3 mL DEPC-treated water. Then, the mixture was purified by HPLC and freeze-dried to give 12006aSM1-B04-BCN as a white powder solid (23.4 mg, 3.16 µmol, yield: 69.3%) (theoretical molecular weight: 7407.02, measured molecular weight: 7408.14).
3. In 2.5 mL of phosphate buffer (0.1 M, pH 7.4), 12006aSM1-B04-BCN (5 mg, 0.85 µmol) was dissolved; in 1 mL of DMF, Ang2(K10N₃) (2.5 mg, 1.0 µmol) was dissolved. The phosphate buffer containing 12006aSM1-B04-BCN was mixed with the DMF solution of Ang2(K10N₃). The mixture was shaken for 2 h under a nitrogen atmosphere, and the reaction was monitored by LC-MS until completion. Then, 0.3 mL of 3 M sodium chloride solution and 10 mL of pre-chilled ethanol were added to the reaction mixture. After thorough shaking, the mixture was centrifuged for 3 min (4°C, 15,000 rpm), the supernatant was discarded, and the precipitate was dissolved in approximately 2 mL of DEPC-treated water. Then, the mixture was purified by HPLC and freeze-dried to give Ang2(K10N₃)-B04-12006aSM1 as a white powder solid (3.5 mg, 0.35 µmol, yield: 41%) (theoretical molecular weight: 9889.73, measured molecular weight: 9890.58).
4. Ang2(K10N₃)-B04-12006aSM1 was mixed with 12006aAM1 and annealed according to the method in Embodiment 13 to give Ang2(K10N₃)-12006a.1-1.

### Embodiment 15 Synthesis of Peptide-Oligonucleotide Conjugate Ang2(K10N₃)-12006.1-1

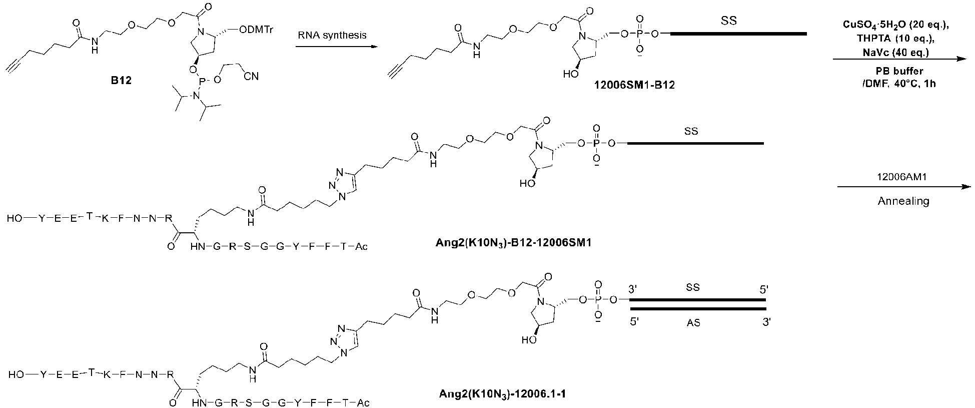

1. Using conventional nucleotide monomers and special monomer B12, synthesis and purification were performed using the standard solid-phase oligonucleotide program according to the method of Embodiment 13 to obtain alkyne-terminated oligonucleotide sense strand 12006SM1-B12 at the 3'-terminus (theoretical molecular weight: 7388.78, measured molecular weight: 7387.59).
2. In 2.5 mL of phosphate buffer (0.1 M, pH 7.4), 12006SM1-B12 (10 mg, 1.35 µmol) was dissolved; in 1 mL of DMF, Ang2(K10N₃) (5 mg, 2.0 µmol) was dissolved. The phosphate buffer of 12006SM1-B12 was mixed with the DMF solution of Ang2(K10N₃). Under a nitrogen atmosphere, pentahydrate CuSO₄ (6.8 mg, 27 µmol), tri-(3-hydroxypropyl-triazolylmethyl)amine (THPTA, 5.9 mg, 13.5 µmol), and sodium L-ascorbate (NaVc, 10.3 mg, 54 µmol) were added and reacted at 40°C for 1 h. The reaction was monitored by LC-MS until completion, then 0.3 mL of 3 M sodium chloride solution and 10 mL of pre-chilled ethanol were added to the reaction mixture. After thorough shaking, the mixture was centrifuged for 3 min (4°C, 15,000 rpm), the supernatant was discarded, and the precipitate was dissolved in approximately 2 mL of DEPC-treated water. Then, the mixture was purified by HPLC and freeze-dried to give Ang2(K10N₃)-B12-12006SM1 as a white powder solid (4.6 mg, 0.47 µmol, yield: 35%) (theoretical molecular weight: 9868.88, measured molecular weight: 9869.42).
3. Ang2(K10N₃)-B12-12006SM1 was mixed with 12006AM1 and annealed according to the method in Embodiment 13 to give Ang2(K10N₃)-12006.1-1.

### Embodiment 16 Synthesis of Peptide-Oligonucleotide Conjugate BiAng2(K10N₃)-12006.1-1

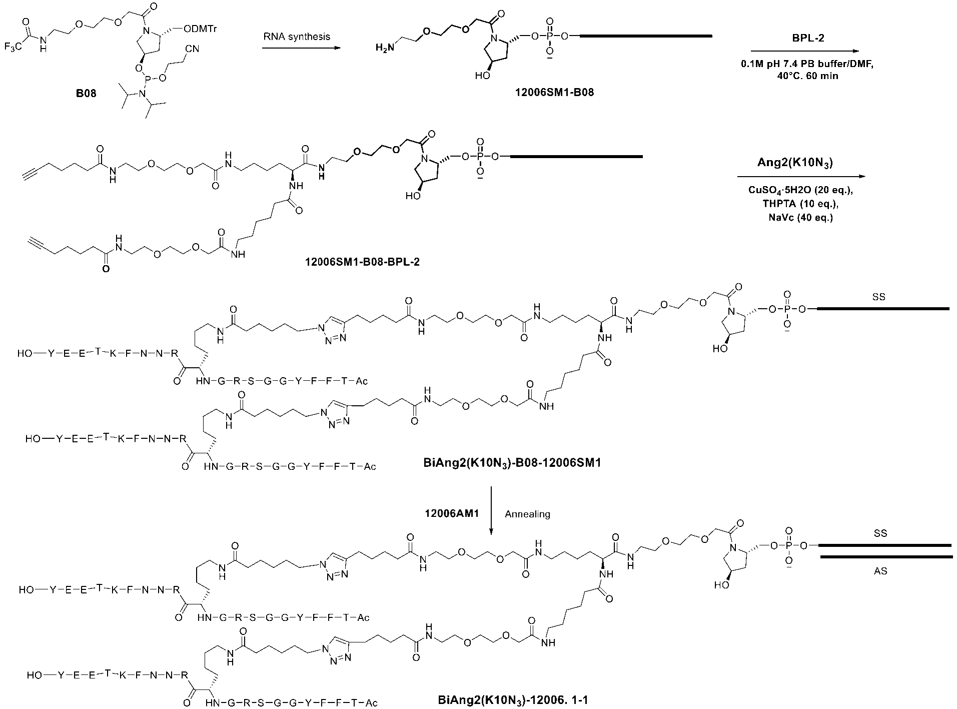

1. Using conventional nucleotide monomers and special monomer B08, synthesis and purification were performed using the standard solid-phase oligonucleotide program according to the method of Embodiment 13 to obtain amino-containing oligonucleotide sense strand 12006SM1-B08 at the 3'-terminus (theoretical molecular weight: 7279.63, measured molecular weight: 7279.64).
2. In 20 mL of phosphate buffer (0.1 M, pH 7.4), 12006SM1-B08 (160 mg, 22 µmol) was dissolved; in 10 mL of DMF, BPL-2 (569 mg, 660 µmol) was dissolved. The DMF solution of BPL-2 was mixed with the phosphate buffer of 12006SM1-B08, and the mixture was shaken at 40°C for 60 min. The reaction was monitored by LC-MS until completion, then 3 mL of 3M sodium chloride solution and 90 mL of pre-chilled ethanol were added to the reaction mixture. After thorough shaking, the mixture was centrifuged for 3 min (4°C, 15,000 rpm), the supernatant was discarded, and the precipitate was dissolved in approximately 5 mL DEPC-treated water. Then, the mixture was purified by HPLC and freeze-dried to give 12006SM1-B08-BPL-2 as a white powder solid (120 mg, 14.9 µmol, yield: 68%) (theoretical molecular weight: 8028.56, measured molecular weight: 8027.78).
3. In 10 mL of phosphate buffer (0.1 M, pH 7.4), 12006SM1-B08-BPL-2 (20 mg, 2.5 µmol) was dissolved; in 10 mL of DMF, Ang2 (K10N₃) (30 mg, 12 µmol) was dissolved. The DMF solution of Ang2(K10N₃) was mixed with the phosphate buffer of 12006SM1-B08-BPL-2. Under a nitrogen atmosphere, pentahydrate CuSO₄ (12.5 mg, 50 µmol), tri-(3-hydroxypropyl-triazolylmethyl)amine (THPTA, 10.9 mg, 25 µmol), and sodium L-ascorbate (NaVc, 19.1 mg, 100 µmol) were added and reacted at 40°C for 2 h. The reaction was monitored by LC-MS until completion, then 0.3 mL of 3M sodium chloride solution and 90 mL of pre-chilled ethanol were added to the reaction mixture. After thorough shaking, the mixture was centrifuged for 3 min (4°C, 15,000 rpm), the supernatant was discarded, and the precipitate was dissolved in approximately 2 mL of DEPC-treated water. Then, the mixture was purified by HPLC and freeze-dried to give BiAng2(K10N₃)-B08-12006SM1 as a white powder solid (9.4 mg, 0.72 µmol, yield: 29%) (theoretical molecular weight: 12,993.98, measured molecular weight: 12,992.92).
4. BiAng2(K10N₃)-B08-12006SM1 was mixed with 12006AM1 and annealed according to the method in Embodiment 13 to give BiAng2(K10N₃)-12006.1-1.

### Embodiment 17 Synthesis of BiAng2-12006a.1-1

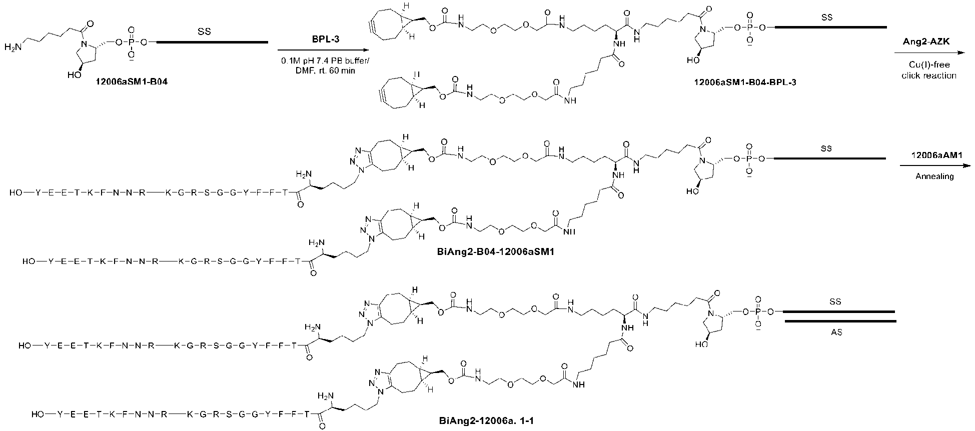

1. In 4 mL of phosphate buffer (0.1 M, pH 7.4), 12006aSM1-B04 (9 mg, 1.24 µmol) was dissolved; in 4 mL of DMF, BPL-3 (61 mg, 70 µmol) was dissolved. The DMF solution of BPL-3 was mixed with the phosphate buffer of 12006aSM1-B04, and the mixture was shaken at room temperature for 60 min. The reaction was monitored by LC-MS until completion, then 0.8 mL of 3M sodium chloride solution and 30 mL of pre-chilled ethanol were added to the reaction mixture. After thorough shaking, the mixture was centrifuged for 3 min (4°C, 15,000 rpm), and the supernatant was discarded. Then, the mixture was purified by HPLC to give 12006aSM1-B04-BPL-3 (3.9 mg, 0.48 µmol, yield: 38.7%) (theoretical molecular weight: 8115.89, measured molecular weight: 8115.57).
2. In 2.5 mL of phosphate buffer (0.1 M, pH 7.4), 12006aSM1-B04-BPL-3 (3.9 mg, 0.48 µmol) was dissolved; in 1.5 mL of DMF, Ang2-AZK (4.7 mg, 1.91 µmol) was dissolved. The phosphate buffer containing 12006aSM1-B04-BPL-3 was mixed with the DMF solution of Ang2-AZK. The mixture was shaken for 2 h under a nitrogen atmosphere, and the reaction was monitored by LC-MS until completion. Then, 0.7 mL of 3 M sodium chloride solution and 20 mL of pre-chilled ethanol were added to the reaction mixture. After thorough shaking, the mixture was centrifuged for 3 min (4°C, 15,000 rpm), the supernatant was discarded, and the precipitate was dissolved in approximately 2 mL of DEPC-treated water. Then, the mixture was purified by HPLC and freeze-dried to give BiAng2-B04-12006aSM1 as a white powder solid (3.1 mg, 0.24 µmol, yield: 47.9%) (theoretical molecular weight: 13,027.26, measured molecular weight: 13,027.09).
3. BiAng2-B04-12006aSM1 was mixed with 12006aAM1 and annealed according to the method in Embodiment 13 to give BiAng2-12006a.1-1.

### Embodiment 18 Synthesis of BiAng2-2-12006a.1-1

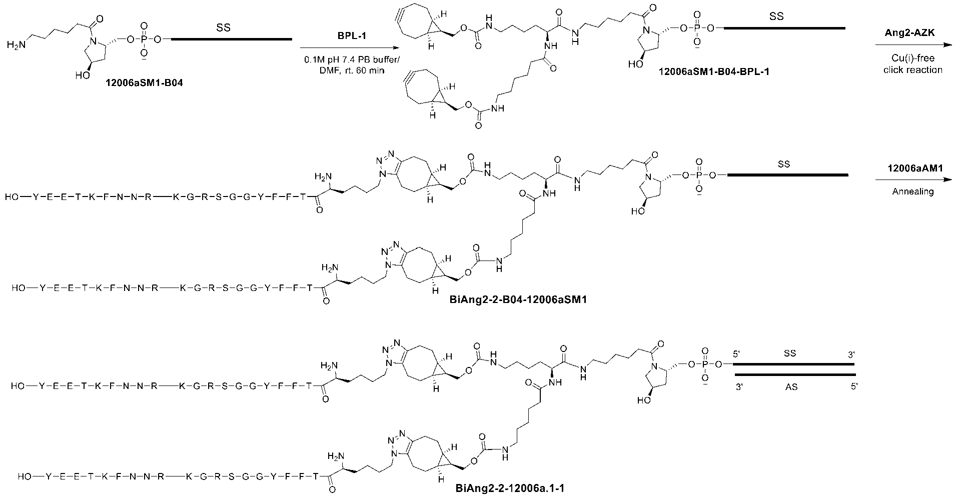

1. Synthesis of siRNA-BCN: In 5 mL of phosphate buffer (0.1 M, pH 7.4), 12006aSM1-B04 (9 mg, 1.24 µmol) was dissolved; in 5 mL of DMF, BPL-1 (61 mg, 70 µmol) was dissolved. The DMF solution of BPL-1 was mixed with the phosphate buffer of 12006aSM1-B04, and the mixture was shaken at room temperature for 60 min. The reaction was monitored by LC-MS until completion, then 1 mL of 3M sodium chloride solution and 30 mL of pre-chilled ethanol were added to the reaction mixture. After thorough shaking, the mixture was centrifuged for 3 min (4°C, 15,000 rpm), the supernatant was discarded, and the precipitate was dissolved in approximately 3 mL DEPC-treated water. Then, the mixture was purified by HPLC and freeze-dried to give 12006aSM1-B04-BPL-1 as a white powder solid (6.8 mg, 0.87 µmol, yield: 70.2%) (theoretical molecular weight: 7826.58, measured molecular weight: 7826.49).
2. Cu(I)-free catalyzed click chemistry reaction: In 3.5 mL of phosphate buffer (0.1 M, pH 7.4), 12006aSM1-B04-BPL-1 (6.8 mg, 0.87 µmol) was dissolved; in 2 mL of DMF, Ang2-AZK (8.5 mg, 3.5 µmol) was dissolved. The phosphate buffer containing 12006aSM1-B04-BPL-1 was mixed with the DMF solution of Ang2-AZK. The mixture was shaken for 2 h under a nitrogen atmosphere at room temperature, and the reaction was monitored by LC-MS until completion. Then, 0.5 mL of 3 M sodium chloride solution and 15 mL of pre-chilled ethanol were added to the reaction mixture. After thorough shaking, the mixture was centrifuged for 3 min (4°C, 15,000 rpm), the supernatant was discarded, and the precipitate was dissolved in approximately 2 mL of DEPC-treated water. Then, the mixture was purified by HPLC and freeze-dried to give BiAng2-2-B04-12006aSM1 as a white powder solid (3.8 mg, 0.31 µmol, yield: 35.6%) (theoretical molecular weight: 12735.94, measured molecular weight: 12736.97).
3. BiAng2-2-B04-12006aSM1 was mixed with 12006aAM1 and annealed according to the method in Embodiment 13 to give BiAng2-2-12006a.1-1.

### Embodiment 19 Synthesis of BiAng2(K10N₃)-12006.2-4

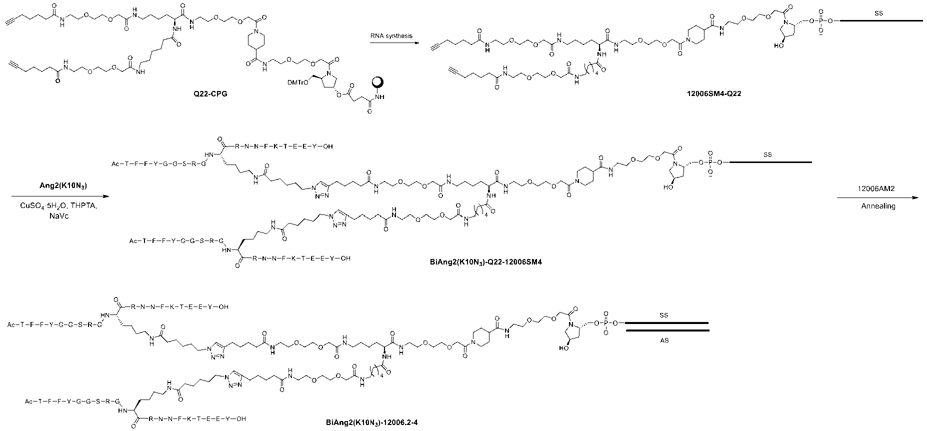

1. Synthesis of 12006SM4-Q22: Using solid-phase carrier Q22-CPG, synthesis and purification were performed using the standard solid-phase oligonucleotide program according to the method of Embodiment 13 to obtain 3'-terminally bis-alkynyl-modified oligonucleotide sense strand 12006SM4-Q22 (theoretical molecular weight: 8276.07, measured molecular weight: 8275.66)
2. Synthesis of BiAng2(K10N₃)-Q22-12006SM4: In 3 mL of phosphate buffer (0.1 M, pH 7.4), 12006SM4-Q22 (25.5 mg, 3.08 µM) was dissolved; in 3 mL of DMF, Ang2(K10N₃) (30.6 mg, 12.3 µmol) was dissolved. The phosphate buffer of 12006SM4-Q22 was mixed with the DMF solution of Ang2(K10N₃). Under a nitrogen atmosphere, pentahydrate CuSO₄ (23.1 mg, 92.4 µmol), tri-(3-hydroxypropyl-triazolylmethyl)amine (THPTA, 20.1 mg, 46.2 µmol), and sodium L-ascorbate (NaVc, 35.3 mg, 184.8 µmol) were added and reacted at 40°C for 1 h. The reaction was monitored by LC-MS until completion, then 0.6 mL of 3 M sodium chloride solution and 20 mL of pre-chilled ethanol were added to the reaction mixture. After thorough shaking, the mixture was centrifuged for 3 min (4°C, 15,000 rpm), the supernatant was discarded, and the precipitate was dissolved in approximately 2 mL of DEPC-treated water. Then, the mixture was purified by HPLC and freeze-dried to give BiAng2(K10N₃)-Q22-12006SM4 as a white powder solid (18.3 mg, 1.38 µmol, yield: 44.8%) (theoretical molecular weight: 13,241.48, measured molecular weight: 13,241.31).
3. BiAng2(K10N₃)-12006SM4 was mixed with 12006AM2 and annealed according to the method in Embodiment 13 to give BiAng2(K10N₃)-12006.2-4.

The structures of the peptide-oligonucleotide conjugates and C16-modified nucleotides synthesized by the above method are shown in Table 10 below.

**Table 10: Structures and Sequences of Peptide-Oligonucleotide Conjugates Targeting APP**

| Name | Type of Structure | Sense Strand Sequence 5'->3' | Antisense Strand Sequence 5'->3' |
|---|---|---|---|
| 12006a.1-1 | / | mC*mA*mCmAm AmGfUmUfCfUdT mUmGmAmGmC mAmAmAmUmA | VPU*fA*mUmUm UmGmCmUmCmA mAdAmGfAmAfC mUmUmG*mU*mG |
| Ang2-12006a.1-1 | Ang2-B04-BCN-siRNA | | |
| Reversed-Ang2-120 06a.1-1 | Reversed-Ang2-B04-BCN-si RNA | | |
| Ang2(K10AZK)-12 006a.1-1 | Ang2(K10AZK)-B04-BCN-s iRNA | | |
| Ang2(K10N₃)-1200 6a. 1-1 | Ang2(K10N₃)-B04-BCN-siR NA | | |
| L57-12006a. 1-1 | L57-B04-BCN-siRNA | | |
| BiAng2-12006a.1-1 | BiAng2-B04-BCN-siRNA | | |
| BiAng2-2-12006a.1-1 | BiAng2-2-B04-BCN-siRNA | | |
| Ang2-Ang2-12006a. 1-1 | Ang2-Ang2-B04-BCN-siRN A | | |
| 12006.1-1 | / | mC*mA*mCmAm AmGfUmUfCfUdT mUmGmAmGmC mAmGmAmUmA | VPU*fA*mUmCmU mGmCmUmCmAm AdAmGfAmAfCmU mUmG*mU*mG |
| BiAng2-12006.1-1 | BiAng2-B08-siRNA | | |
| BiAng2(K10N₃)-120 06.1-1 | BiAng2(K10N₃)-B08-siRNA | | |
| BiL57-12006.1-1 | BiL57-B08-siRNA | | |
| Ang2(K10N₃)-1200 6.1-1 | Ang2(K10N₃)-B12-siRNA | | |
| L57-12006.1-1 | L57-B12-siRNA | | |
| C16(6-G)-12006.1-1 | / | mC*mA*mCmAm AGhdfUmUfCfUd TmUmGmAmGmC mAmGmAmUmA | VPU*fA*mUmCmU mGmCmUmCmAm AdAmGfAmAfCmU mUmG*mU*mG |
| C16(7-U)-12006a.1-1 | / | mC*mA*mCmAm AmGUhdmUfCfU dTmUmGmAmGm CmAmAmAmUm A | VPU*fA*mUmUm UmGmCmUmCmA mAdAmGfAmAfC mUmUmG*mU*mG |
| BiAng2(K10N₃)-120 06.2-4 | BiAng2(K10N₃)-Q22-siRNA | mC*mA*mCmAm AmGmUmUfCfUf UfUmGfAmGmCm AmGmAmUmA | VPU-S*fA*mUmC mUmGmCmUmCm AmAdAmGfAmAfC mUmUmG*mU*mG |
| BiAng2(K10N₃)-120 37.2-4 | BiAng2(K10N₃)-Q22-siRNA | mG*mC*mUmGm UmCmCmAfAfGf AfUmGfCmAmGm CmAmGmAmA | VPU-S*fU*mCmU mGmCmUmGmCm AmUdCmUfUmGfG mAmCmA*mG*mC |
| BiAng2(K10N₃)-120 75.2-4 | BiAng2(K10N₃)-Q22-siRNA | mC*mA*mAmGm UmCmUmAfCfCf CfUmGfAmAmCm UmGmCmAmA | VPU-S*fU*mGmC mAmGmUmUmCm AmGdGmGfUmAf GmAmCmU*mU*m G |
| BiAng2(K10N₃)-120 76.2-4 | BiAng2(K10N₃)-Q22-siRNA | mG*mG*mUmCm UmAfCfCfCfUmG fAmAmCmUmGm CmAmA | VPU-S*fU*mGmC mAmGmUmUmCm AmGdGmGfUmAf GmAmCmC*mU*m C |
| Ang2(K10N₃)-1202 4.1-1 | Ang2(K10N₃)-B12-siRNA | mC*mU*mAmCm CmCfUmGfAfAdC mUmGmCmAmG mAmUmCmAmA | VPU*fU*mGmAm UmCmUmGmCmA mGdTmUfCmAfGm GmGmUmAmG*m G*mC |
| Ang2(K10N₃)-1207 5a.1-3 | Ang2(K10N₃)-B12-siRNA | mG*mA*mGmGm UmCmUmAfCmCf CmUfGmAmAmC mUmGmCmAmA | VPU*fU*mGmCmA mGmUmUmCmAm GdGmGfUmAfGm AmCmC*mU*mC |
| Ang2(K10N₃)-1207 6.1-3 | Ang2(K10N₃)-B12-siRNA | mG*mG*mUmCm UmAfCmCfCmUf GmAmAmCmUm GmCmAmA | VPU*fU*mGmCmA mGmUmUmCmAm GdGmGfUmAfGm AmCmC*mU*mC |
| Bi-Sym-Ang2(K10N ₃)-12006.1-1 | Bi-Sym-Ang2(K10N₃)-2-B0 8-siRNA | mC*mA*mCmAm AmGfUmUfCfUdT mUmGmAmGmC mAmGmAmUmA | VPU*fA*mUmCmU mGmCmUmCmAm AdAmGfAmAfCmU mUmG*mU*mG |
| Bi-Sym-Ang2(K10P eg2N₃)-12006.1-1 | Bi-Sym-Ang2(K10Peg2N₃)-2-B08-siRNA | | |

The specific structure of the peptide-oligonucleotide conjugate is as follows:

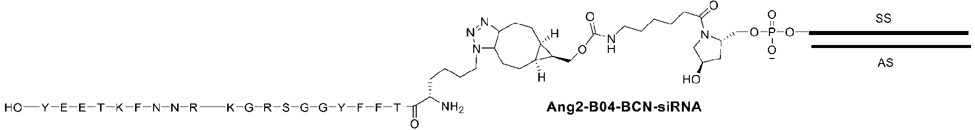

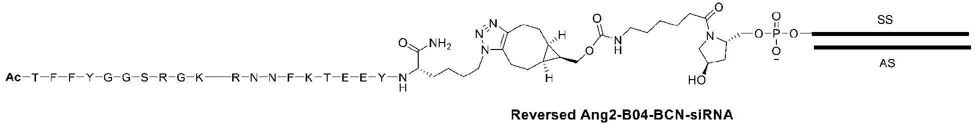

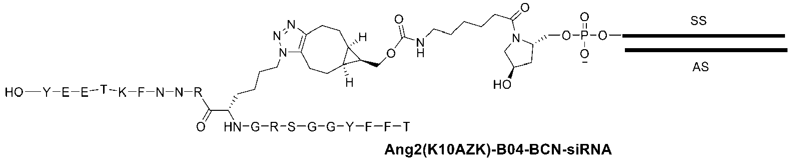

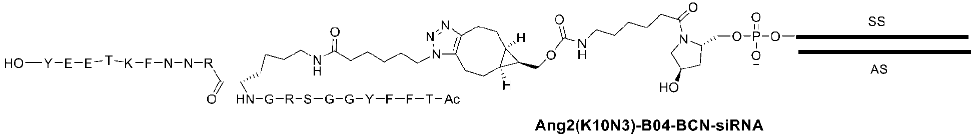

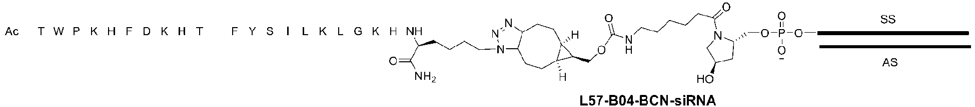

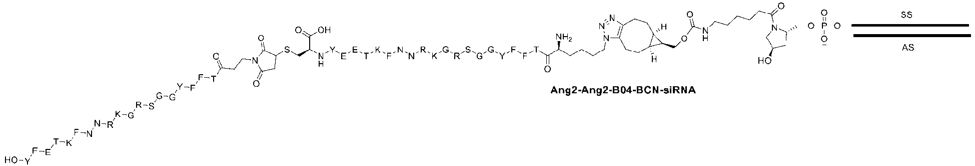

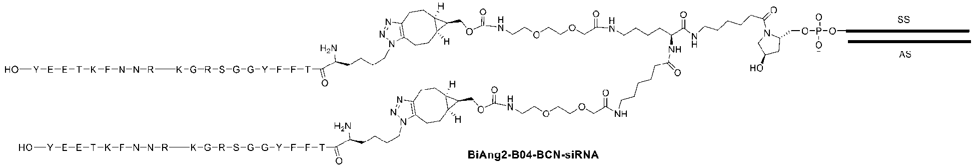

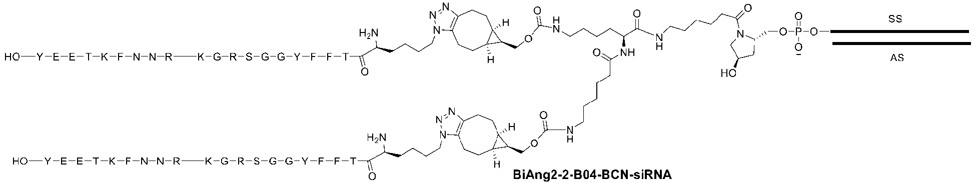

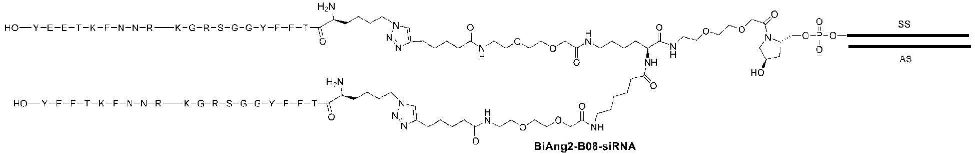

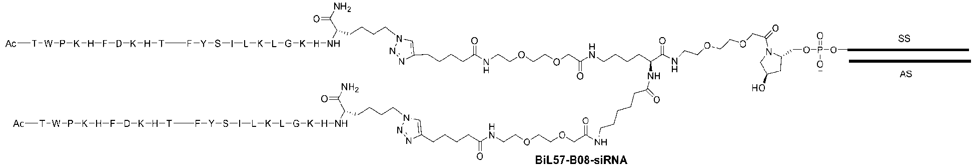

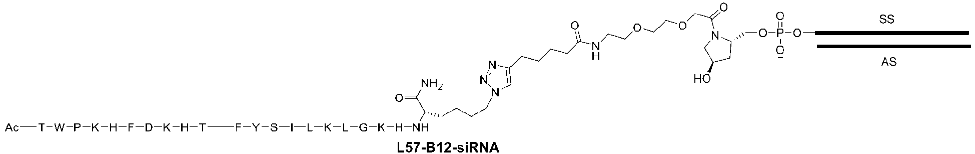

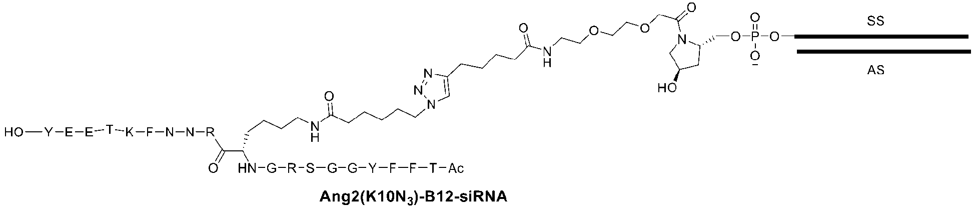

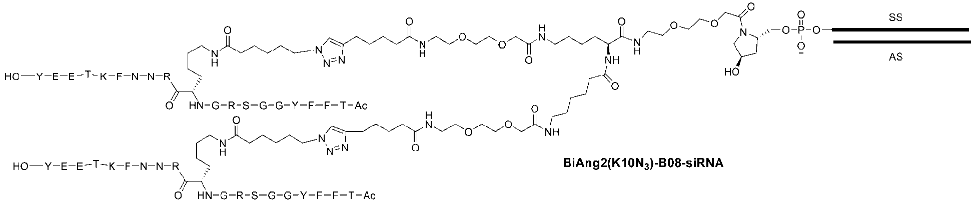

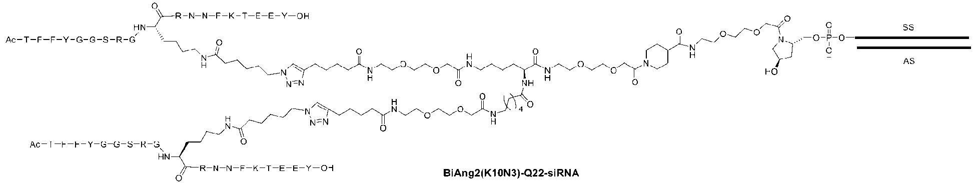

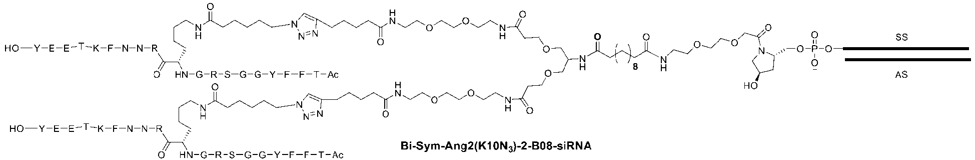

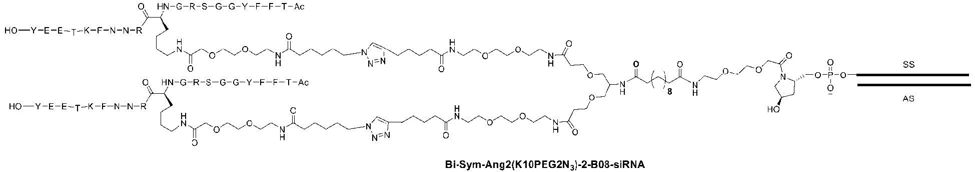

The molecular weight and purity of the synthesized peptide-oligonucleotide conjugate and the sense strand of the C16-modified nucleotide in the present invention are shown in Table 11 below.

**Table 11: Molecular Weight and Purity of Peptide-Oligonucleotide Conjugate and Sense Strand of C16-Modified Nucleotide**

| Name | Theoretical Molecular Weight of Sense Strand | Measured Molecular Weight of Sense Strand | Purity of Double-Stranded Conjugate (%) |
|---|---|---|---|
| 12006a.1-1 | / | / | 90.0 |
| Ang2-12006a.1-1 | 9864.72 | 9864.72 | 96.0 |
| Reversed-Ang2-12006a.1-1 | 9903.76 | 9904.54 | 96.5 |
| Ang2(K10AZK)-12006a.1-1 | 9734.52 | 9736.46 | 95.0 |
| Ang2(K10N₃)-12006a.1-1 | 9889.73 | 9890.58 | 90.0 |
| L57-12006a.1-1 | 10088.18 | 10086.88 | 96.1 |
| BiAng2-12006a.1-1 | 13027.26 | 13027.09 | 93.9 |
| BiAng2-2-12006a.1-1 | 12735.94 | 12736.97 | 95.9 |
| Ang2-Ang2-12006a.1-1 | 12418.47 | 12418.47 | 95.8 |
| BiAng2-12006.1-1 | 12939.94 | 12938.86 | 94.4 |
| BiAng2(K10N₃)-12006.1-1 | 12993.98 | 12992.92 | 93.1 |
| BiL57-12006.1-1 | 13386.85 | 13385.50 | 94.8 |
| Ang2(K10N₃)-12006.1-1 | 9868.88 | 9869.42 | 87.0 |
| L57-12006.1-1 | 10066.91 | 10066.67 | 90.5 |
| C16(6-G)-12006.1-1 | 7164.93 | 7164.34 | 99.01 |
| C16(7-U)-12006a.1-1 | 7160.97 | 7160.52 | 91.57 |
| BiAng2(K10N₃)-12006.2-4 | 13241.48 | 13241.31 | 99.8 |
| BiAng2(K10N₃)-12037.2-4 | 13318.57 | 13317.90 | 98.2 |
| BiAng2(K10N₃)-12075.2-4 | 13199.48 | 13199.06 | 99.1 |
| BiAng2(K10N₃)-12076.2-4 | 12553.04 | 12552.53 | 94.8 |
| Ang2(K10N₃)-12024.1-1 | 9814.73 | 9812.64 | 96.4 |
| Ang2(K10N₃)-12075a.1-3 | 9900.78 | 9900.6 | 97.9 |
| Ang2(K10N₃)-12076.1-3 | 9198.31 | 9198.54 | 98.3 |
| Bi-Sym-Ang2(K10N₃)-12006.1-1 | 13153.8 | 13150.0 | 97.6 |
| Bi-Sym-Ang2(K10Peg2N₃)-12006. 1-1 | 13444.1 | 13440.6 | 99.0 |

### Embodiment 20 In Vivo Activity of Peptide-oligonucleotide Conjugates Targeting APP Tested via Intrathecal Injection in Wild-type Mice

### 1. Drug Preparation

Following freeze-drying to obtain the peptide-siRNA double-stranded powder (peptide-oligonucleotide conjugate) targeting APP, the peptide-siRNA drug was prepared in a sterile environment using artificial cerebrospinal fluid (CSF) as the solvent, with a stock solution concentration of 20 mg/mL.

### 2. Grouping and Administration

The 4-week-old female C57 wild-type mice of similar body weight were selected as experimental animals and randomized into different groups, with 6 mice per group receiving the drug. After anesthetizing the mice, the peptide-siRNA drug was administered via intrathecal injection at a dose of 10 mg/kg. On the day of drug injection, the mouse's condition was observed after recovery from anesthesia, and daily weight records were maintained.

### 3. Sampling and Testing

Mice were euthanized on Day 10 of administration, and tissue samples were harvested from the cerebral cortex, brainstem, hypothalamus, hippocampus, and cervical spinal cord. Tissue samples were preserved in RNA Later to prevent RNA degradation. RNA was subsequently extracted from each brain tissue using TRI REAGENT (MRC, Cat. No.: TR118). The extracted RNA was reverse transcribed into cDNA using the PrimeScript RT Reagent Kit (Takara, Cat. No.: RR047A). The prepared QPCR system was added to a 96-well PCR plate, the plate was sealed with sealing film, and QPCR was conducted on a StepOnePlus real-time PCR System (Applied Biosystems) to detect APP expression. Besides, the content of the antisense strand targeting APP in brain tissue was detected via liquid chromatography-tandem mass spectrometry.

Mice were euthanized on Day 10 of administration, and tissue samples were collected to detect APP expression levels. Results are shown in Table 4. At a dose of 10 mg/kg, the *in vivo* activity of APP siRNA varied among different peptide delivery systems. C16(7-U)-12006a.1-1 (with a hexadecyl fatty chain modification at the 7th nucleotide residue of the sense strand) served as the positive control for the delivery system, while 12006a.1-1 represented the sequence of the naked chain without conjugation to peptides or C16. Multiple peptide-siRNA sequences demonstrated significant efficacy in degrading target genes in mice. The results are shown in Table 12.

**Table 12: Activity Detection Results of Different Peptide-oligonucleotide Conjugates for Single Peptide Delivery in Various Mouse Brain Tissues**

| Peptide-oligonucleotide Conjugate | Remaining APP mRNA Level (%) (relative to the treated control group) | | | | |
|---|---|---|---|---|---|
| | Cerebral cortex | Brain stem | Hypothala mus | Hippocam pus | Cervical spinal cord |
| 12006a.1-1 | 120.49 | 23.08 | 44.21 | 53.26 | 20.87 |
| C16(7-U)-12006a.1-1 | 41.06 | 9.19 | 16.94 | 23.29 | 7.00 |
| Ang2(K10N₃)-12006a.1-1 | 24.59 | 13.53 | 23.71 | 21.17 | 7.70 |
| Ang2(K10N₃)-12024.1-1 | 27.02 | 20.91 | 37.60 | 28.57 | 25.74 |
| Ang2(K10N₃)-12075a.1-3 | 21.79 | 10.07 | 36.87 | 29.75 | 15.04 |
| Ang2(K10N₃)-12076.1-3 | 11.15 | 3.64 | 17.23 | 34.13 | 6.03 |

Table 12 results showed that the siRNA targeting APP linked to the single peptide Ang2(K10N₃) exhibited activity across various mouse brain tissues, with Ang2(K10N₃)-12006a.1-1 and Ang2(K10N₃)-12076.1-3 exhibiting the highest activity, even surpassing the positive control C16(7-U)-12006a.1-1. While naked chain 12006a.1-1 only exhibited superior activity in the cervical spinal cord. This demonstrates that the Ang2 peptide can effectively deliver siRNA targeting APP to brain tissue cells in mice, exhibiting superior delivery efficacy compared to C16. Furthermore, based on the results in Table 8, the liver homogenate stability of 12006.1-1 was the highest. Subsequently, 12006.1-1 was selected for the conjugation of different peptides to identify the most efficient peptide delivery platform.

### Embodiment 21 Exploring the Effects of Linking Single Peptides or Dipeptides on the In Vivo Activity of Peptide-siRNA in Wild-Type Mice via Intrathecal Injection

This Embodiment explores the effects of Ang2 linkage methods (linkage sites and valence states), tandem dipeptides, and parallel dipeptides on the *in vivo* activity of peptide-siRNA. Following administration via intrathecal injection to 4-week-old female C57 wild-type mice according to the method of Embodiment 16, mice were euthanized on Day 11 and different brain tissues were collected to detect APP expression levels. The results are shown in Table 13.

**Table 13: Activity Detection Results of Different Peptide-Oligonucleotide Conjugates Targeting APP in Various Mouse Brain Tissues**

| Peptide-oligonucleotide Conjugate | Remaining APP mRNA Level (%) (relative to the treated control group) | | | | |
|---|---|---|---|---|---|
| | Cerebral cortex | Brain stem | Hypothal amus | Hippocam pus | Cervical spinal cord |
| Ang2-12006a.1-1 | 20.59 | 10.38 | 17.82 | 21.72 | 5.64 |
| Reverse-Ang2-12006a.1-1 | 59.58 | 26.84 | 56.18 | 53.59 | 3.81 |
| Ang2(K10AZK)-12006a.1-1 | 14.09 | 23.32 | 22.54 | 21.43 | 2.08 |

The results in Table 13 showed that Ang2-12006a.1-1 (siRNA conjugated to the N-terminus of the Ang2 peptide) exhibited satisfactory activity across various brain tissues, with particularly pronounced effects in regions such as the cerebral cortex and brainstem. Further exploration of Ang2 linkage methods revealed that the peptide-siRNA with APP siRNA linked to azidolysine at position 10 of Ang2 (Ang2(K10AZK)-12006a.1-1) exhibited optimal activity. Conversely, delivery efficiency was reduced when APP siRNA was linked to the C-terminal region of Ang2 (Reverse-Ang2-12006a.1-1). These results demonstrate that the azidolysine linkage at position 10 of Ang2 enables efficient delivery of APP siRNA to brain tissue cells in mice, thereby enabling the therapeutic efficacy of the APP-targeting siRNA.

**Table 14: Effects of Different Forms of Monopeptides/Dipeptides on Activity of APP-Targeting Peptide-Oligonucleotide Conjugates in Mice**

| Peptide-oligonucleotide Conjugate | Remaining APP mRNA Level (%) (relative to the treated control group) | | | | |
|---|---|---|---|---|---|
| | Cerebral cortex | Brain stem | Hypothala mus | Hippoca mpus | Cervical spinal cord |
| Ang2-12006a.1-1 | 39.43 | 16.00 | 36.32 | 51.53 | 5.82 |
| Ang2(K10N₃)-12006a.1-1 | 24.59 | 13.53 | 23.70 | 21.73 | 7.70 |
| L57-12006a.1-1 | 37.81 | 12.85 | 16.54 | 26.24 | 5.12 |
| Ang2-Ang2-12006a.1-1 | 22.49 | 9.74 | 20.63 | 25.34 | 4.77 |
| BiAng2-12006a.1-1 | 16.69 | 14.32 | 20.31 | 22.94 | 3.46 |
| BiAng2-2-12006a.1-1 | 54.26 | 11.97 | 16.11 | 28.54 | 3.62 |

The results in Table 14 showed that all peptide-oligonucleotide conjugates exhibited good activity in the brainstem and cervical spinal cord. Within the cerebral cortex, compared with connecting APP siRNA to the N-terminus of Ang2 and to the C-terminus of L57, linking APP siRNA to lysine at position 10 of Ang2 (Ang2(K10N₃)), tandem dipeptides (Ang2-Ang2), or parallel dipeptides (BiAng2) significantly enhanced the activity of peptide-siRNA, with the BiAng2-12006a.1-1 group exhibiting the highest activity. This demonstrates that linking dipeptides to siRNA enhances its delivery efficiency, and further indicates that the peptide linkage sites significantly impact the activity of peptide-siRNA. It was also found that BiAng2-2-12006a.1-1 exhibited poor activity, indicating that the structure of the linker segment significantly impacts the activity of peptide-siRNA.

### Embodiment 22 Exploring the Effects of Different Dipeptide Linkages on the In Vivo Activity of Peptide-oligonucleotide Conjugates via Intracisternal Injection in Wild-type Mice

This embodiment explores the impact of different dipeptide linkage methods on the activity of peptide-oligonucleotide conjugates. In this embodiment, except for the difference in the route of administration, the other experimental methods are the same as those in Embodiment 15. This Embodiment employs intracisternal injection for drug administration, with each mouse receiving a dose of 0.25 mg. Following drug administration, mice were euthanized on Day 17, and various brain tissues were collected to detect APP expression levels. The results are shown in Table 15.

**Table 15: Effects of Different Forms of Dipeptides on Activity of APP-Targeting Peptide-Oligonucleotide Conjugates in Mice**

| Peptide-oligonucleotide Conjugate | Remaining APP mRNA Level (%) (relative to the treated control group) | | | | |
|---|---|---|---|---|---|
| | Cerebral cortex | Brain stem | Hypothal amus | Hippocam pus | Cervical spinal cord |
| BiAng2-12006.1-1 | 15.26 | 5.69 | 37.01 | 22.00 | 10.72 |
| BiAng2(K10N₃)-12006.1-1 | 7.28 | 2.72 | 14.37 | 13.80 | 3.95 |

The results of Table 15 showed that Ang2 in parallel configuration exhibited excellent drug delivery efficacy across various brain tissues. Among these, Ang2 with siRNA connected to lysine at position 10 (BiAng2(K10N₃)-12006.1-1) displayed higher activity than Ang2 with siRNA attached at the N-terminus.

### Embodiment 23 Exploring the Effects of Monopeptide or Dipeptide Linkages on the In Vivo Activity of Peptide-oligonucleotide Conjugates via Intracisternal Injection in Wild-type Rats

This embodiment tests the *in vivo* activity of the peptide-oligonucleotide conjugate of the present invention in rats. Except for the difference in the route of administration, the other experimental methods are the same as those in Embodiment 16. In this Embodiment, the drug was administered to rats via intracisternal injection at a dose of 1 mg per rat. Rats were euthanized on Days 21 and 42, and different brain tissues were collected to detect APP expression levels. The results are shown in Tables 16 and 17.

**Table 16: Effects of Different Forms of Monopeptides or Dipeptides on 21-day Activity of APP-Targeting Peptide-Oligonucleotide Conjugates in Rats**

| Peptide-oligonucleotide Conjugate | Remaining APP mRNA Level (%) (relative to the treated control group) | | | | |
|---|---|---|---|---|---|
| | Cerebral cortex | Brain stem | Hypothal amus | Hippocam pus | Cervical spinal cord |
| BiAng2-12006.1-1 | 17.74 | 25.48 | 39.86 | 19.81 | 17.54 |
| BiAng2(K10N₃)-12006.1-1 | 5.66 | 8.62 | 20.49 | 6.34 | 7.37 |
| Ang2(K10N₃)-12006.1-1 | 6.45 | 18.01 | 42.17 | 11.19 | 13.76 |

The results in Table 16 showed that on Day 21, Ang2(K10N₃)-12006.1-1 in its single-peptide form exhibited significantly enhanced activity compared with BiAng2-12006.1-1, which was linked from the N-terminus of Ang2 in a dipeptide form. This further demonstrates that linking siRNA from lysine at position 10 of Ang2 confers higher activity. Simultaneously, it was discovered that the peptide-oligonucleotide conjugate in the dual Ang2 (K10N₃) form (BiAng2(K10N₃)-12006.1-1) exhibited further enhanced activity compared with the peptide-oligonucleotide conjugate in the single Ang2 (K10N₃) form (Ang2(K10N₃)-12006.1-1), further demonstrating that linking dual peptides to siRNA enhances its delivery efficiency.

**Table 17: Effects of Dipeptides with Different Linkage Methods on the 42-Day Activity of APP-Targeting Peptide-Oligonucleotide Conjugates in Rats**

| Peptide-oligonucleotide Conjugate | Remaining APP mRNA Level (%) (relative to the treated control group) | | | | |
|---|---|---|---|---|---|
| | Cerebral cortex | Brain stem | Hypothal amus | Hippocam pus | Cervical spinal cord |
| BiAng2-12006.1-1 | 13.77 | 23.61 | 34.56 | 21.71 | 21.44 |
| BiAng2(K10N₃)-12006.1-1 | 10.13 | 7.42 | 12.35 | 8.66 | 6.62 |

The results in Table 17 showed that the dual Ang2 (K10N₃) form peptide-siRNA (BiAng2(K10N₃)-12006.1-1) demonstrated superior activity maintenance compared to the dipeptide form BiAng2-12006.1-1 on Day 42. It was also found that BiAng2(K10N₃)-12006.1-1 maintained extremely high target inhibitory activity on Day 42, indicating that the BiAng2(K10N₃) structure can deliver siRNA with exceptionally long-lasting efficacy.

This embodiment also investigates the effects of different forms of dipeptides on the 26-day activity of peptide-oligonucleotide conjugates in rats. Optimizations were performed on the original dipeptide structure by modifying the symmetric peptide and introducing polyethylene glycol (PEG) into the symmetric peptide. The results are shown in Table 18.

**Table 18: Effects of Different Forms of Dipeptides on 26-day Activity of APP-Targeting Peptide-Oligonucleotide Conjugates in Rats**

| Peptide-oligonucleotide Conjugate | Remaining APP mRNA Level (%) (relative to the treated control group) | | | |
|---|---|---|---|---|
| | Cerebral cortex | Brain stem | Hypotha lamus | Cervical spinal cord |
| BiAng2(K10N₃)-12006.1-1 | 10.3284 | 17.65 | 20.47 | 10.17 |
| Bi-sym-Ang2(K10N₃)-12006.1-1 | 11.8858 | 20.19 | 26.61 | 6.562 |
| Bi-sym-Ang2(K10Peg2N₃)-12006.1-1 | 19.0382 | 77.67 | 30.25 | 18.98 |

The results in Table 18 showed that on Day 26, the BiAng2(K10N₃)-12006.1-1 peptide-oligonucleotide conjugate with an asymmetric linker in dual Ang2 (K10N₃) form, and the Bi-sym-Ang2(K10N₃)-12006.1-1 peptide-oligonucleotide conjugate with a symmetric linker in dual Ang2 (K10N₃) form exhibited superior activity maintenance compared to Bi-sym-Ang2(K10Peg2N₃)-12006.1-1. This indicates that the use of the BiAng2(K10N₃) structure for siRNA delivery is the most efficient delivery structure among the current embodiments.

### Embodiment 24 Exploring the In Vivo Activity of Low-Dose Peptide-Oligonucleotide Conjugates via Intracisternal Injection in Wild-Type Rats

This Embodiment compares the *in vivo* activity of the peptide-oligonucleotide conjugate (BiAng2(K10N₃)-12006.1-1) of the present invention with that of the hexadecyl chain-modified oligonucleotide C16(6-G)-12006.1-1 in rats (C16(6-G) -12006.1-1 features a hexadecyl fatty chain modification at the 6th nucleotide residue of its sense strand; hexadecyl modification at the 6th nucleotide residue is an effective strategy for enhancing nucleic acid delivery efficiency), against naked siRNA without conjugation to peptides or C16 as controls. In this Embodiment, the drug was also administered to rats via intracisternal injection, with the dose reduced to 0.3 mg per rat. On Day 14, rats were euthanized, and different brain tissues were collected to detect APP expression levels. The results are shown in Table 19.

**Table 19: Comparison of 14-day Activity in Rats between Low-dose APP-targeting Peptide-oligonucleotide Conjugates and C16-siRNA**

| Peptide-oligonucleotide Conjugate | Remaining APP mRNA Level (%) (relative to the treated control group) | | | | |
|---|---|---|---|---|---|
| | Cerebral cortex | Brain stem | Hypothalamus | Hippocampus | Cervical spinal cord |
| 12006.1-1 | 34.71 | 41.74 | 52.10 | 42.23 | 32.94 |
| BiAng2(K10N₃)-12006.1-1 | 9.28 | 13.28 | 25.34 | 11.95 | 8.96 |
| C16(6-G)-12006.1-1 | 26.37 | 12.53 | 41.14 | 19.63 | 20.68 |

The results in Table 19 showed that at low doses, the dipeptide form BiAng2(K10N₃)-12006.1-1 exhibited significantly enhanced activity compared with C16(6-G)-12006.1-1 delivered by C16 on Day 14. This demonstrates that peptide-delivered siRNA exhibits greater specificity and efficiency compared with C16-delivered siRNA. Additionally, lower injection doses can reduce toxic side effects, making peptides an ideal platform for delivering siRNA to the central nervous system.

### Embodiment 25 Sequence Optimization of siRNA Targeting APP

The results from the preceding Embodiments indicate that BiAng2(K10N₃) serves as an efficient peptide platform for delivering APP siRNA to the brains of mice and rats. Next, we will modify and optimize the sequence targeting APP siRNA. Based on the data from Embodiment 2, 12006, 12037, 12075, and 12076 were selected for optimization. The *in vitro* results are shown in Tables 20, 21, and 22.

**Table 20: Experimental Results of 10 nM and 1 nM Modified siRNA Transfection via Liposome in U87-MG Cells**

| siRNA Duplex | Residual APP mRNA Levels in U87-MG Cells (%) (relative to negative control cells) | | | |
|---|---|---|---|---|
| | Mean at 10 nM | Standard Deviation at 10 nM | Mean at 1 nM | Standard Deviation at 1 nM |
| 12006.1-1 | 7.57 | 2.69 | 11.13 | 3.21 |
| 12006.2-4 | 3.60 | 1.67 | 6.32 | 2.82 |
| 12075.1-1 | 8.41 | 1.24 | 5.79 | 0.31 |
| 12075.2-4 | 7.39 | 4.42 | 8.29 | 2.99 |
| 12076.1-1 | 9.61 | 2.54 | 13.96 | 1.58 |
| 12076.2-4 | 9.15 | 5.29 | 9.73 | 1.58 |

**Table 21: Experimental Results of 0.1 nM and 1 nM Modified siRNA Transfection via Liposome in U87-MG Cells**

| siRNA Duplex | Residual APP mRNA Levels in U87-MG Cells (%) (relative to negative control cells) | | | |
|---|---|---|---|---|
| | Mean at 1 nM | Standard Deviation at 1 nM | Mean at 0.1 nM | Standard Deviation at 0.1 nM |
| AD-454973 | 5.05 | 0.54 | 24.54 | 0.87 |
| 12006.2-4 | 1.85 | 0.26 | 11.51 | 2.64 |
| 12037.2-4 | 2.38 | 0.51 | 18.09 | 5.35 |
| 12075.2-4 | 2.58 | 0.29 | 11.67 | 2.84 |
| 12076.2-4 | 3.04 | 0.80 | 14.22 | 1.45 |

**Table 22: Experimental Results of 0.1 nM and 1 nM Modified siRNA Transfection via Liposome in Hep3B Cells**

| siRNA Duplex | Residual APP mRNA Levels in Hep3B Cells (%) (relative to negative control cells) | | | |
|---|---|---|---|---|
| | Mean at 1 nM | Standard Deviation at 1 nM | Mean at 0.1 nM | Standard Deviation at 0.1 nM |
| AD-454973 | 2.92 | 0.27 | 10.00 | 2.17 |
| 12006.2-4 | 1.66 | 0.11 | 6.00 | 0.55 |
| 12037.2-4 | 1.89 | 0.30 | 8.32 | 0.64 |
| 12075.2-4 | 2.08 | 0.09 | 5.00 | 0.43 |
| 12076.2-4 | 2.56 | 0.11 | 6.03 | 1.45 |

**Table 23: Experimental Results of 10 nM and 1 nM Modified siRNA Transfection via Liposome in Mouse Primary Hepatocytes**

| siRNA Duplex | Residual APP mRNA Levels in Mouse Primary Hepatocytes (%) (relative to negative control cells) | |
|---|---|---|
| | Mean at 1 nM | Standard Deviation at 1 nM |
| AD-454973 | 109.25 | 10.54 |
| 12006.2-4 | 1.56 | 0.24 |
| 12037.2-4 | 0.69 | 0.14 |
| 12075.2-4 | 0.74 | 0.07 |
| 12076.2-4 | 0.56 | 0.09 |

**Table 24: Stability Results of siRNA Targeting APP in Brain Homogenate after Sequence Optimization**

| siRNA | Residual Percentage of Antisense Strand at 36 h (%) |
|---|---|
| AD-454913 | 65.11 |
| 12006.2-4 | 76.44 |
| 12037.2-4 | 89.09 |
| 12075.2-4 | 80.29 |
| 12076.2-4 | 93.62 |

The above results indicate that multiple sequences exhibit greater stability than the positive control. Subsequent *in vivo* experiments will utilize this modification for screening.

### Embodiment 26 Activity Verification of APP-Targeting Peptide-Oligonucleotide Conjugates after Sequence Optimization in Mice

This Embodiment employs an intraspinal injection method for drug administration, with each mouse receiving a dose of 0.25 mg. Following the injection of the sequence-optimized APP-targeting peptide-oligonucleotide conjugate into the mice, the animals were euthanized on Day 7, and different brain tissues were then collected to detect mAPP expression levels. The results are shown in Table 25.

**Table 25: Activity Detection Results of Different Peptide-oligonucleotide Conjugates Delivered by BiAng2(K10N₃) in Various Mouse Brain Tissues**

| Peptide-oligonucleotide Conjugate | Remaining APP mRNA Level (%) (relative to the treated control group) | | | |
|---|---|---|---|---|
| | Cerebral cortex | Brain stem | Hypothalam us | Cervical spinal cord |
| C16(6-G)-12006.1-1 | 71.97 | 26.38 | 37.21 | 16.41 |
| BiAng2(K10N₃)-12006.2-4 | 39.56 | 15.32 | 27.54 | 9.92 |
| BiAng2(K10N₃)-12037.2-4 | 44.18 | 20.09 | 39.25 | 16.23 |
| BiAng2(K10N₃)-12075.2-4 | 31.84 | 9.50 | 23.80 | 3.78 |
| BiAng2(K10N₃)-12076.2-4 | 20.50 | 12.01 | 22.87 | 4.71 |

The results in Table 25 showed that different peptide-oligonucleotide conjugates delivered by BiAng2(K10N₃) exhibited high activity across various mouse brain tissues. We also observed that siRNA delivered via BiAng2(K10N₃) demonstrated higher activity than that delivered by C16. Subsequent safety testing of this peptide-oligonucleotide conjugate will be conducted.

### Embodiment 27 Safety Testing of APP-Targeting Peptide-Oligonucleotide Conjugates

In this Embodiment, we administered 0.7 mg of the BiAng2(K10N₃)-12006.2-4 peptide-oligonucleotide conjugate into the brains of wild-type C57/Bl6 mice via intracisternal injection. Each group consisted of 6 mice, with mice receiving CSF injections serving as negative controls. The safety of the APP-targeting peptide-oligonucleotide conjugates was assessed by evaluating the Functional Observational Battery (FOB) score in mice 24 and 48 h after intracisternal injection of the drug, based on 7 distinct criteria. The criteria for FOB are: (1) the mouse is smart, alert, and responsive; (2) the mouse stands or arches its back in the absence of stimulation; (3) the mouse displays any movement in the absence of stimulation; (4) the mouse exhibits forward movement after being lifted; (5) the mouse exhibits any movement after being lifted; (6) the mouse responds to tail pinch; (7) the mouse breathes evenly. If the mouse met any of the 7 criteria, a subscore of 0 was assigned; otherwise, a subscore of 1 was assigned. Following evaluation against all 7 criteria, the scores for each mouse were summed and averaged within each treatment group. The results are presented in Table 26.

**Table 26: Mouse FOB Scores Following Injection of Peptide-Oligonucleotide Conjugates**

| Peptide-oligonucleotide Conjugate | FOB 24h | FOB 48h |
|---|---|---|
| CSF | 0.00 | 0.00 |
| BiAng2(K10N₃)-12006.2-4 | 0.30 | 0.00 |

The results in Table 26 showed that 24 h after administration of a high-dose peptide-oligonucleotide conjugate into the mouse brain via intracisternal injection, the mice exhibited an FOB score of 0.3. This indicates that the peptide-oligonucleotide conjugate exhibits good tolerability in mice.

**Table 27: Results of Blood Biochemistry Following Injection of Peptide-Oligonucleotide Conjugates in Mice**

| Peptide-oligonucleoti de Conjugate | ALT (U/L) | AST (U/L) | ALP (U/L) | CREA (µmol) | UREA (µmol) |
|---|---|---|---|---|---|
| CSF | 41.67 | 167.83 | 194.27 | 14.93 | 10.32 |
| BiAng2(K10N₃)-1200 6.2-4 | 35.50 | 95.80 | 165.47 | 12.63 | 9.83 |

The results in Table 27 showed that biochemical testing of mouse blood samples collected 15 days after drug injection revealed no evidence of hepatic injury (ALT, AST, and ALP) or renal injury (CREA and UREA). This demonstrates that BiAng2(K10N₃)-12006.2-4 did not cause hepatic or renal injury, confirming the safety profile of the peptide-oligonucleotide conjugate.

The above embodiments only express several embodiments of the present invention. Although the description is relatively specific and detailed, it cannot be understood as limiting the patent scope of the present invention. It is to be pointed out that ordinarily skilled persons in the art may make several modifications and improvements without departing from the concept of the present invention, which all fall within the protection scope of the present invention. Therefore, the protection scope of the present invention shall be subject to the attached claims.

## Claims

1. An siRNA or a pharmaceutically acceptable salt thereof for suppressing the expression of the amyloid precursor protein (APP) gene, wherein the siRNA comprises a sense strand and a corresponding antisense strand, and wherein the siRNA is selected from any of the following pairs of sequences, or sequences differing from its sense strand or antisense strand by no more than one nucleotide:
A) 12006: its sense strand is shown in SEQ ID NO: 11, and its antisense strand is shown as SEQ ID NO: 12;
B) 12037: its sense strand is shown in SEQ ID NO: 75, and its antisense strand is shown as SEQ ID NO: 76;
C) 12075: its sense strand is shown in SEQ ID NO: 151, and its antisense strand is shown as SEQ ID NO: 152;
D) 12076: its sense strand is shown in SEQ ID NO: 153, and its antisense strand is shown as SEQ ID NO: 154.

2. An siRNA or a pharmaceutically acceptable salt thereof according to claim 1, wherein the nucleotides in the sense strand and the antisense strand of the siRNA are modified, wherein the sense strand comprises no more than 3, 2, 1, or 0 unmodified nucleotides, and the modified nucleotides in the sense strand include those selected from 2'-O-methyl-modified nucleotides, 2'-deoxyribonucleotides, 2'-fluorinated nucleotides, or reverse non-base residues, and the 5'- and 3'-termini of the sense strand contain 0, 1, 2, or 3 phosphorothioate bonds; and the antisense strand comprises no more than 3, 2, 1, or 0 unmodified nucleotides, wherein the modified nucleotides in the antisense strand are selected from 2'-O-methyl-modified nucleotides, 2'-deoxyribonucleotides, 2'-fluorinated nucleotides, VPU (2'-O-methyluridine-5'-(E)-vinylphosphate-3'-phosphate), VPU-S (2'-S-methyluridine-5'-(E)-vinylphosphate-3'-phosphate), or other VPU derivatives, and the antisense strand contains 1-3 phosphorothioate bonds at both its 5'- and 3'-termini.

3. An siRNA or a pharmaceutically acceptable salt thereof according to claim 2, wherein the siRNA is selected from any one of the following in Table 2: 12006.1-1, 12006.2-4, 12037.1-1, 12037.2-4, 12075.1-1, 12075.2-4, 12076.1-1, and 12076.2-4.

4. An siRNA or a pharmaceutically acceptable salt thereof according to claim 3, wherein the siRNA is selected from one of the following sequences, or a sequence differing from its sense strand or antisense strand by no more than one nucleotide:
a) 12006.2-4: Its 5'-3' sense strand is mC*mA*mCmAmAmGmUmUfCfUfUfUmGfAmGmCmAmGmAmUmA, and its 5'-3' antisense strand is VPU-S*fA*mUmCmUmGmCmUmCmAmAdAmGfAmAfCmUmUmG*mU*mG;
b) 12037.2-4: Its 5'-3' sense strand is mG*mC*mUmGmUmCmCmAfAfGfAfUmGfCmAmGmCmAmGmAmA, and its 5'-3' antisense strand is VPU-S*fU*mCmUmGmCmUmGmCmAmUdCmUfUmGfGmAmCmA*mG*mC;
c) 12075.2-4: Its 5'-3' sense strand is mC*mA*mAmGmUmCmUmAfCfCfCfUmGfAmAmCmUmGmCmAmA, and its 5'-3' antisense strand is VPU-S*fU*mGmCmAmGmUmUmCmAmGdGmGfUmAfGmAmCmU*mU*mG;
d) 12076.2-4: Its 5'-3' sense strand is mG*mG*mUmCmUmAfCfCfCfUmGfAmAmCmUmGmCmAmA, and its 5'-3' antisense strand is VPU-S*fU*mGmCmAmGmUmUmCmAmGdGmGfUmAfGmAmCmC*mU*mC;
wherein VPU-S refers to 2'-S-methyluridine-5'-(E)-vinylphosphonate-3'-phosphate, mA refers to 2'-O-methyladenosine-3'-phosphate, mU refers to 2'-O-methyluridine-3'-phosphate, mC refers to 2'-O-methylcytidine-3'-phosphate, mG refers to 2'-O-methylguanosine-3'-phosphate, fA refers to 2'-fluoroadenosine-3'-phosphate, fU refers to 2'-fluorouridine-3'-phosphate, fC refers to 2'-fluorocytidine-3'-phosphate, fG refers to 2'-fluoroguanosine-3'-phosphate, dA refers to 2'-deoxyadenosine-3'-phosphate, dT refers to 2'-deoxythymidine-3'-phosphate, dC refers to 2'-deoxycytidine-3'-phosphate, dG refers to 2'-deoxyguanosine-3'-phosphate, and * refers to a phosphorothioate bond.

5. An siRNA or a pharmaceutically acceptable salt thereof according to claim 1, wherein the pharmaceutically acceptable salt is a sodium salt or a potassium salt.

6. A conjugate for suppressing APP gene expression, comprising an siRNA or a pharmaceutically acceptable salt thereof according to any one of claims 1-5, and a targeted delivery ligand conjugated to the siRNA, or a targeted delivery carrier encapsulating the siRNA.

7. A conjugate according to claim 6, wherein the targeted delivery ligand comprises at least one peptide targeting low-density lipoprotein receptor-related protein 1 (LRP1) or transferrin receptor (TfR), and a linker connecting the peptide and the siRNA.

8. A conjugate according to claim 7, wherein the targeted delivery ligand comprises at least one peptide that targets low-density LRP1, which is the Angiopep-2 (ANG2) peptide.

9. A conjugate according to claim 8, wherein the N-to-C-terminal amino acid sequence of the ANG2 peptide is TFFYGGSRGKRNNFKTEEY;
and/or the targeted delivery ligand comprises two ANG2 peptides.

10. A conjugate according to claim 9, wherein the linker is conjugated to the N-terminus or a lysine side-chain amino group of each ANG2 peptide.

11. A conjugate according to claim 10, wherein the linker is conjugated to lysine at position 10 of each ANG2 peptide.

12. A conjugate according to any one of claims 7-11, wherein the structure of the linker connecting the peptide and the siRNA is selected from any one of the following: 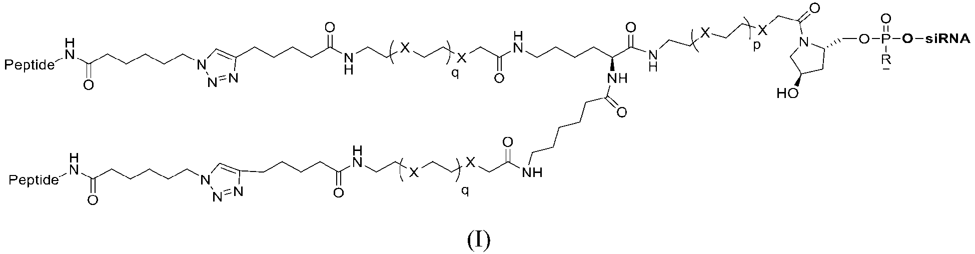 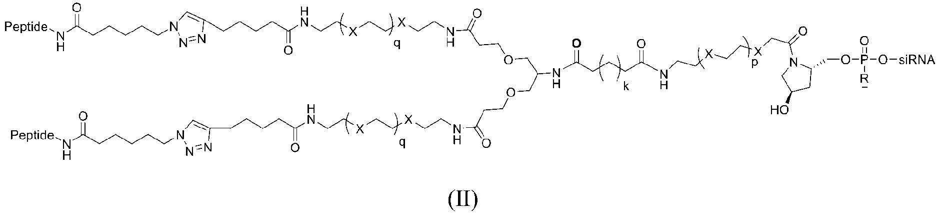 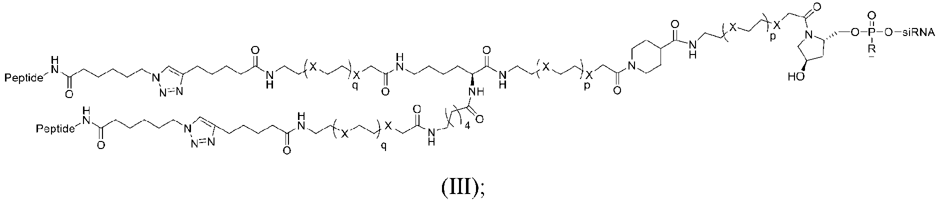 Wherein X and Y are O or CH₂; R is O or S; p and q are integers from 0 to 4; and k is 1, 2, 4, or 8.

13. A conjugate according to claim 12, wherein the conjugate structure formed by linking the ANG2 peptide to the siRNA via the linker is selected from any one of the following: 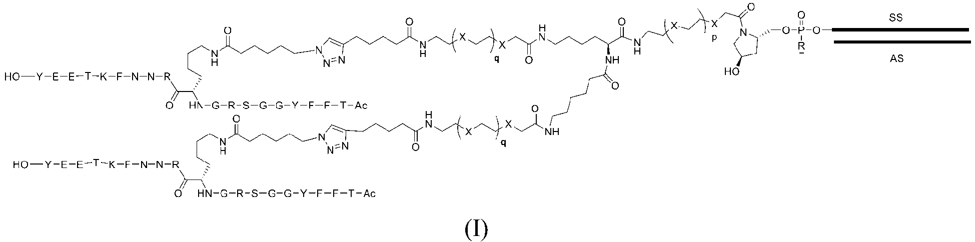 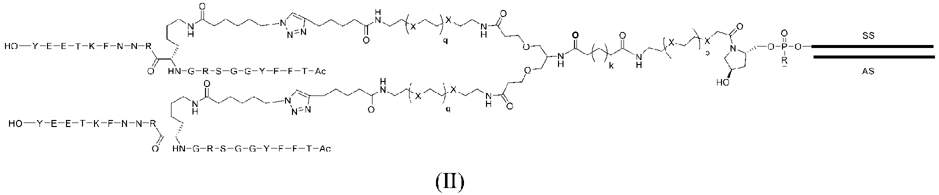 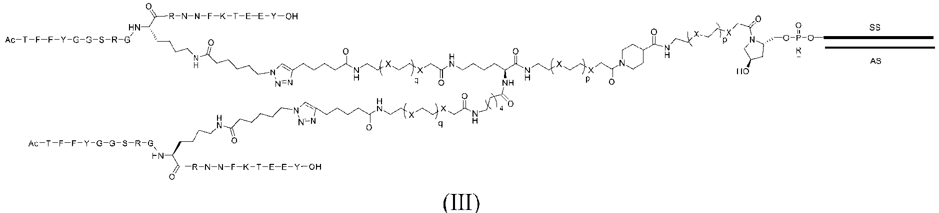 Wherein X and Y are O or CH₂; R is O or S; p and q are integers from 0 to 4; and k is 1, 2, 4, or 8.

14. A conjugate according to claim 13, wherein the structure of the targeted delivery ligand formed by connecting the ANG2 peptide to the siRNA via a linker is one of the following: 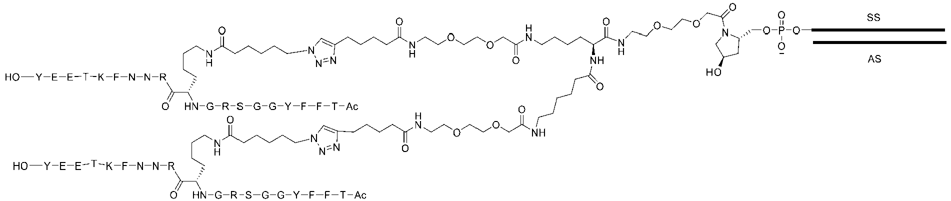 BiAng2(K10N₃)-B08-siRNA 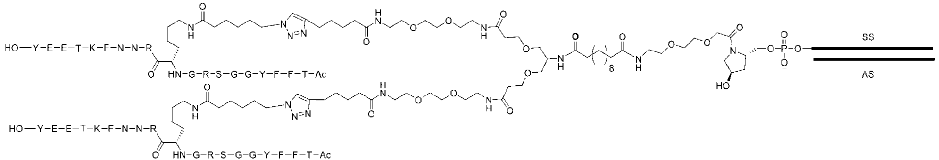 Bi-Sym-Ang2(K10N₃)-siRNA 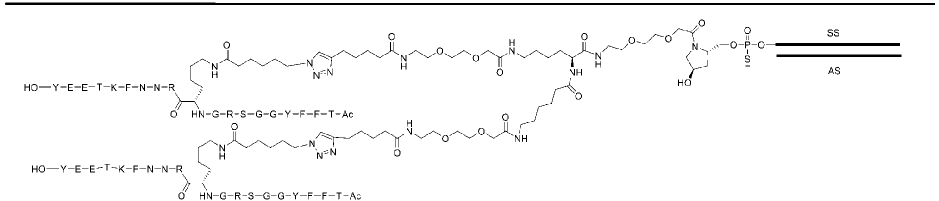 BiAng2(K10N₃)-B08-siRNA-2 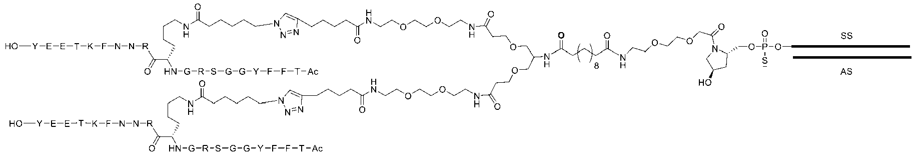 Bi-Sym-Ang2(K10N₃)-siRNA-2 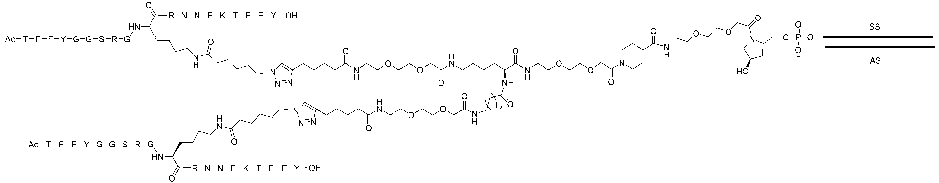 BiAng2(K10N₃)-Q22-siRNA 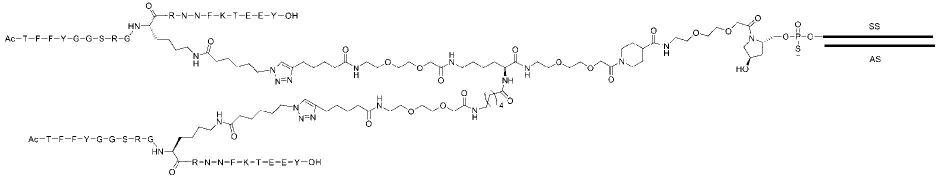 BiAng2(K10N₃)-Q22-siRNA-2.

15. An application of the siRNA or a pharmaceutically acceptable salt thereof according to any one of claims 1-5, or the conjugate according to any one of claims 6-14, in the preparation of medications for preventing or treating APP gene-mediated diseases.

16. An application according to claim 15, wherein the APP gene-mediated disease is cerebral amyloid angiopathy (CAA), early-onset familial Alzheimer's disease (EOFAD), or Alzheimer's disease (AD).

17. A method for preventing or treating APP gene-mediated diseases, wherein a subject is administered an appropriate dose of the siRNA or a pharmaceutically acceptable salt thereof according to any one of claims 1-5, or the conjugate according to any one of claims 6-14.

18. A method according to claim 17, wherein the subject is a mammal, preferably a primate.

19. A method for suppressing APP gene expression, wherein the method comprises the following steps:
salt thereof according to any one of claims 1-5, or the conjugate according to any one of claims 6-14; and
(b) Maintaining the cells generated in step (a) for a duration sufficient to allow degradation of mRNA transcripts encoding APP, thereby simultaneously suppressing APP expression within the cells.
